# EUROPEAN PATENT APPLICATION

(11) **EP 4 814 009 A1**
(43) Date of publication of application: **30.09.2026**
(21) Application number: 24894712.9
(22) Date of filing: 25.11.2024
(51) Int. Cl.: C07K 16/18, C07K 14/495, C07K 16/28, A61P 35/00, A61K 39/00

(54) **FUSION PROTEIN SPECIFICALLY BINDING TO EXTRADOMAIN B OF FIBRONECTIN (EDB-FN) AND TRANSFORMING GROWTH FACTOR BETA (TGFB), AND USE THEREOF**

(30) Priority: 24.11.2023 KR 20230166052
(71) Applicant: Medpacto Inc., Seoul 06668 (KR); Kim, Seong Jin, Seoul 06667 (KR)
(72) Inventor: KANG, Dong Woo, Hanam-si Gyeonggi-do 12912 (KR); KIM, Seong Jin, Seoul 06667 (KR)
(74) Representative: Maiwald GmbH
(86) International application number: PCT/KR2024/018743
(87) International publication number: WO 2025/110822

(57) **Abstract**

The present invention relates to a fusion protein that specifically binds to extradomain B of fibronectin (EDB-FN) and transforming growth factor β (TGFβ), and use thereof, and, more specifically, to a fusion protein comprising a polypeptide that specifically binds to EDB-FN and a polypeptide that specifically binds to TGFβ, and use thereof. The fusion protein according to the present invention anchors TGFβ, which is important in antitumor immune responses, to the extracellular matrix so as to localize TGFβ inhibition directly within the tumor microenvironment and ensure local rather than systemic effect, thereby exhibiting excellent anticancer effect in all carcinomas, and also in pancreatic cancer in which common TGFβ inhibitors do not act due to the rigidity of the extracellular matrix, and thus can be effectively used for preventing or treating cancer.

## Description

### TECHNICAL FIELD

The present invention relates to a fusion protein that specifically binds to extradomain B of fibronectin (EDB-FN) and transforming growth factor β (TGFβ) and use thereof, and more specifically to a fusion protein including a polypeptide that specifically binds to EDB-FN and a polypeptide that specifically binds to TGFβ and use thereof.

### BACKGROUND ART

One of the primary functions of TGFβ is to suppress the activation and infiltration of immune cells, particularly CD8+ cytotoxic T cells, which are critical for effective antitumor immune responses. TGFβ performs this immunosuppressive function by promoting the differentiation of regulatory T cells (Tregs), which enhance immune tolerance. It also sustains the immunosuppressive activity of tumor-associated macrophages (TAMs) and cancer-associated fibroblasts (CAFs), both of which contribute to the immunosuppressive and desmoplastic (stromal-rich) properties of the tumor microenvironment (TME). In addition to suppressing immune responses, these stromal cells also contribute to the formation of a physical barrier that further inhibits immune cell infiltration by increasing the stiffness and density of the extracellular matrix (ECM). TGFβ signaling also plays a pivotal role in promoting epithelial-to-mesenchymal transition (EMT), which is a process by which tumor cells acquire invasiveness and metastatic potential. TGFβ-driven EMT is known to promote metastasis and cancer progression by increasing tumor cell motility, resisting apoptosis and evading immune detection (Derynck R, et al., Nat. Rev. Clin. Oncol. Vol. 18(1), pp. 9-34, 2021; Hao Y, et al., Int. J. Mol. Sci. Vol. 20(11):2767, 2019).

Considering these functions, the inhibition of TGFβ within the TME is essential to counteract its tumor-promoting and immunosuppressive effects while minimizing systemic toxicity. In particular, tumor-specific TGFβ inhibition targeting the ECM using fusion proteins or antibody conjugates can effectively reprogram the TME by reducing matrix stiffness, increasing immune cell infiltration and enhancing the efficacy of immune-based therapies such as checkpoint inhibitors. Therefore, the inhibition of TGFβ within the TME offers the dual benefit of reversing the immunosuppressive state that protects tumors from immune attack and suppressing the metastatic and invasive properties of cancer cells. These effects make tumor-specific TGFβ inhibitors a promising therapeutic strategy in cancer, particularly in solid tumors where the TME plays a critical role in disease progression (Karin E. de Visser et al., Vol. 41(3), pp. 374-403, 2023).

Meanwhile, pancreatic ductal adenocarcinoma (PDAC) is one of the most lethal cancers, and is characterized by desmoplasia that forms a dense fibrous barrier within the pancreatic duct, thereby causing physical and immunosuppressive problems. This dense matrix impedes the efficacy of existing treatments, including immune checkpoint inhibitors (ICIs), which have shown success in other cancers but are mostly ineffective in PDAC due to the rigid nature of the ECM. Therefore, targeting the ECM has become very important to improve the treatment outcomes of PDAC patients.

Fibronectin, which is a glycoprotein found in the ECM, plays a key role in cancer progression, particularly in PDAC. Among its isoforms, extradomain B (EDB-FN) is re-expressed in malignant tissues and supports tumor growth, angiogenesis and immune evasion. EDB-FN is overexpressed in a variety of tumors, including pancreatic, lung and prostate cancers, while it is rarely present in normal adult tissues, making it an attractive target for tumor-specific therapies. Targeting EDB-FN within the ECM offers several advantages, including the ability to localize therapies to the TME, thereby minimizing off-target effects and improving tumor penetration. In addition, the stiffness of the ECM due to fibronectin and collagen cross-linking creates a physical barrier that impedes the infiltration of immune cells, and this stiffness traps cytokines such as TGFβ, which exacerbates immunosuppressive effects. Studies have shown that a stiff ECM insulates tumors from immune attack, thereby reducing the therapeutic efficacy of immune checkpoint inhibitors such as anti-PD-1 in cancers where they should be effective (Principe DR. et al., Cancers. Vol. 13(20):5086, 2021; Perez VM. et al., Front. Oncol. Vol. 11:751-311, 2021). Therefore, a dual-targeting strategy that reduces ECM stiffness while inhibiting TGFβ is important to realize the potential of immune-based therapy in fibrotic tumors such as PDAC.

Structures that combine an EDB-FN targeting motif (e.g., L19 scFv or anti-EDB antibody) with a TGFβ-Trap are expected to localize TGFβ inhibition directly within the TME by anchoring TGFβ-Trap to the ECM via EDB-FN binding, thereby ensuring local rather than systemic effects. Therefore, they may enhance anti-tumor immune responses while minimizing side effects. Such fusion proteins could overcome ECM resistance to immunotherapy, converting "cold" tumors (immunologically inert) into "hot" tumors (immunologically active), making them more susceptible to immune checkpoint blockade.

However, to date, no fusion protein or bispecific antibody with the above structure has been known, and only L19 (EDB Ab)-IL12, L19-IL9 (Aliyah BS et al., Trends in Pharmacological Sciences, Vol. 42(12), pp. 1064-81, 2021), LTBR-EDB multispecific antibody (KR 2022-0130687) and TGFβ-Trap-PD-L1 (Lind H, et al., J. Immunother. Cancer. Vol. 8(1):e000433, 2020) are known.

Under this technical background, the inventors of the present invention have made efforts to develop a fusion protein having excellent anticancer efficacy by simultaneously binding to EDF-FN targeting motif and TGFβ, and as a result, the inventors of the present invention confirmed that an excellent anticancer effect is exhibited when a polypeptide that specifically binds to TGFβ and a polypeptide that specifically binds to EDF-FN are fused with Fc as the center, thereby completing the present invention.

The above information set forth in this Background Art section is intended solely to enhance understanding of the background of the present invention and may not include information that constitutes prior art already known to a person skilled in the art to which the present invention pertains.

### SUMMARY OF THE INVENTION

An object of the present invention is to provide a fusion protein that specifically binds to extradomain B of fibronectin (EDB-FN) and transforming growth factor β (TGFβ).

Another object of the present invention is to provide a nucleic acid encoding the fusion protein.

Still another object of the present invention is to provide a recombinant expression vector including the nucleic acid or a cell into which the nucleic acid or expression vector is introduced.

Still another object of the present invention is to provide a method for producing the fusion protein.

Still another object of the present invention is to provide a composition for preventing or treating cancer, including a fusion protein.

In order to achieve the above objects, the present invention provides a fusion protein, including (a) a polypeptide that specifically binds to extradomain B of fibronectin (EDB-FN); and (b) a polypeptide that specifically binds to transforming growth factor β (TGFβ).

The present invention also provides a nucleic acid encoding the fusion protein.

The present invention also provides a recombinant expression vector, including the nucleic acid.

The present invention also provides a host cell transfected with the recombinant expression vector.

The present invention also provides a method for producing the fusion protein, including culturing the host cell to produce the fusion protein; and isolating and purifying the produced fusion protein.

The present invention also provides a composition for preventing or treating cancer, including the fusion protein.

### DESCRIPTION OF DRAWINGS

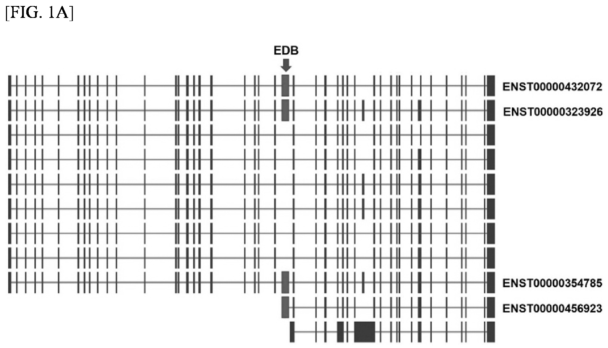

FIG. 1A shows the results of analyzing the Fibronectin transcript in the human genome region.

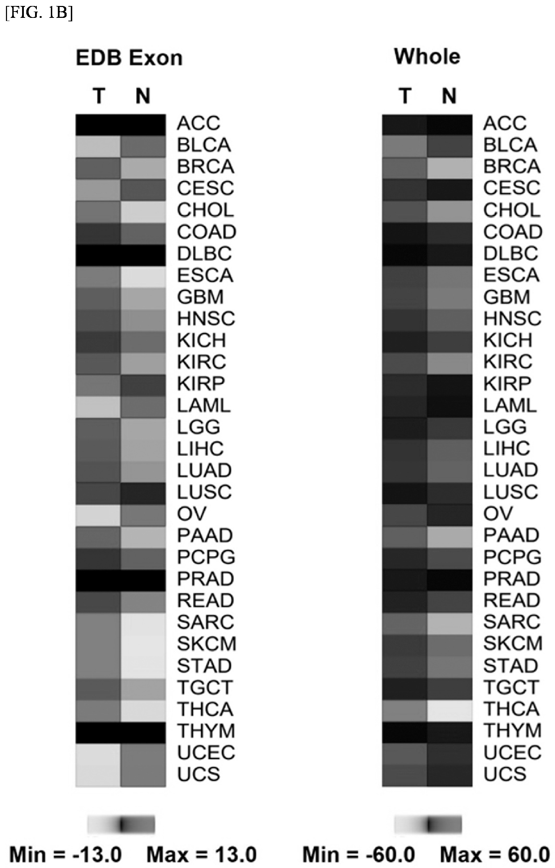

FIG. 1B shows the results of analyzing the expression of the EDB exon and the entire FN1 gene in cancer patients and normal people using the TCGA and GTEx databases.

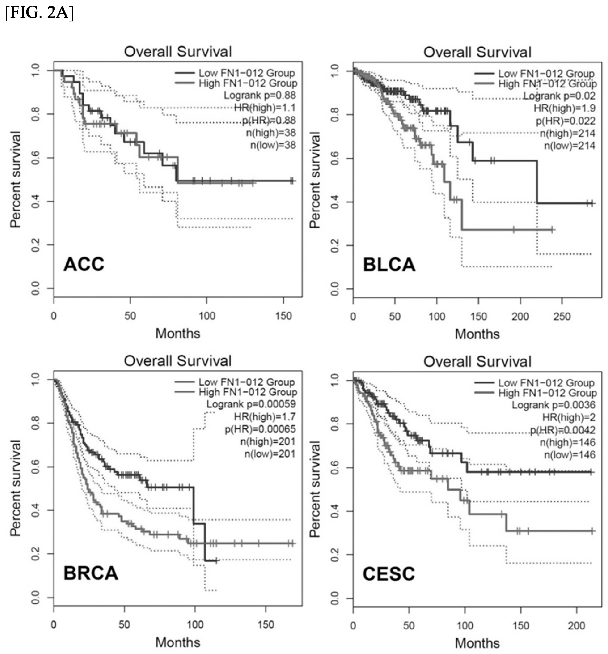

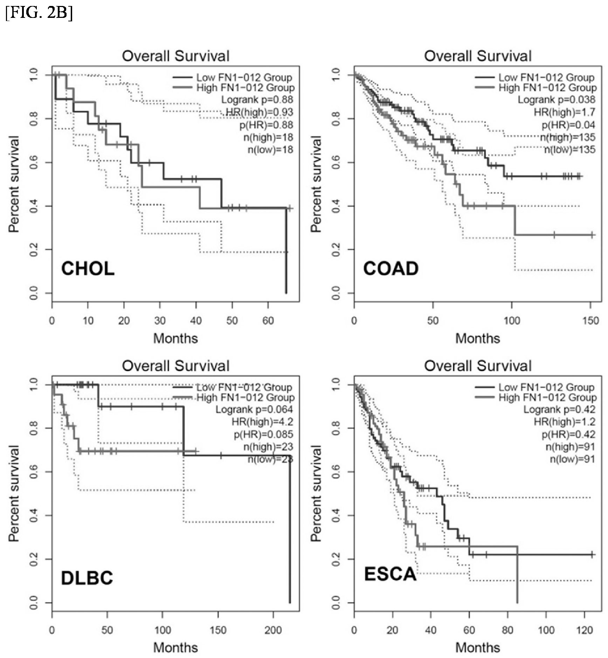

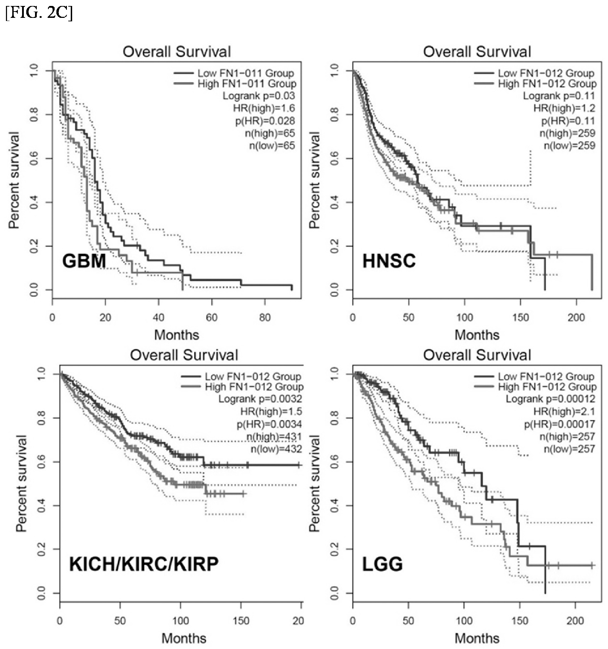

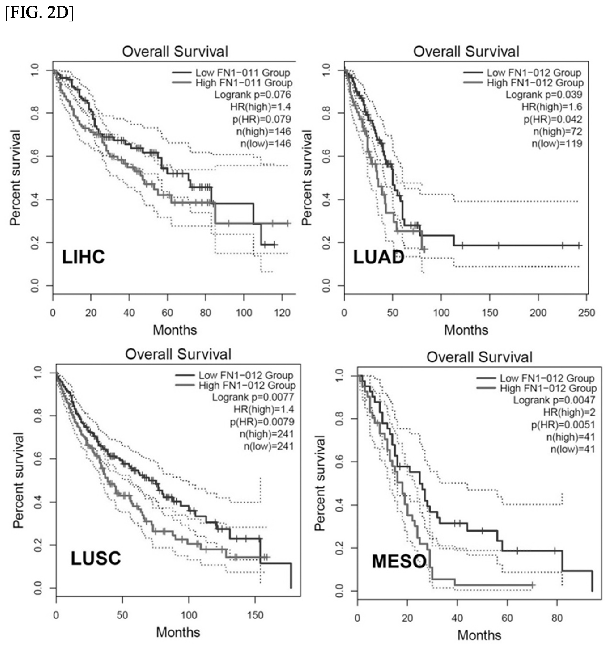

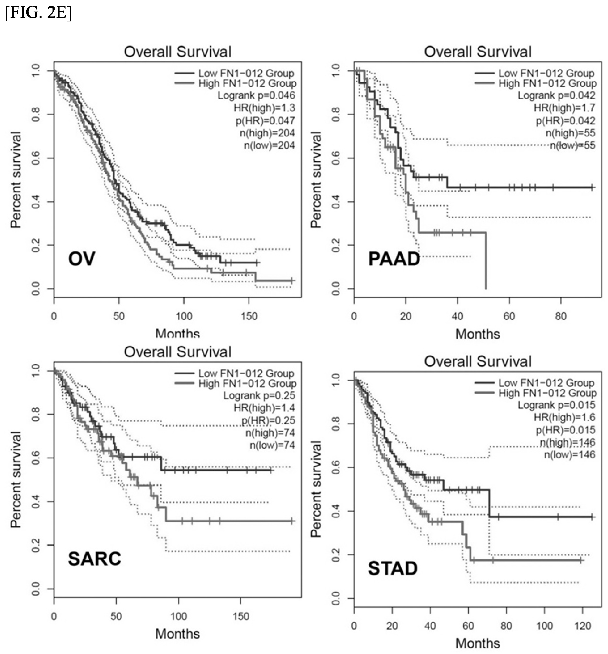

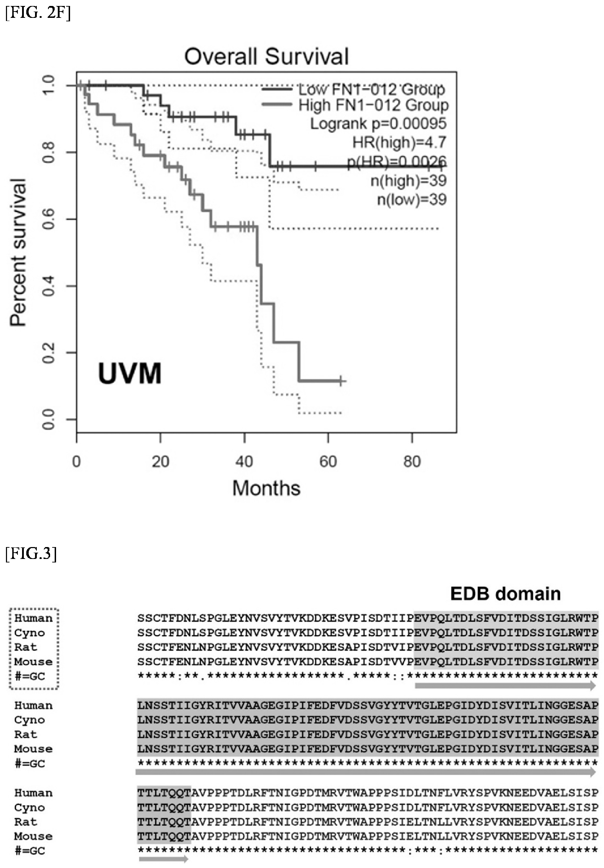

FIGS. 2A to F show the results of analyzing the association between FN1 EDB+ gene expression and prognosis in the TCGA database. ACC means adenoid cystic carcinoma, BLCA means bladder urothelial carcinoma, BRCA means breast invasive carcinoma, CHOL means cholangiocarcinoma, COAD means colon adenocarcinoma, DLBC means lymphoid neoplasm diffuse large B-cell lymphoma, ESCA means esophageal carcinoma, GBM means glioblastoma multiforme, HNSC means head and neck squamous cell carcinoma, KICH means kidney chromophobe, KIRC means kidney renal clear cell carcinoma, KIRP means kidney renal papillary cell carcinoma, LGG means brain lower grade glioma, LIHC means liver hepatocellular carcinoma, LUAD means lung adenocarcinoma, LUSC means lung squamous cell carcinoma, MESO means mesothelioma, OV means ovarian serous cystadenocarcinoma, PAAD means pancreatic adenocarcinoma, SARC means sarcoma, STAD means stomach adenocarcinoma, and UVM means uveal melanoma.
FIG. 3 shows the sequence alignment results of human, monkey, rat and mouse of the FN1 EDB domain.

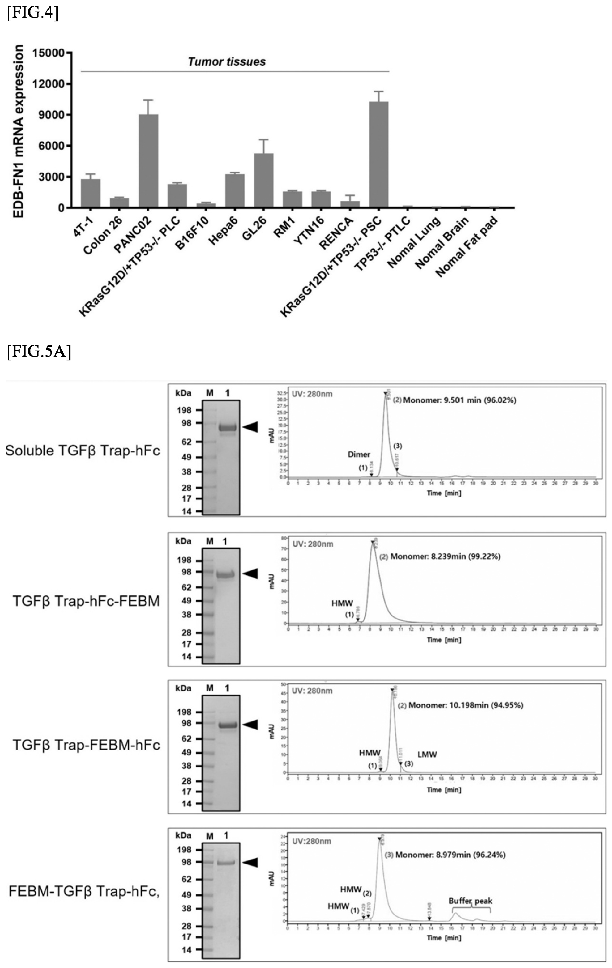

FIG. 4 shows the results comparing the expression levels of FN1-EDB+ transcripts in 11 orthotopically transplanted tumor tissues and normal tissues.

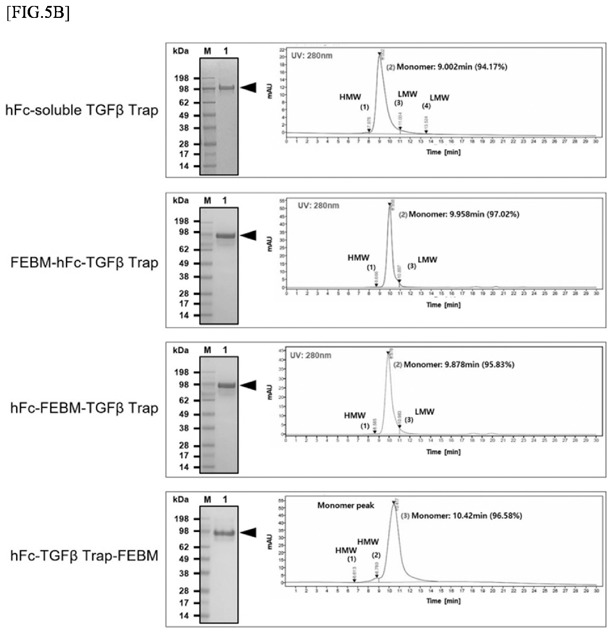

FIGS. 5A and B show the purification results of a dual-specific Fc fusion protein including FEBM and TGFβ Trap produced according to one embodiment of the present invention, wherein the portions indicated by the marker and lane 1 are the SDS-PAGE results, and the portions indicated by the graph are the size exclusion chromatography (SEC) results.

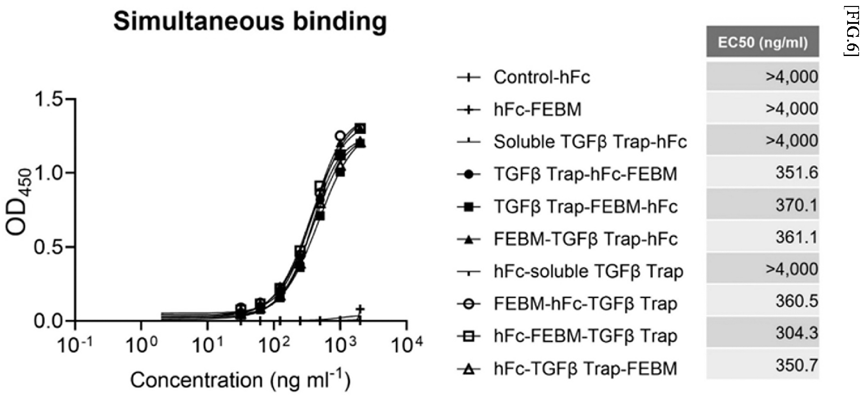

FIG. 6 shows the results of evaluating the simultaneous binding level of EDB-FN and TGF-β1 of a dual-specific Fc fusion protein including FEBM and TGFβ Trap produced according to one embodiment of the present invention.

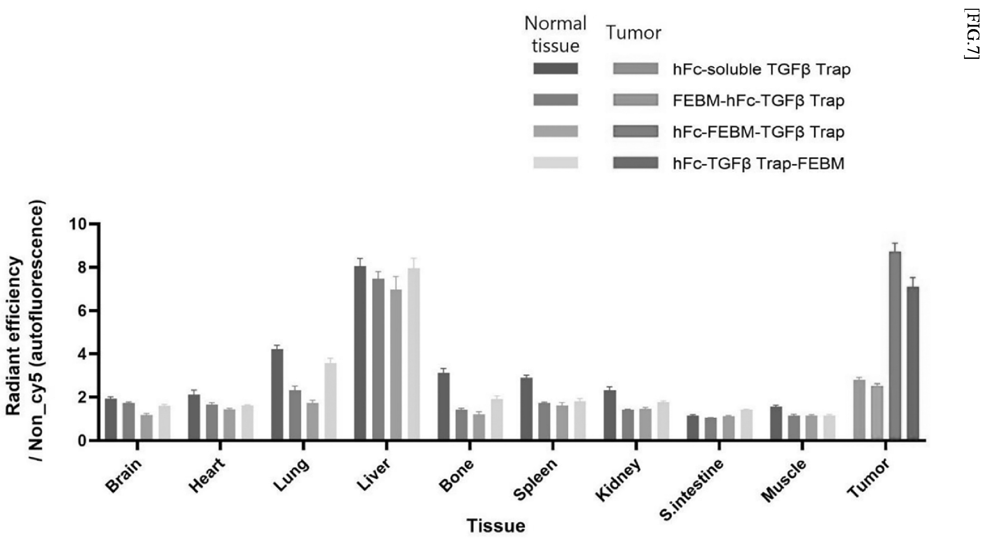

FIG. 7 shows the results of confirming the tissue-specific distribution of a dual-specific Fc fusion protein including FEBM and TGFβ Trap produced according to one embodiment of the present invention in a 4T-1 orthotopic mouse model.

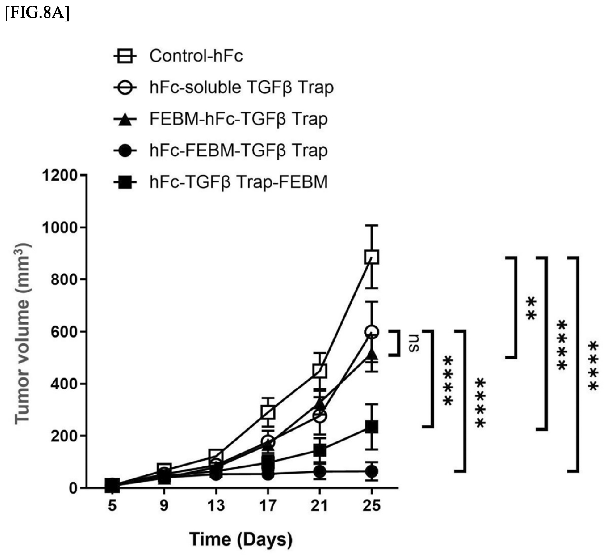

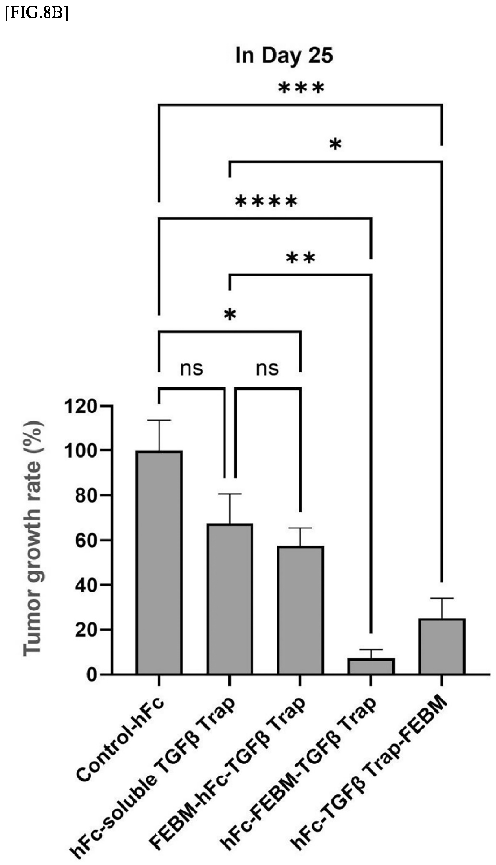

FIG. 8 shows the results of analyzing the tumor growth rate in a breast cancer orthotopic transplantation model by the administration of a dual-specific Fc fusion protein including FEBM and TGFβ Trap according to one embodiment of the present invention. FIG. 8A shows the results of measuring the tumor volume over time for each fusion protein, and FIG. 8B shows the results of measuring the growth rate on day 25 for each fusion protein. Each data represents ± s.e.m.; *P < 0.05, **P < 0.01, ***P < 0.001, ****P < 0.0001; NS represents not significant; and the results were analyzed by two-way ANOVA with post hoc Bonferroni t-test.

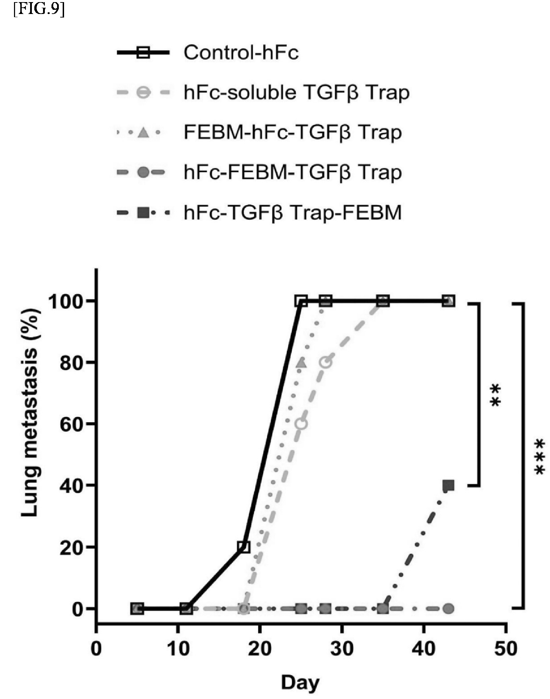

FIG. 9 shows the results of analyzing the lung metastasis rate in a breast cancer orthotopic transplantation model by the administration of a dual-specific Fc fusion protein including FEBM and TGFβ Trap according to one embodiment of the present invention. **P < 0.01, ***P < 0.001; the results are analyzed by two-way ANOVA with post hoc Bonferroni t-test.

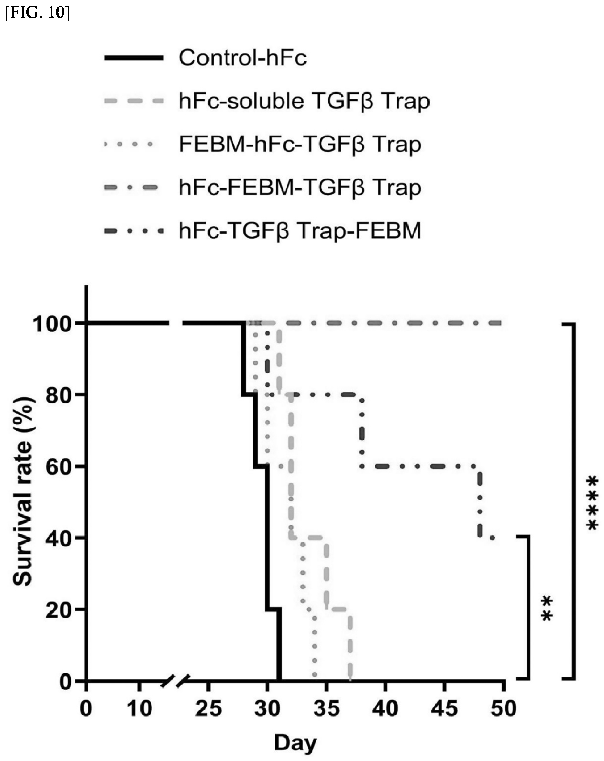

FIG. 10 shows the results of measuring the survival rate in a breast cancer orthotopic transplantation model by the administration of a dual-specific Fc fusion protein including FEBM and TGFβ Trap according to one embodiment of the present invention. **P < 0.01, ****P < 0.0001; the results are analyzed by two-way ANOVA with log-rank test.

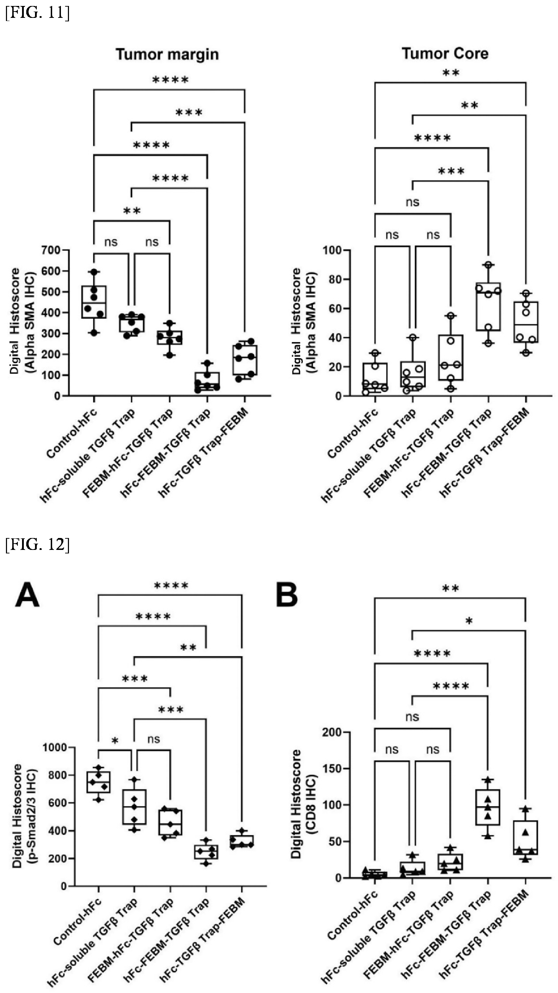

FIG. 11 shows the results of measuring the expression levels of α-SMA in the tumor margin and center in a breast cancer orthotopic transplantation model administered with a dual-specific Fc fusion protein including FEBM and TGFβ Trap according to one embodiment of the present invention. The left panel shows the results of quantifying α-SMA staining in the tumor margin and the right panel shows the results of quantifying α-SMA staining in the tumor center. Each data represents ± sem; *** P < 0.05, ** *P < 0.01,* *** P < 0.001, **** P <0.0001; NS represents not significant; and the results were analyzed by two-way ANOVA with post hoc Bonferroni t -test.
FIG. 12 shows the results of measuring the Smad2 phosphorylation level and CD8 expression level in a breast cancer orthotopic transplantation model administered with a dual-specific Fc fusion protein including FEBM and TGFβ Trap according to one embodiment of the present invention, wherein A represents the Smad2 phosphorylation level, and B represents the results of measuring the CD8 expression level. Each data represents ± sem; *P < 0.05, ***P* < 0.01, ****P* < 0.001, **** *P* <0.0001; NS represents not significant; and the results were analyzed by two-way ANOVA with post hoc Bonferroni t-test.

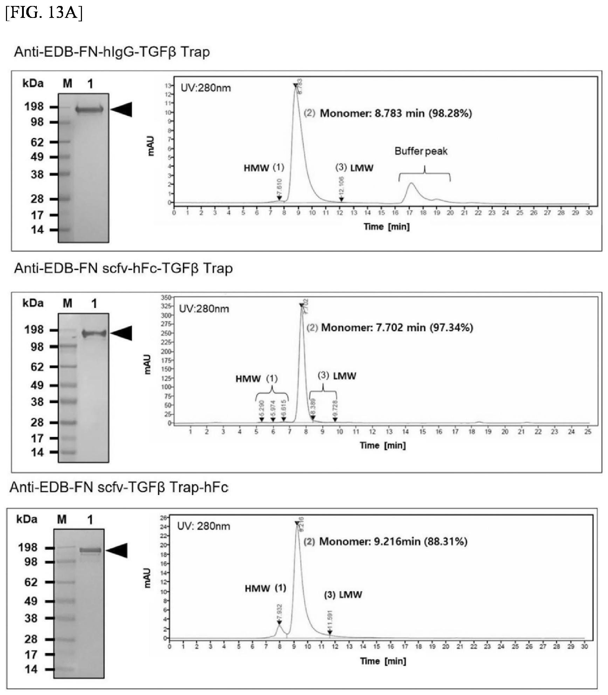

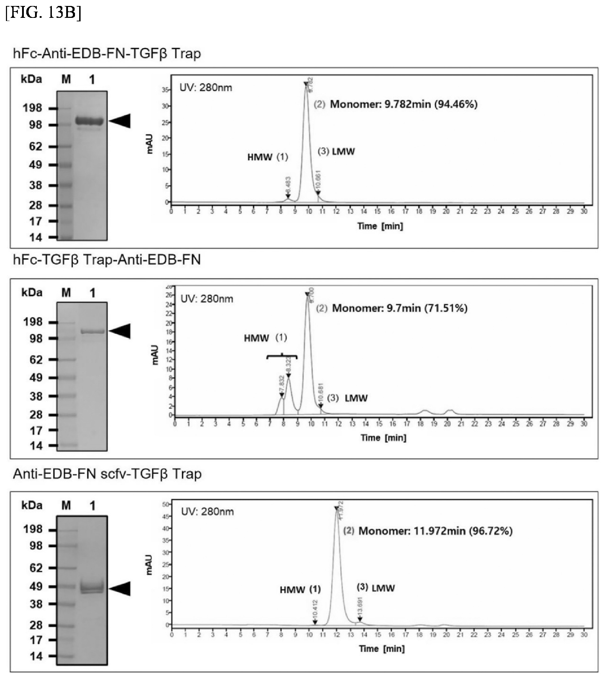

FIGS. 13A and B show the results of purification of a fusion protein including Anti-EDB-FN antibody, TGFβ Trap and hFc produced according to one embodiment of the present invention, wherein the portions indicated by the marker and lane 1 are the results of SDS-PAGE, and the portions indicated by the graph are the results of size exclusion chromatography (SEC).

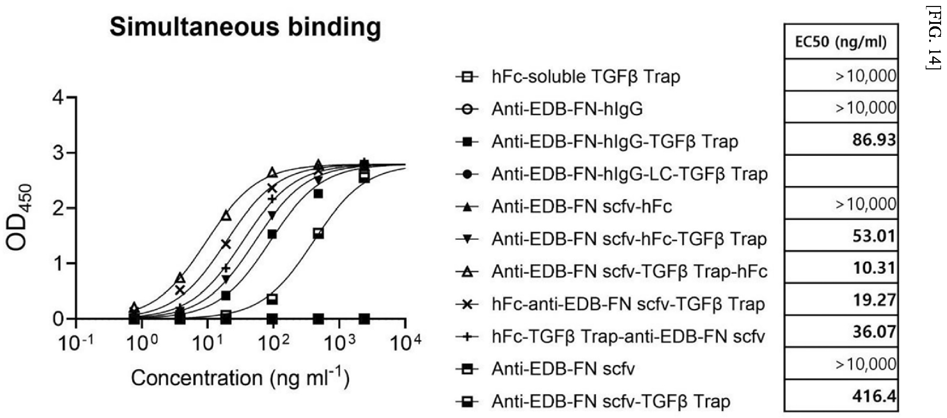

FIG. 14 shows the results of evaluating the simultaneous binding level of EDB-FN and TGF-β1 of a dual-specific Fc fusion protein including an anti-EDB-FN antibody and a TGFβ Trap produced according to one embodiment of the present invention.

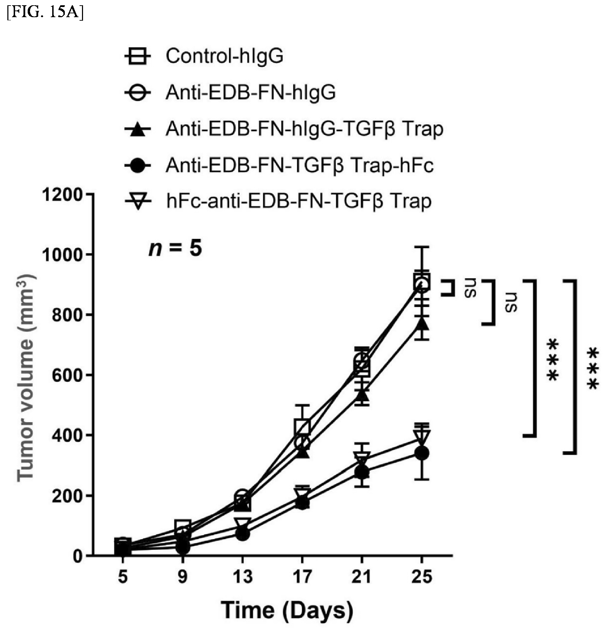

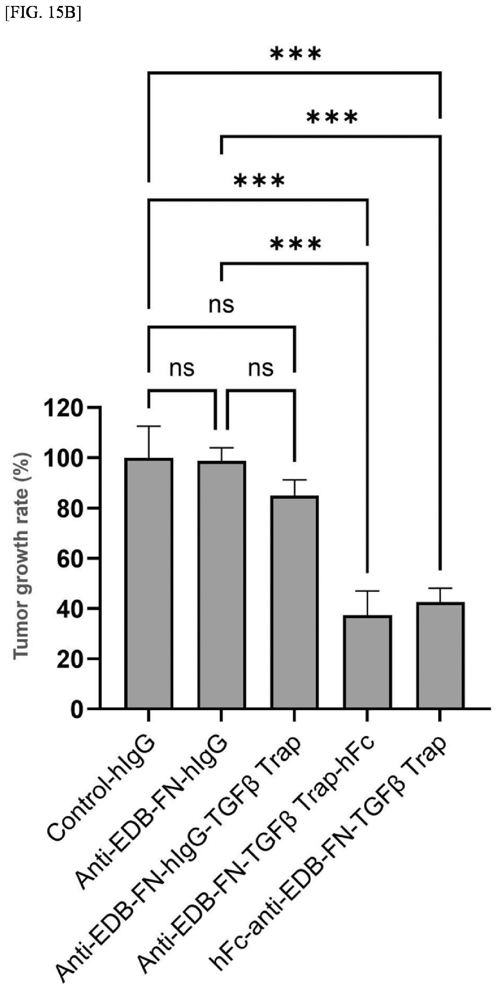

FIG. 15 shows the results of analyzing the tumor growth rate in a breast cancer orthotopic transplantation model by the administration of a dual-specific Fc fusion protein including Anti-EDB-FN antibody and TGFβ Trap according to one embodiment of the present invention. FIG. 15A shows the results of measuring the tumor volume over time for each fusion protein, and FIG. 15B shows the results of measuring the growth rate over time for each fusion protein. Each data represents ± s.e.m; *** represents P < 0.001; NS represents not significant; and is the result of analysis by two-way ANOVA with post hoc Bonferroni t-test.

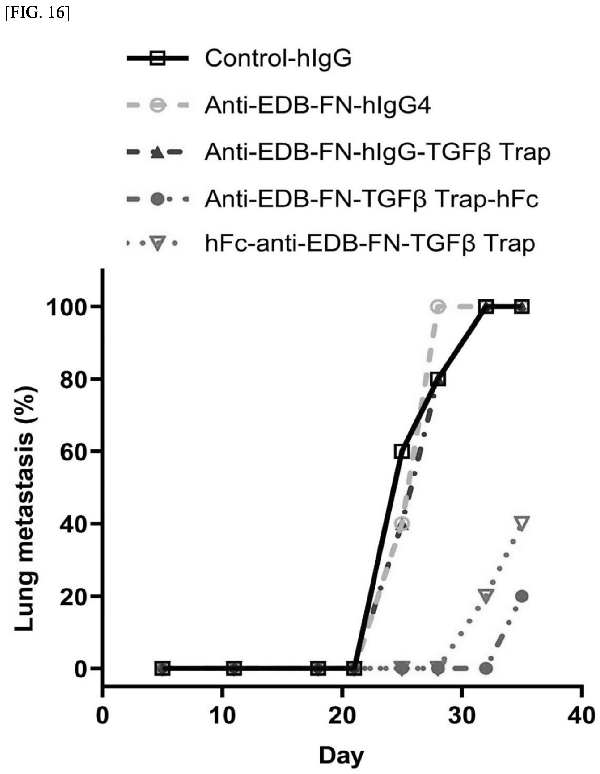

FIG. 16 shows the results of analyzing the lung metastasis rate in a breast cancer orthotopic transplantation model by the administration of a dual-specific Fc fusion protein including an Anti-EDB-FN antibody and TGFβ Trap produced according to one embodiment of the present invention. **P < 0.01; the results are analyzed by two-way ANOVA with post hoc Bonferroni t-test.

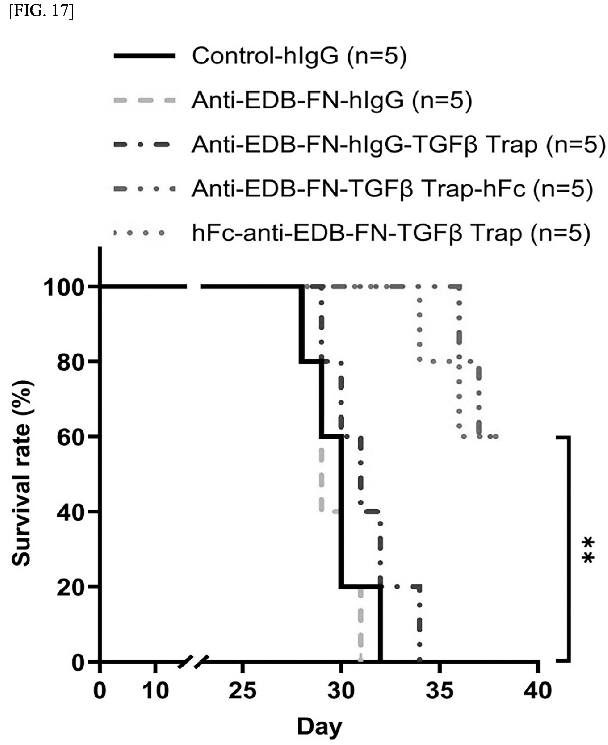

FIG. 17 shows the results of measuring the survival rate in a breast cancer orthotopic transplantation model by the administration of a dual-specific Fc fusion protein including an Anti-EDB-FN antibody and TGFβ Trap produced according to one embodiment of the present invention. **P < 0.01; the results are analyzed by two-way ANOVA with log-rank test.

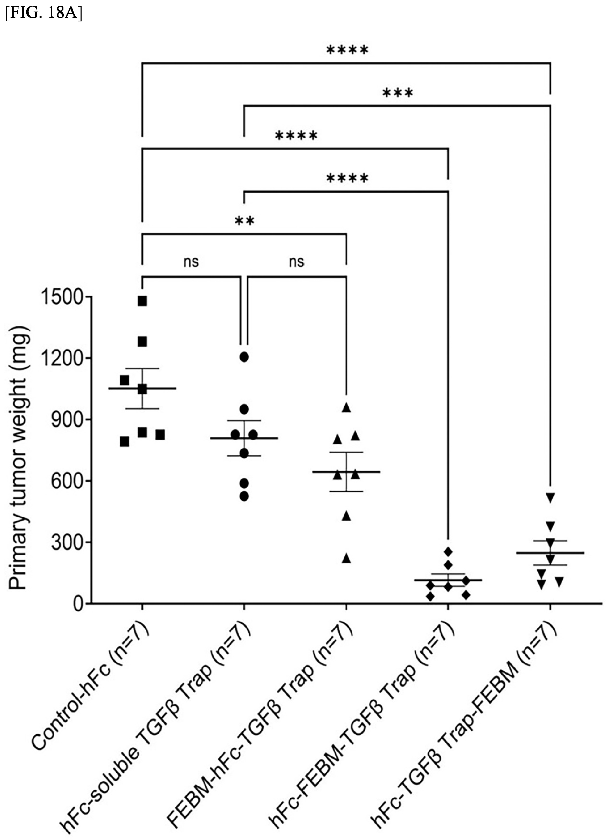

FIGS. 18A to 18C show the results of analyzing tumor growth in a pancreatic cancer orthotopic transplantation model administered with a dual-specific Fc fusion protein including FEBM and TGFβ Trap according to one embodiment of the present invention, where a represents tumor weight, b represents growth rate, and c represents survival rate. Each data represents ± s.e.m.; *P < 0.01, **P < 0.001, ***P < 0.0001; NS represents not significant; and the results were analyzed by two-way ANOVA with post hoc Bonferroni t-test.

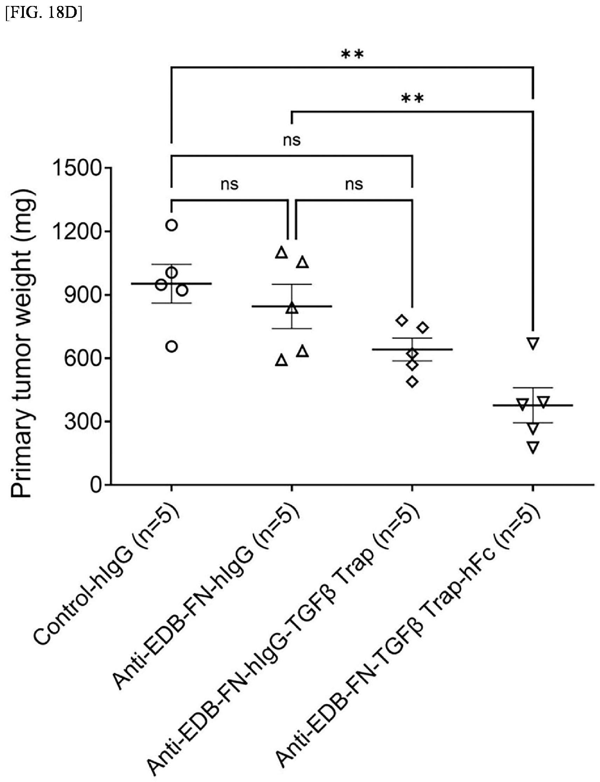

FIGS. 18D to 18F show the results of analyzing tumor growth in a pancreatic cancer orthotopic transplantation model by the administration of a dual-specific Fc fusion protein including Anti-EDB-FN antibody and TGFβ Trap produced according to one embodiment of the present invention, where a represents tumor weight, b represents growth rate, and c represents survival rate. Each data represents ± s.e.m.; *P < 0.01, **P < 0.001, ***P < 0.0001; NS represents not significant; and the results were analyzed by two-way ANOVA with post hoc Bonferroni t-test.

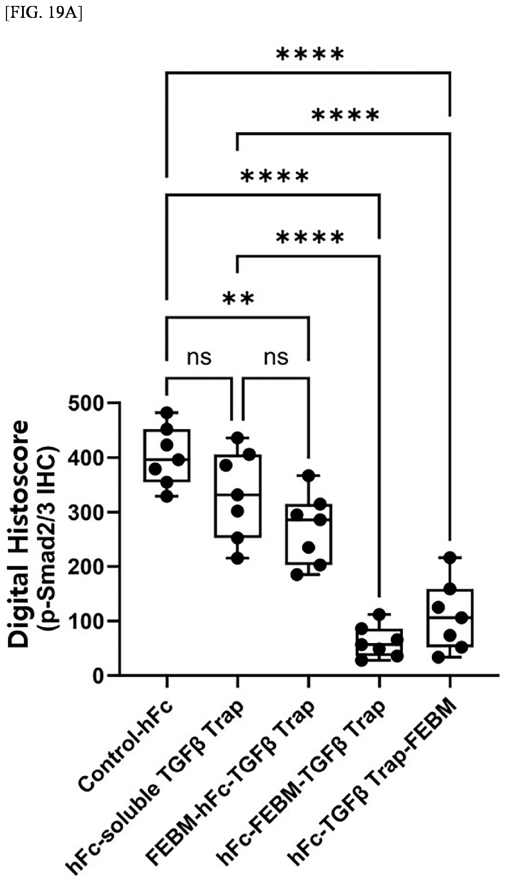

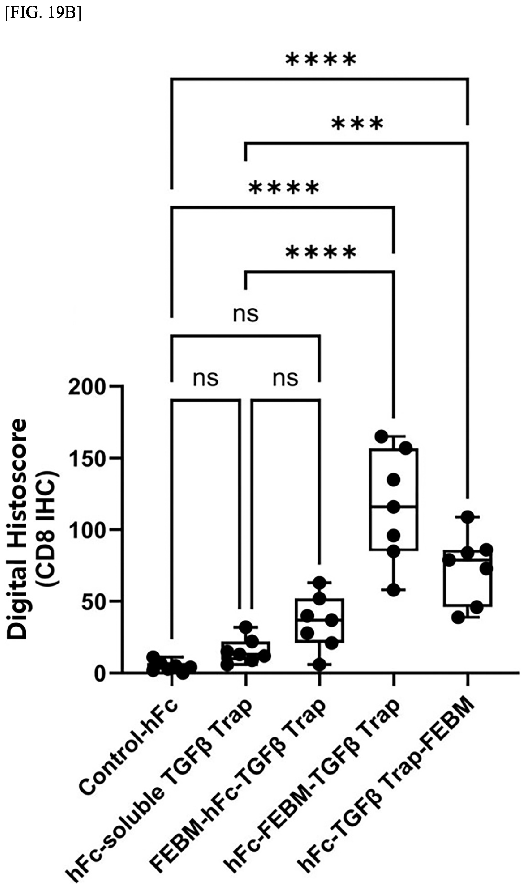

FIG. 19 shows the results of measuring the Smad2 phosphorylation level and CD8 expression level in a pancreatic cancer orthotopic transplantation model administered with a dual-specific Fc fusion protein including FEBM and TGFβ Trap according to one embodiment of the present invention. FIG. 19A shows the results of measuring the Smad2 phosphorylation level, and FIG. 19B shows the results of measuring the CD8 expression level. Each data means ± sem; *P < 0.05, ***P* < 0.01, ***P< 0.001, *****P*<0.0001*;* NS means not significant; and the results were analyzed by two-way ANOVA with post hoc Bonferroni t-test.

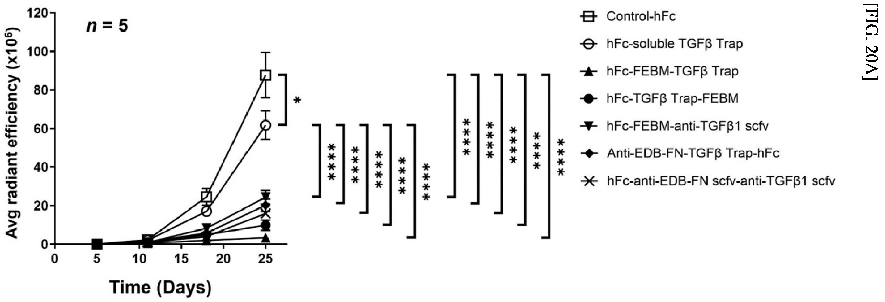

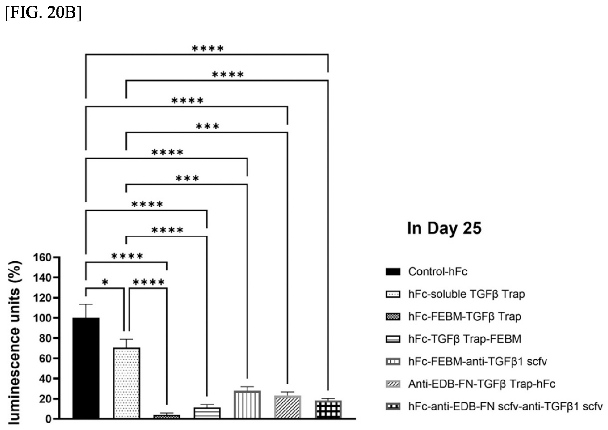

FIG. 20 is the results of confirming the tumor growth inhibitory effect in a melanoma orthotopic transplantation -> lung metastasis tumor growth model by the administration of a dual-specific Fc fusion protein including FEBM and TGFβ Trap and a dual-specific Fc fusion protein including Anti-EDB-FN antibody and TGFβ Trap, produced according to an embodiment of the present invention. FIG. 20A is the result of measuring the tumor growth rate over time, and FIG. 20B is the tumor growth rate measured on day 25. Each data means ± sem; *P < 0.05, ***P* < 0.05, ****P* < 0.001, *****P* <0.0001; NS means not significant; and the result is analyzed by two-way ANOVA with post hoc Bonferroni t-test.

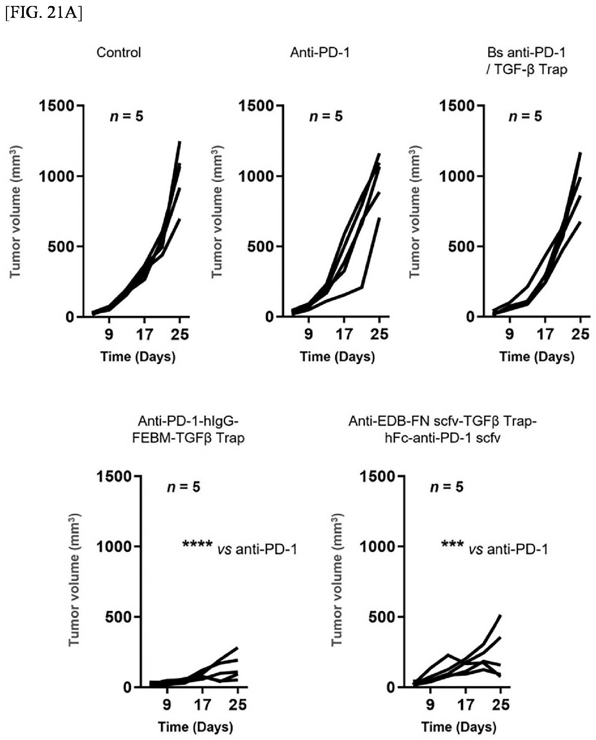

FIG. 21A shows the results of analyzing the tumor growth rate in a breast cancer orthotopic transplantation model by the administration of a trispecific Fc fusion protein including Anti-EDB-FN antibody, FEBM and TGFβ Trap produced according to one embodiment of the present invention. Each data means ± sem; *P < 0.01, ***P* < 0.05, ****P* <0.001; NS means not significant; and the results were analyzed by two-way ANOVA with post hoc Bonferroni t-test.

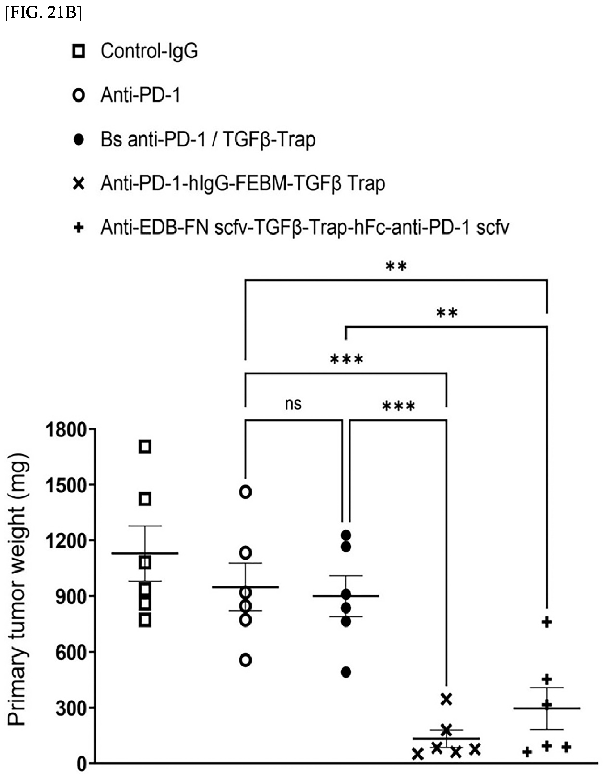

FIG. 21B shows the results of analyzing the tumor growth rate in a pancreatic cancer orthotopic transplantation model by the administration of a trispecific Fc fusion protein including Anti-EDB-FN antibody, FEBM and TGFβ Trap produced according to one embodiment of the present invention. Each data means ± sem; *P < 0.01, ***P* < 0.05, ****P* <0.001; NS means not significant; and the results were analyzed by two-way ANOVA with post hoc Bonferroni t-test.

### DETAILED DESCRIPTION OF THE INVENTION AND PREFERRED EMBODIMENTS

Unless otherwise defined, all technical and scientific terms used herein have the same meanings as commonly understood by one of ordinary skill in the art to which this invention pertains. In general, the nomenclature used herein and the experimental methods described below are well known and commonly used in the art.

In the present invention, it was attempted to confirm that a high anticancer effect is exhibited in various cancer types when a fusion protein that simultaneously binds to EDB-FN and TGFβ is used.

That is, in one embodiment of the present invention, when a fusion protein (Table 2) that simultaneously binds to EDB-FN and TGFβ is used, it was confirmed that excellent anticancer effects were observed in tumors with high (pancreatic cancer), low (melanoma) and intermediate (breast cancer) expression of EDB-FN (FIGS. 10 to 20).

Therefore, in one aspect,
the present invention relates to a fusion protein, including
(a) a polypeptide that specifically binds to extradomain B of fibronectin (EDB-FN); and
(b) a polypeptide that specifically binds to transforming growth factor β (TGFβ).

In the present invention, the fusion protein may further include (c) an antibody constant region.

In the present invention, the fusion protein including (a), (b) and (c) may include, from the N-terminus to the C-terminus,
one or more structures selected from the group consisting of:
(i) a polypeptide that specifically binds to EDB-FN; and a polypeptide that specifically binds to TGFβ;
(ii) a polypeptide that specifically binds to TGFβ; and a polypeptide that specifically binds to EDB-FN;
(iii) one or more polypeptides that specifically bind to EDB-FN; an antibody constant region; and one or more polypeptides that specifically bind to TGFβ;
(iv) one or more polypeptides that specifically bind to TGFβ; an antibody constant region; and one or more polypeptides that specifically bind to EDB-FN;
(v) one or more polypeptides that specifically bind to EDB-FN; one or more polypeptides that specifically bind to TGFβ; and an antibody constant region;
(vi) one or more polypeptides that specifically bind to EDB-FN; one or more polypeptides that specifically bind to TGFβ; an antibody constant region; and one or more polypeptides that specifically bind to EDB-FN;
(vii) one or more polypeptides that specifically bind to TGFβ; one or more polypeptides that specifically bind to EDB-FN; an antibody constant region; and one or more polypeptides that specifically bind to EDB-FN;
(viii) an antibody constant region; one or more polypeptides that specifically bind to EDB-FN; and one or more polypeptides that specifically bind to TGFβ;
(ix) an antibody constant region; one or more polypeptides that specifically bind to TGFβ; and one or more polypeptides that specifically bind to EDB-FN; and
(x) one or more polypeptides that specifically bind to EDB-FN; an antibody constant region; one or more polypeptides that specifically bind to TGFβ; and one or more polypeptides that specifically bind to EDB-FN;

In the present invention, the fusion protein includes, from the N-terminus to the C-terminus,
a structure selected from the group consisting of:
(7-1) an antibody constant region; a polypeptide that specifically binds to EDB-FN; and a polypeptide that specifically binds to TGFβ; and
(7-2) an antibody constant region; a polypeptide that specifically binds to TGFβ; and a polypeptide that specifically binds to EDB-FN.

In the present invention, the fusion protein may further include a polypeptide that specifically binds to (d) a tumor antigen.

In the present invention, the term "tumor antigen" means an antigenic (poly-)peptide or protein derived from or associated with a (preferably malignant) tumor or cancer disease. The terms "cancer" and "tumor" as used herein are used interchangeably to refer to a neoplasm characterized by uncontrolled and usually rapid proliferation of cells that tend to invade surrounding tissues and metastasize to distant body sites. The term includes benign and malignant neoplasms. Malignant neoplasms of cancer are typically characterized by anaplasia, invasiveness and metastasis; and benign neoplasms typically do not have these characteristics. The terms "cancer" and "tumor" refer in particular to neoplasms characterized by tumor growth, as well as cancers of the blood and lymphatic system. A "tumor antigen" is typically derived from a tumor/cancer cell, preferably a mammalian tumor/cancer cell, and may be located within or on a tumor cell, for example a systemic or solid tumor, derived from a mammal, preferably a mammalian, preferably a human. "Tumor antigens" generally include tumor-specific antigens (TSAs) and tumor-associated antigens (TAAs). TSAs are usually due to tumor-specific mutations and are specifically expressed by tumor cells. The more common TAAs are generally presented by tumor and "normal" (healthy, non-tumor) cells.

In the present invention, the tumor antigen may be 4-1BB, 5T4, integrin, Activin, Angiopoietin, angiopoietin-like 3, B cell maturation antigen (BCMA), B-cell activating factor (BAFF), BAGE-1, BCL-2, B7-H3, bcr/abl, beta-catenin/m, BING-4, BRCA1/m, BRCA2/m, CA 1 5-3/CA 27-29, CA 19-9, CA72-4, CA125, calreticulin, CAMEL, CASP-8/m, cathepsin B, cathepsin L, CCR4, CCR5, CCL11, CD11a, CD16A, CD19, CD2, CD20, CD22, CD25, CD3, CDE30, CD33, CD4, CD40, CD45, CD46, CD47, CD52, CD55, CD56, CD6, CD80, CD86, CTLA4, CD105, CD123, CD154, CD166, CD262, CD278, CD319, CD326, CDC27/m, CDK4/m, CDKN2A/m, CEA, CLCA2, CML28, CML66, COA-1/m, c-met, coactosin-like protein, collagen XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, HER2, HER3, HERVK-MEL, HLA-A*0201 - R1 7I, HLA-A1 1/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1 R, IL-13Ra2, IL-2R, IL-5, immature laminin receptor, kallikrein-2, kallikrein-4, i67, KIAA0205, KIAA0205/m, KK-LC- 1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B1 6, MAGE-B1 7, MAGE-C1, MAGE-C2, MAGE-C3, MAGE- D1, MAGED2, MAGE-D4, MAGE-E1, MAGE-E2, MAGE-F1, MAGE-H I, MAGEL2, mammaglobin A, MART-1/melan-A, MART-2, MART-2/m, matrix protein 22, MC1 R, M-CSF, ME 1/m, mesothelin, MG50/PXDN, MMP1 1, MN/CA IX-antigen, MRP-3, MUC-1, MUC-2, MUM-1/m, MUM-2/m, MUM-3/m, myosin class l/m, NA88-A, N-acetylglucosaminyltransferase- V, neo-PAP, neo-PAP/m, NFYC/m, NGEP, NMP22, NPM/ALK, N-Ras/m, NSE, NY-ESO-1, NY-ESOB, OA1, OFA-iLRP, OGT, OGT/m, OS-9, OS- 9/m, osteocalcin, osteopontin, pi 5, p190 minor ber-abl, p53, p53/m, PAGE-4, PAI-1, PAI-2, PAP, PART-1, PATE, PDEF, PD-1, PD-L1, Pim-1 -Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD1 68, RU1, RU2, S-100, SAGE, SART-1, SART-2, SART-3, SCC, SIRT2/m, Sp1 7, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGM-4, TPI/m, TRAG-3, TRG, TRP-1, TRP-2/6b, TRP/INT2, TRP-p8, T cell immunoglobulin and mucin-domain containing-3 (Tim-3), tissue factor, tissue factor pathway inhibitor (TFPI), tumor necrosis factor (TNF), tyrosinase, UPA, vascular endothelial growth factor (VEGF), VEGF receptor, vWF (von Willebrand Factor), VEGFR-2/FLK-1, WT1 and immunoglobulin genotypes of lymphocytes or T cell receptors of lymphocytes or homologs, fragments, variants or derivatives of the tumor antigen, and more preferably, it may be any one or more selected from the group consisting of PD-1, PD-L1, CTLA-4, CD80, CD86 and VEGF, and most preferably, it may be PD-1.

In the present invention, the polypeptide that specifically binds to (d) a tumor antigen may be a polypeptide that specifically binds to PD-1.

In the present invention, when the polypeptide that specifically binds to the (d) tumor antigen is an antibody, the antibody may be one or more selected from the group consisting of Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Tegoprubart, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Dinutuximab beta, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Fabezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociferlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Cinrebafusp alfa, Rozibafusp alfa, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab and Navicixizumab.

In the present invention, the fusion protein including (a), (b), (c) and (d) may include, from the N-terminus to the C-terminus,
one or more structures selected from the group consisting of:
(A) a polypeptide that specifically binds to PD-1; a polypeptide that specifically binds to EDB-FN; and a polypeptide that specifically binds to TGFβ;
(B) a polypeptide that specifically binds to PD-1; a polypeptide that specifically binds to TGFβ; and a polypeptide that specifically binds to EDB-FN;
(C) a polypeptide that specifically binds to TGFβ; a polypeptide that specifically binds to EDB-FN; and a polypeptide that specifically binds to PD-1;
(D) a polypeptide that specifically binds to PD-1; an antibody constant region; a polypeptide that specifically binds to EDB-FN; and a polypeptide that specifically binds to TGFβ;
(E) a polypeptide that specifically binds to PD-1; an antibody constant region; a polypeptide that specifically binds to TGFβ; and a polypeptide that specifically binds to EDB-FN;
(F) a polypeptide that specifically binds to TGFβ; a polypeptide that specifically binds to EDB-FN; a polypeptide that specifically binds to PD-1; an antibody constant region; and
(G) a polypeptide that specifically binds to EDB-FN; a polypeptide that specifically binds to TGFβ; a polypeptide that specifically binds to PD-1; and an antibody constant region.

In the present invention, each component of the fusion protein may be linked via a linker.

In the present invention, the linker may be a peptide linker and may have a length of about 1 to 25 aa. For example, it may include a hydrophilic amino acid such as glycine and/or serine, but is not limited thereto.

Specifically, the linker may include, for example, GGG, (GS)ₙ, (GGS)ₙ, (GGGGS)ₙ, (SSSSG)ₙ or (GₙS)ₙ, (where n and m are each 1 to 10), which impart structural flexibility without being cleaved by proteolytic enzymes, and more specifically, the linker may be, for example, GGG, (GGGGS)ₙ or (SSSG)ₙ (where n and m are each 1 to 10).

In the present invention, the polypeptide that specifically binds to EDB-FN may be a fibronectin EDB binding motif (FEBM), an antibody that specifically binds to EDB-FN or a fragment thereof.

The term "antibody" as used herein means an antibody that specifically binds to a target substance (e.g., EDB-FN, TGFβ and PD-1).

A complete antibody is a structure having two full-length light chains and two full-length heavy chains, and each light chain is linked to a heavy chain by a disulfide bond.

As used herein, the term "heavy chain" refers to both a full-length heavy chain and fragments thereof, including a variable region domain VH including an amino acid sequence having sufficient variable region sequence to confer specificity to an antigen, and three constant region domains CH1, CH2 and CH3. In addition, the term "light chain" as used herein refers to both a full-length light chain and fragments thereof, including a variable region domain VL including an amino acid sequence having sufficient variable region sequence to confer specificity to an antigen, and a constant region domain CL.

The whole antibodies include subtypes of IgA, IgD, IgE, IgM and IgG, and in particular, IgG includes IgG1, IgG2, IgG3 and IgG4. The heavy-chain constant region has gamma (γ), mu (µ), alpha (α), delta (δ) and epsilon (ε) types, and has gamma 1 (γ1), gamma 2 (γ2), gamma 3 (γ3), gamma 4 (γ4), alpha 1 (α1) and alpha 2 (α2) as subclasses. The light-chain constant region has kappa (κ) and lambda (λ) types.

An antigen-binding fragment of an antibody, or antibody fragment, refers to a fragment that has an antigen-binding function, and includes Fab, F(ab'), F(ab')₂ and Fv. Among antibody fragments, Fab has a structure having a variable region of the light chain and heavy chain, a constant region of the light chain, and a first constant region (CH1) of the heavy chain, and has one antigen-binding site. Fab' differs from Fab in that it has a hinge region including one or more cysteine residues at the C-terminus of the heavy chain CH1 domain. F(ab')₂ is generated when the cysteine residues in the hinge region of Fab' form a disulfide bond.

Fv corresponds to the smallest antibody fragment that has only a heavy chain variable region and a light chain variable region. A two-chain Fv has a heavy chain variable region and a light chain variable region non-covalently linked, and a single-chain Fv (scFv) has a heavy chain variable region and a light chain variable region covalently linked, usually via a peptide linker or directly linked at the C-terminus, such that it can form a dimer-like structure like a two-chain Fv. These antibody fragments may be produced using proteolytic enzymes (e.g., Fab can be obtained by restriction digestion of a complete antibody with papain, and F(ab')₂ can be obtained by digestion with pepsin) or using genetic recombination technology.

The "Fv" fragment is an antibody fragment that contains a complete antibody recognition and binding site. This region is a dimer of one heavy chain variable domain and one light chain variable domain.

The "Fab" fragment includes the variable and constant domains of the light chain. and the variable and first constant domains (CH1) of the heavy chain. An F(ab')₂ antibody fragment typically includes a pair of Fab' fragments covalently linked by cysteine in the hinge region at the C-terminus of the Fab' fragment.

A "single-chain Fv (scFv)" antibody fragment is a construct consisting of a single polypeptide chain including the VH and VL domains of an antibody. The scFv may further include a polypeptide linker between the VH and VL domains that enables it to form the desired structure for antigen binding.

In one embodiment, the antibody of the present invention includes, but are not limited to, monoclonal antibodies, multispecific antibodies, human antibodies, humanized antibodies, chimeric antibodies, scFv, Fab fragments, F(ab')₂ fragments, disulfide-linked Fvs (sdFv) and anti-idiotypic (anti-Id) antibodies, or epitope-binding fragments of the antibodies.

The heavy-chain constant region may be selected from any one of the isotypes of gamma (γ), mu (µ), alpha (α), delta (δ) or epsilon (ε). For example, the constant region is gamma 1 (IgG1), gamma 2 (IgG2), gamma 3 (IgG3) or gamma 4 (IgG4). The light-chain constant region may be kappa or lambda type.

In the present invention, the polypeptide that specifically binds to TGFβ may be a TGFβ receptor type 2 ectodomain (TGFβRII ectodomain, TGFβ Trap), an antibody that specifically binds to TGFβ or a fragment thereof.

In the present invention, the polypeptide that specifically binds to TGFβ may be any one or more of polypeptides represented by amino acid sequences of SEQ ID NOs: 5 to 8.

In the present invention, the antibody constant region may be a heavy-chain constant region or a light-chain constant region.

In the present invention, the antibody constant region may any one or more of polypeptides represented by amino acid sequences of SEQ ID NOs: 12 to 15.

In the present invention, the polypeptide that specifically binds to PD-1 may be an antibody that specifically binds to PD-1or a fragment thereof.

In the present invention, the polypeptide that specifically binds to PD-1 may be any one or more of polypeptides represented by amino acid sequences of SEQ ID NOs: 9 to 11.

In the present invention, the fusion protein including (a), (b) and (c) may include, from the N-terminus to the C-terminus, any one or more polypeptides selected from the group consisting of:
(4-1) a polypeptide of SEQ ID NO: 5; a polypeptide of SEQ ID NO: 12 and a polypeptide of SEQ ID NO: 1;
(4-2) a polypeptide of SEQ ID NO: 5; a polypeptide of SEQ ID NO: 1; and a polypeptide of SEQ ID NO: 12;
(4-3) a polypeptide of SEQ ID NO: 1; a polypeptide of SEQ ID NO: 5; and a polypeptide of SEQ ID NO: 12;
(4-4) a polypeptide of SEQ ID NO: 1; a polypeptide of SEQ ID NO: 12; and a polypeptide of SEQ ID NO: 5;
(4-5) a polypeptide of SEQ ID NO: 12; a polypeptide of SEQ ID NO: 1; and a polypeptide of SEQ ID NO: 5;
(4-6) a polypeptide of SEQ ID NO: 12; a polypeptide of SEQ ID NO: 5; and a polypeptide of SEQ ID NO: 1;
(4-7) a polypeptide of SEQ ID NO: 1; a polypeptide of SEQ ID NO: 5; a polypeptide of SEQ ID NO: 1; and a polypeptide of SEQ ID NO: 12;
(4-8) a polypeptide of SEQ ID NO: 12; a polypeptide of SEQ ID NO: 1; a polypeptide of SEQ ID NO: 5; and a polypeptide of SEQ ID NO: 1;
(4-9) a polypeptide of SEQ ID NO: 1; a polypeptide of SEQ ID NO: 5; a polypeptide of SEQ ID NO: 1; a polypeptide of SEQ ID NO: 12; and a polypeptide of SEQ ID NO: 1;
(4-10) a polypeptide of SEQ ID NO: 1; a polypeptide of SEQ ID NO: 12; a polypeptide of SEQ ID NO: 1; a polypeptide of SEQ ID NO: 5; and a polypeptide of SEQ ID NO: 1;
(4-11) a polypeptide of SEQ ID NO: 5; a polypeptide of SEQ ID NO: 1; and a polypeptide of SEQ ID NO: 13;
(4-12) a polypeptide of SEQ ID NO: 13; a polypeptide of SEQ ID NO: 1; and a polypeptide of SEQ ID NO: 5;
(4-13) a polypeptide of SEQ ID NO: 2; a polypeptide of SEQ ID NO: 14; and a polypeptide of SEQ ID NO: 5;
(4-14) a polypeptide of SEQ ID NO: 3; a polypeptide of SEQ ID NO: 15; a polypeptide of SEQ ID NO: 5;
(4-15) a polypeptide of SEQ ID NO: 5; a polypeptide of SEQ ID NO: 2; and a polypeptide of SEQ ID NO: 13;
(4-16) a polypeptide of SEQ ID NO: 4; a polypeptide of SEQ ID NO: 12; and a polypeptide of SEQ ID NO: 5;
(4-17) a polypeptide of SEQ ID NO: 12; a polypeptide of SEQ ID NO: 4; and a polypeptide of SEQ ID NO: 5;
(4-18) a polypeptide of SEQ ID NO: 4; a polypeptide of SEQ ID NO: 5; and a polypeptide of SEQ ID NO: 12;
(4-19) a polypeptide of SEQ ID NO: 12; a polypeptide of SEQ ID NO: 5; and a polypeptide of SEQ ID NO: 4;
(4-20) a polypeptide of SEQ ID NO: 4; and a polypeptide of SEQ ID NO: 5;
(4-21) a polypeptide of SEQ ID NO: 6; a polypeptide of SEQ ID NO: 14; and a polypeptide of SEQ ID NO: 1;
(4-22) a polypeptide of SEQ ID NO: 7; a polypeptide of SEQ ID NO: 15; and a polypeptide of SEQ ID NO: 1;
(4-23) a polypeptide of SEQ ID NO: 1; a polypeptide of SEQ ID NO: 6; and a polypeptide of SEQ ID NO: 14;
(4-24) a polypeptide of SEQ ID NO: 2; a polypeptide of SEQ ID NO: 14; and a polypeptide of SEQ ID NO: 5;
(4-25) a polypeptide of SEQ ID NO: 1; a polypeptide of SEQ ID NO: 8; and a polypeptide of SEQ ID NO: 12;
(4-26) a polypeptide of SEQ ID NO: 12; a polypeptide of SEQ ID NO: 1; and a polypeptide of SEQ ID NO: 8;
(4-27) a polypeptide of SEQ ID NO: 12; a polypeptide of SEQ ID NO: 8; and a polypeptide of SEQ ID NO: 1;
(4-28) a polypeptide of SEQ ID NO: 8; and a polypeptide of SEQ ID NO: 1;
(4-29) a polypeptide of SEQ ID NO: 1; and a polypeptide of SEQ ID NO: 8;
(4-30) a polypeptide of SEQ ID NO: 2; a polypeptide of SEQ ID NO: 14; and a polypeptide of SEQ ID NO: 8;
(4-31) a polypeptide of SEQ ID NO: 8; a polypeptide of SEQ ID NO: 14; and a polypeptide of SEQ ID NO: 4;
(4-32) a polypeptide of SEQ ID NO: 8; a polypeptide of SEQ ID NO: 2; and a polypeptide of SEQ ID NO: 14;
(4-33) a polypeptide of SEQ ID NO: 4; a polypeptide of SEQ ID NO: 6; and a polypeptide of SEQ ID NO: 14;
(4-34) a polypeptide of SEQ ID NO: 4; a polypeptide of SEQ ID NO: 8; and a polypeptide of SEQ ID NO: 12;
(4-35) a polypeptide of SEQ ID NO: 8; a polypeptide of SEQ ID NO: 4; and a polypeptide of SEQ ID NO: 12;
(4-36) a polypeptide of SEQ ID NO: 12; a polypeptide of SEQ ID NO: 4; and a polypeptide of SEQ ID NO: 8;
(4-37) a polypeptide of SEQ ID NO: 12; a polypeptide of SEQ ID NO: 8; and a polypeptide of SEQ ID NO: 4;
(4-38) a polypeptide of SEQ ID NO: 4; and a polypeptide of SEQ ID NO: 8; and
(4-39) a polypeptide of SEQ ID NO: 8; and a polypeptide of SEQ ID NO: 4, and
more preferably, it may include (4-5) a polypeptide of SEQ ID NO: 12; a polypeptide of SEQ ID NO: 1; and a polypeptide of SEQ ID NO: 5; or (4-6) a polypeptide of SEQ ID NO: 12; a polypeptide of SEQ ID NO: 5; and a polypeptide of SEQ ID NO: 1;

In the present invention, the fusion protein including (a), (b) and (c) may be any one or more selected from the group consisting of SEQ ID NOs: 16 to 56, and more preferably, it may be represented by an amino acid sequence of SEQ ID NO: 20, or represented by an amino acid sequence of SEQ ID NO: 21.

In the present invention, the fusion protein including (a), (b), (c) and (d) may include, from the N-terminus to the C-terminus,
any one or more polypeptides selected from the group consisting of:
(5-1) a polypeptide of SEQ ID NO: 9; a polypeptide of SEQ ID NO: 14; a polypeptide of SEQ ID NO: 1; and a polypeptide of SEQ ID NO: 5;
(5-2) a polypeptide of SEQ ID NO: 9; a polypeptide of SEQ ID NO: 14; a polypeptide of SEQ ID NO: 5; and a polypeptide of SEQ ID NO: 1;
(5-3) a polypeptide of SEQ ID NO: 10; a polypeptide of SEQ ID NO: 15; a polypeptide of SEQ ID NO: 1; and a polypeptide of SEQ ID NO: 5;
(5-4) a polypeptide of SEQ ID NO: 10; a polypeptide of SEQ ID NO: 15; a polypeptide of SEQ ID NO: 5; and a polypeptide of SEQ ID NO: 1;
(5-5) a polypeptide of SEQ ID NO: 5; a polypeptide of SEQ ID NO: 1; a polypeptide of SEQ ID NO: 9; and a polypeptide of SEQ ID NO: 14;
(5-6) a polypeptide of SEQ ID NO: 1; a polypeptide of SEQ ID NO: 5; a polypeptide of SEQ ID NO: 9; and a polypeptide of SEQ ID NO: 14;
(5-7) a polypeptide of SEQ ID NO: 11; a polypeptide of SEQ ID NO: 12; a polypeptide of SEQ ID NO: 1; and a polypeptide of SEQ ID NO: 5;
(5-8) a polypeptide of SEQ ID NO: 11; a polypeptide of SEQ ID NO: 12; a polypeptide of SEQ ID NO: 5; and a polypeptide of SEQ ID NO: 1;
(5-9) a polypeptide of SEQ ID NO: 5; a polypeptide of SEQ ID NO: 1; a polypeptide of SEQ ID NO: 12; and a polypeptide of SEQ ID NO: 11;
(5-10) a polypeptide of SEQ ID NO: 11; a polypeptide of SEQ ID NO: 1; and a polypeptide of SEQ ID NO: 5;
(5-11) a polypeptide of SEQ ID NO: 11; a polypeptide of SEQ ID NO: 5; and a polypeptide of SEQ ID NO: 1;
(5-12) a polypeptide of SEQ ID NO: 5; a polypeptide of SEQ ID NO: 1; and a polypeptide of SEQ ID NO: 11;
(5-13) a polypeptide of SEQ ID NO: 9; a polypeptide of SEQ ID NO: 14; a polypeptide of SEQ ID NO: 4; and a polypeptide of SEQ ID NO: 5;
(5-14) a polypeptide of SEQ ID NO: 9; a polypeptide of SEQ ID NO: 14; a polypeptide of SEQ ID NO: 5; and a polypeptide of SEQ ID NO: 4;
(5-15) a polypeptide of SEQ ID NO: 10; a polypeptide of SEQ ID NO: 15; a polypeptide of SEQ ID NO: 4; and a polypeptide of SEQ ID NO: 5;
(5-16) a polypeptide of SEQ ID NO: 10; a polypeptide of SEQ ID NO: 15; a polypeptide of SEQ ID NO: 5; and a polypeptide of SEQ ID NO: 4;
(5-17) a polypeptide of SEQ ID NO: 4; a polypeptide of SEQ ID NO: 5; a polypeptide of SEQ ID NO: 9; and a polypeptide of SEQ ID NO: 14;
(5-18) a polypeptide of SEQ ID NO: 5; a polypeptide of SEQ ID NO: 4; a polypeptide of SEQ ID NO: 9; and a polypeptide of SEQ ID NO: 14;
(5-19) a polypeptide of SEQ ID NO: 11; a polypeptide of SEQ ID NO: 12; a polypeptide of SEQ ID NO: 4; and a polypeptide of SEQ ID NO: 5;
(5-20) a polypeptide of SEQ ID NO: 11; a polypeptide of SEQ ID NO: 12; a polypeptide of SEQ ID NO: 5; and a polypeptide of SEQ ID NO: 4;
(5-21) a polypeptide of SEQ ID NO: 4; a polypeptide of SEQ ID NO: 5; a polypeptide of SEQ ID NO: 12; and a polypeptide of SEQ ID NO: 11;
(5-22) a polypeptide of SEQ ID NO: 11; a polypeptide of SEQ ID NO: 4; and a polypeptide of SEQ ID NO: 5;
(5-23) a polypeptide of SEQ ID NO: 11; a polypeptide of SEQ ID NO: 5; and a polypeptide of SEQ ID NO: 4;
(5-24) a polypeptide of SEQ ID NO: 5; a polypeptide of SEQ ID NO: 11; and a polypeptide of SEQ ID NO: 4;
(5-25) a polypeptide of SEQ ID NO: 9; a polypeptide of SEQ ID NO: 14; a polypeptide of SEQ ID NO: 1; and a polypeptide of SEQ ID NO: 8;
(5-26) a polypeptide of SEQ ID NO: 9; a polypeptide of SEQ ID NO: 14; a polypeptide of SEQ ID NO: 8; and a polypeptide of SEQ ID NO: 1;
(5-27) a polypeptide of SEQ ID NO: 10; a polypeptide of SEQ ID NO: 15; a polypeptide of SEQ ID NO: 1; and a polypeptide of SEQ ID NO: 8;
(5-28) a polypeptide of SEQ ID NO: 10; a polypeptide of SEQ ID NO: 15; a polypeptide of SEQ ID NO: 8; and a polypeptide of SEQ ID NO: 1;
(5-29) a polypeptide of SEQ ID NO: 8; a polypeptide of SEQ ID NO: 1; a polypeptide of SEQ ID NO: 9; and a polypeptide of SEQ ID NO: 14;
(5-30) a polypeptide of SEQ ID NO: 1; a polypeptide of SEQ ID NO: 8; a polypeptide of SEQ ID NO: 9; and a polypeptide of SEQ ID NO: 14;
(5-31) a polypeptide of SEQ ID NO: 11; a polypeptide of SEQ ID NO: 12; a polypeptide of SEQ ID NO: 1; and a polypeptide of SEQ ID NO: 8;
(5-32) a polypeptide of SEQ ID NO: 11; a polypeptide of SEQ ID NO: 12; a polypeptide of SEQ ID NO: 8; and a polypeptide of SEQ ID NO: 1;
(5-33) a polypeptide of SEQ ID NO: 8; a polypeptide of SEQ ID NO: 1; a polypeptide of SEQ ID NO: 12; and a polypeptide of SEQ ID NO: 11;
(5-34) a polypeptide of SEQ ID NO: 11; a polypeptide of SEQ ID NO: 1; and a polypeptide of SEQ ID NO: 8;
(5-35) a polypeptide of SEQ ID NO: 11; a polypeptide of SEQ ID NO: 8; and a polypeptide of SEQ ID NO: 1;
(5-36) a polypeptide of SEQ ID NO: 8; a polypeptide of SEQ ID NO: 1; and a polypeptide of SEQ ID NO: 11;
(5-37) a polypeptide of SEQ ID NO: 9; a polypeptide of SEQ ID NO: 14; a polypeptide of SEQ ID NO: 4; and a polypeptide of SEQ ID NO: 8;
(5-38) a polypeptide of SEQ ID NO: 9; a polypeptide of SEQ ID NO: 14; a polypeptide of SEQ ID NO: 8; and a polypeptide of SEQ ID NO: 4;
(5-39) a polypeptide of SEQ ID NO: 10; a polypeptide of SEQ ID NO: 15; a polypeptide of SEQ ID NO: 4; and a polypeptide of SEQ ID NO: 8;
(5-40) a polypeptide of SEQ ID NO: 10; a polypeptide of SEQ ID NO: 15; a polypeptide of SEQ ID NO: 8; and a polypeptide of SEQ ID NO: 4;
(5-41) a polypeptide of SEQ ID NO: 4; a polypeptide of SEQ ID NO: 8; a polypeptide of SEQ ID NO: 9; and a polypeptide of SEQ ID NO: 14;
(5-42) a polypeptide of SEQ ID NO: 8; a polypeptide of SEQ ID NO: 4; a polypeptide of SEQ ID NO: 9; and a polypeptide of SEQ ID NO: 14;
(5-43) a polypeptide of SEQ ID NO: 11; a polypeptide of SEQ ID NO: 12; a polypeptide of SEQ ID NO: 4; and a polypeptide of SEQ ID NO: 8;
(5-44) a polypeptide of SEQ ID NO: 11; a polypeptide of SEQ ID NO: 12; a polypeptide of SEQ ID NO: 8; and a polypeptide of SEQ ID NO: 4;
(5-45) a polypeptide of SEQ ID NO: 4; a polypeptide of SEQ ID NO: 8; a polypeptide of SEQ ID NO: 12; and a polypeptide of SEQ ID NO: 11;
(5-46) a polypeptide of SEQ ID NO: 11; a polypeptide of SEQ ID NO: 4; and a polypeptide of SEQ ID NO: 8;
(5-47) a polypeptide of SEQ ID NO: 11; a polypeptide of SEQ ID NO: 8; and a polypeptide of SEQ ID NO: 4; and
(5-48) a polypeptide of SEQ ID NO: 8; a polypeptide of SEQ ID NO: 4; and a polypeptide of SEQ ID NO: 11.

In the present invention, the fusion protein including (a), (b), (c) and (d) may be any one selected from the group consisting of SEQ ID NOs: 57 to 104.

The fusion protein of the present invention may include not only the sequence of the fusion protein of the present invention described herein, but also biological equivalents thereof, within the scope of specifically recognizing EDB-FN and TGFβ. For example, additional changes may be made to the amino acid sequence of the protein to further improve the binding affinity and/or other biological properties of the protein. Such changes may include, for example, deletions, insertions and/or substitutions of amino acid sequence residues of the protein. Such amino acid mutations are made based on the relative similarity of the amino acid side chain substituents, such as hydrophobicity, hydrophilicity, charge, size and the like. Analysis of the size, shape and type of the amino acid side chain substituents reveals that arginine, lysine and histidine are all positively charged residues; alanine, glycine and serine have similar sizes; and phenylalanine, tryptophan and tyrosine have similar shapes. Accordingly, based on these considerations, arginine, lysine and histidine; alanine, glycine and serine; and phenylalanine, tryptophan and tyrosine are biologically functional equivalents.

Considering the mutation having the above-described biological equivalent activity, the fusion protein of the present invention or the nucleic acid molecule encoding the same is interpreted to also include a sequence showing substantial identity with the sequence described in the sequence number. The substantial identity means a sequence showing at least 90% homology, most preferably at least 95% homology, 96% or more, 97% or more, 98% or more, or 99% or more homology when the sequence of the present invention and any other sequence are aligned to the greatest extent possible and the aligned sequence is analyzed using an algorithm commonly used in the art. Alignment methods for sequence comparison are known in the art. The NCBI Basic Local Alignment Search Tool (BLAST) is accessible from NBCI, and the like, and may be used in conjunction with sequence analysis programs such as blastp, blasm, blastx, tblastn and tblastx on the Internet. BLAST is accessible at www.ncbi.nlm.nih.gov/BLAST/. Methods for comparing sequence homology using this program can be found at www.ncbi.nlm.nih.gov/BLAST/blast_ help.html.

Based on this, the fusion protein of the present invention may have 90%, 91%, 92%, 93%, 94%, 95%, 96%, 97%, 98%, 99%, or more homology to the specified sequences or all of them described in the specification. Such homology may be determined by sequence comparison and/or alignment by methods known in the art. For example, the percent sequence homology of the nucleic acids or proteins of the present invention may be determined using a sequence comparison algorithm (i.e., BLAST or BLAST 2.0), manual alignment, or visual inspection.

In another aspect, the present invention relates to a nucleic acid encoding the fusion protein. The nucleic acid encoding the fusion protein of the present invention may be isolated to recombinantly produce the fusion protein.

"Nucleic acid" has a comprehensive meaning including DNA (gDNA and cDNA) and RNA molecules, and nucleotides, which are the basic structural units of nucleic acids, include not only natural nucleotides but also analogues in which sugar or base moieties are modified. The sequence of a nucleic acid encoding the fusion protein of the present invention may be modified. The modifications include addition, deletion or non-conservative or conservative substitution of nucleotides.

The DNA encoding the above fusion protein may be readily isolated or synthesized using conventional molecular biological techniques (e.g., by using an oligonucleotide probe capable of specifically binding to DNA encoding the antibody and the heavy and light chains), and the nucleic acid may be isolated and further cloned (amplification of the DNA) or further expressed by inserting the same into a replicable vector.

Based on this, in still another aspect, the present invention relates to a recombinant expression vector including the nucleic acid.

The term "vector" as used herein refers to a means for expressing a target gene in a host cell, and includes viral vectors such as plasmid vectors, cosmid vectors, bacteriophage vectors, adenovirus vectors, retrovirus vectors, and adeno-associated virus vectors. Components of a vector typically include, but are not limited to, one or more of the following: a signal sequence, an origin of replication, one or more antibiotic resistance marker genes, an enhancer element, a promoter, and a transcription termination sequence. A nucleic acid encoding a fusion protein is operably linked to the promoter and the transcription termination sequence.

"Operatively linked" means a functional linkage between a nucleic acid expression regulatory sequence (e.g., a promoter, a signal sequence, or an array of transcription factor binding sites) and another nucleic acid sequence, whereby the regulatory sequence regulates transcription and/or translation of the other nucleic acid sequence.

In the case of a prokaryotic host, it usually includes a strong promoter capable of driving transcription (e.g., tac promoter, lac promoter, lacUV5 promoter, 1pp promoter, pLλ promoter, pRλ promoter, rac5 promoter, amp promoter, recA promoter, SP6 promoter, trp promoter, and T7 promoter), a ribosome binding site for translation initiation and a transcription/translation termination sequence. In addition, for example, when a eukaryotic cell is used as a host, promoters derived from the genome of mammalian cells (e.g., metallothionine promoter, β-actin promoter, human hemoglobin promoter, and human muscle creatine promoter) or promoters derived from mammalian viruses (e.g., adenovirus late promoter, vaccinia virus 7.5K promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), and promoter of Rous sarcoma virus (RSV)) may be used, which generally have a polyadenylation sequence as a transcription termination sequence.

In some cases, the vector may be fused with other sequences to facilitate purification of the fusion protein expressed therefrom. Sequences to be fused include, for example, glutathione S-transferase (Pharmacia, USA), maltose binding protein (NEB, USA), FLAG (IBI, USA), and 6x His (hexahistidine) (Quiagen, Germany).

The vector includes antibiotic resistance genes commonly used in the art as selectable markers, and for example, there are resistance genes for ampicillin, gentamicin, carbenicillin, chloramphenicol, streptomycin, kanamycin, geneticin, neomycin and tetracycline.

In still another aspect, the present invention relates to a host cell transfected with the recombinant expression vector. The host cell used to produce the fusion protein of the present invention may be, but is not limited to, a prokaryotic, yeast or higher eukaryotic cell.

Prokaryotic host cells may be used, such as strains of the genus *Bacillus,* such as *Escherichia coli, Bacillus subtilis and Bacillus thuringiensis,* and *Streptomyces, Pseudomonas* (e.g., *Pseudomonas putida), Proteus mirabilis* and *Staphylococcus* (e.g., *Staphylocus carnosus).*

However, animal cells are of greatest interest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S or HT1080.

In still another aspect, the present invention relates to a method for producing a fusion protein, comprising culturing the host cell to produce a fusion protein; and isolating and purifying the produced fusion protein.

The host cell may be cultured in various media. Any commercially available media may be used as a culture medium without limitation. Any other necessary supplements known to those skilled in the art may be included in appropriate concentrations. Culture conditions, such as temperature, pH and the like, are already used with the host cell selected for expression and will be apparent to those skilled in the art.

The fusion protein may be recovered by removing impurities, for example, by centrifugation or ultrafiltration, and the resultant product may be purified, for example, using affinity chromatography. Additional purification techniques may be used, for example, anion or cation exchange chromatography, hydrophobic interaction chromatography, hydroxyapatite chromatography and the like.

In still another aspect, the present invention relates to a composition for preventing or treating cancer, including the fusion protein.

In the present invention, the cancer may be selected from the group consisting of squamous cell carcinoma, small cell lung cancer, non-small cell lung cancer, adenocarcinoma of the lung, squamous cell carcinoma of the lung, peritoneal cancer, skin cancer, cutaneous or intraocular melanoma, rectal cancer, anal cancer, esophageal cancer, small intestinal cancer, endocrine cancer, parathyroid cancer, adrenal cancer, soft tissue sarcoma, urethral cancer, chronic or acute leukemia, lymphocytic lymphoma, hepatocellular carcinoma, gastrointestinal cancer, pancreatic cancer, glioblastoma, cervical cancer, ovarian cancer, liver cancer, bladder cancer, hepatoma, breast cancer, colon cancer, large intestine cancer, endometrial or uterine cancer, salivary gland cancer, kidney cancer, liver cancer, prostate cancer, vulvar cancer, thyroid cancer, liver cancer, and head and neck cancer.

The term "prevention" as used in the present invention means any act of suppressing or delaying the onset of an immune disease or an inflammatory disease by administering the pharmaceutical composition of the present invention.

The term "treatment" as used in the present invention means any act in which the symptoms of an immune disease or an inflammatory disease are improved or beneficially changed by administration of the pharmaceutical composition of the present invention.

In the present invention, the composition may include a pharmaceutically acceptable carrier. The pharmaceutically acceptable carrier included in the composition is one commonly used in formulation, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil. In addition to the above components, the pharmaceutical composition may further include a lubricant, a wetting agent, a sweetening agent, a flavoring agent, an emulsifier, a suspending agent, a preservative and the like.

The appropriate dosage of the pharmaceutical composition for preventing or treating cancer may be prescribed in various ways depending on factors such as the formulation method, administration method, patient's age, weight, gender, pathological condition, food, administration time, administration route, excretion rate and response sensitivity. The preferred dosage of the composition is within the range of 0.001-100 mg/kg for adults. The term "pharmaceutically effective amount" means an amount sufficient to prevent or treat cancer, or prevent or treat a disease caused by angiogenesis.

The composition may be prepared in a unit dose form or may be prepared by introducing the same into a multi-dose container by formulating the same using a pharmaceutically acceptable carrier and/or excipient according to a method that can be easily performed by a person skilled in the art. In this case, the formulation may be in the form of a solution, suspension, syrup or emulsion in an oil or aqueous medium, or may be in the form of an extract, powder, granule, tablet or capsule, and may additionally contain a dispersant or a stabilizer. In addition, the composition may be administered as an individual therapeutic agent or in combination with another therapeutic agent, and may be administered sequentially or simultaneously with conventional therapeutic agents. Meanwhile, since the composition includes an antibody or an antigen-binding fragment, it may be formulated as an immunoliposome. A liposome including an antibody may be prepared according to a method widely known in the art. The immunoliposome may be prepared by a reverse phase evaporation method as a lipid composition including phosphatidylcholine, cholesterol and polyethylene glycol-derivatized phosphatidylethanolamine. For example, the Fab' fragment of an antibody may be conjugated to liposomes via a disulfide-exchange reaction. A chemotherapeutic agent such as doxorubicin may be additionally included within the liposomes.

In still another aspect, the present invention relates to a composition for treating cancer, including the fusion protein as an active ingredient.

In still another aspect, the present invention relates to a method for treating cancer, including administering the fusion protein.

In still another aspect, the present invention relates to the use of the fusion protein in the manufacture of a medicament for treating cancer.

In still another aspect, the present invention relates to the use of a fusion protein including a) a polypeptide that specifically binds to extradomain B of fibronectin (EDB-FN); and (b) a polypeptide that specifically binds to transforming growth factor β (TGFβ).

In still another aspect, the present invention relates to the use of a fusion protein including a) a polypeptide that specifically binds to extradomain B of fibronectin (EDB-FN); and (b) a polypeptide that specifically binds to transforming growth factor β (TGFβ).

In still another aspect, the present invention relates to the combined therapeutic use of a fusion protein including a) a polypeptide that specifically binds to extradomain B of fibronectin (EDB-FN); and (b) a polypeptide that specifically binds to transforming growth factor β (TGFβ).

### Example

Hereinafter, the present invention will be described in more detail through examples. It will be apparent to those skilled in the art that these examples are intended only to illustrate the present invention, and the scope of the present invention is not to be construed as being limited by these examples.

### Example 1. Materials and methods

### 1-1. Fibronection transcriptome expression and prognosis analysis

TCGA or GTEX database analysis was performed by downloading the exon mRNA level including the EDB domain of FN1 (Fibronectin 1) through gepia2 ((http:/gepia2.cancer-pku.cn/#index) to obtain data, and then analyzing the same by utilizing TCGA (human cancer genome/transcriptome and cancer patient information data resource) and GTEX (normal tissue genome/transcriptome information data resource) according to the user manual of gepia2.

### 1-2. Culture and expression analysis of orthotopically transplanted tumor tissue and normal cells

The expression levels of FN1-EDB+ transcripts were compared in 11 different orthotopically transplanted tumor tissues and normal tissues.

That is, Renca murine renal adenocarcinoma (RENCA, kidney cancer), Colon 26 murine colorectal cancer (Colon26, colon cancer), 4T1-Luc murine breast cancer (4T-1, breast cancer), B16F10-Luc murine melanoma (B16F10, melanoma), Hepa6 murine hepatoma (Hepa6, liver cancer), and RM1 murine prostate cancer (RM1, prostate cancer) cell lines were purchased from the American Type Culture Collection (ATCC, USA). The GL26 murine glioblastoma (brain tumor) cell line was obtained from Dr. Henry Brem of the Johns Hopkins Medical School. The YTN-16 murine gastric cell line (YTN16, stomach cancer) was obtained from Professor Sachiyo Nomura of the University of Tokyo. The Panc02 murine pancreatic adenocarcinoma cell line (PANC02, pancreatic cancer) was obtained from Chungnam National University. KRasG12D/+TP53-/- PLC (Primary lung cancer cell lines derived from spontaneous Kras/p53 GEMMs, lung cancer), KRasG12D/+TP53-/- PSC (Primary sarcoma cell lines derived from spontaneous Kras/p53 GEMMs, sarcoma) and TP53-/- PTLC (Primary T lymphoma cell lines derived from spontaneous p53 GEMMs, lymphoma) cell lines were obtained from primary tumors obtained from KRasG12D/+TP53-/-mice provided by the Howard Hughes Medical Institute *(*Nature. 2001, 26;410(6832):1111-6.). C57BL/6 mice and BALB/c mice (Orient Bio, Korea) were purchased for the recovery of normal tissues.

4T-1-Luc cells were transplanted into the mammary fat pad of BALB/c mice (OrientBio, Korea), and one month later, lung metastatic nodules were collected and digested into single cells. This process was repeated twice to isolate metastatic cancer cells called 4T-1-LMT-2-Luc (a cancer cell line with high lung metastasis potential), which were then cultured *in vitro* and used to create an allograft mouse model.

4T1, 4T-1-Luc and 4T-1-LMT-2-Luc cell lines were cultured in R10 medium (RPMI1640 (Welgene, Korea) containing penicillin/streptomycin (Welgene, Korea) and 10% heat-inactivated fetal bovine serum (Welgene, Korea)). Panc02-luc, Panc02, B16F10-luc, B16F10, colon 26, Hepa6, GL26, RM1 and Renca cell lines were cultured in Dulbecco's Modified Eagle Medium (DMEM) (Welgene, Korea) containing 10% FBS. KRasG12D/+ TP53-/- PLC (primary lung cancer cells derived from a spontaneous KRas/p53 GEMM; LA1, Nature. 2001, 26;410(6832):1111-6), PSC (primary sarcoma cell line derived from a spontaneous KRas/p53 GEMM, sarcoma), and TP53-/- PTLC (primary T lymphoma cell line derived from a spontaneous p53 GEMM, lymphoma) cells were generated from soft bone sarcoma, lung tumor, and thymic tumor of p53-deficient oncogenic KrasG12D mice, respectively. Their origin was confirmed by PCR of excised Kras and p53 alleles, and they were cultured in DMEM/F12 medium containing 10% FBS after collagen-coated tissue culture. All cell lines were cultured for 2 to 16 passages at 37°C in a 5% CO₂ concentration, and then harvested using TrypLE Express (Thermo fisher scientific, USA) or 0.25% trypsin before performing *in vivo* transplantation.

The harvested cell lines were lysed using 700 µL QIAzol lysis reagent (QIAGEN, Germany) and then homogenized. Total RNA was extracted using RNeasy Micro Kit (Qiagen), reverse transcription was performed using SuperScript II Reverse Transcriptase (Thermo fisher Scientific, USA), and amplification was performed using SuperScript II Reverse Transcriptase (Thermo fisher Scientific, USA) in StepOnePlus Real-Time PCR System (Thermo fisher Scientific, USA). The expression level of the target gene was determined using the change-in-cycling-threshold (2-ΔΔCt) method and normalized using Gapdh.

### 1-3. Design of Fusion proteins

Each component of the fusion protein to be used in the examples below was constructed as shown in Table 1 below, and then, the fusion protein was designed by connecting each component with a linker as described in Table 2.

**[Table 1]**

| SEQ ID NO: | Name | Sequence |
|---|---|---|
| 1 | FEBM | SSSPIQGSWTWENGKWTWKGIIRLEQ |
| 2 | Anti-EDB-FN-HC | |
| 3 | Anti-EDB-FN-LC | |
| 4 | anti-EDB-FN-scfv | EVQLLESGGGLVQPGGSLRLSCAASGFTFSSFSMSWVRQAPG KGLEWVSSISGSSGTTYYADSVKGRFTISRDNSKNTLYLQMNS LRAEDTAVYYCAKPFPYFDYWGQGTLVTVSSGGGGSGGGGS GGGGSEIVLTQSPGTLSLSPGERATLSCRASQSVSSSFLAWYQ QKPGQAPRLLIYYASSRATGIPDRFSGSGSGTDFTLTISRLEPED FAVYYCQQTGRIPPTFGQGTKVEIK |
| 5 | TGFβ Trap | IPPHVQKSVNNDMIVTDNNGAVKFPQLCKFCDVRFSTCDNQK SCMSNCSITSICEKPQEVCVAVWRKNDENITLETVCHDPKLPY HDFILEDAASPKCIMKEKKKPGETFFMCSCSSDECNDNIIFSEE YNTSNPD |
| 6 | Anti-TGFβ1-HC | QVQLVQSGAEVKKPGSSVKVSCKASGGTFSSYAISWVRQAPG QGLEWMGGIIPIFGTANYAQKFQGRVTITADESTSTAYMELSS LRSEDTAVYYCARGLWEVRALPSVYWGQGTLVTVSS |
| 7 | Anti-TGFβ1-LC | SYELTQPPSVSVAPGQTARITCGANDIGSKSVHWYQQKAGQA PVLVVSEDIIRPSGIPERISGSNSGNTATLTISRVEAGDEADYYC QVWDRDSDQYVFGTGTKVTVL |
| 8 | Anti-TGFβ1-scfv | QVQLVQSGAEVKKPGSSVKVSCKASGGTFSSYAISWVRQAPG QGLEWMGGIIPIFGTANYAQKFQGRVTITADESTSTAYMELSS LRSEDTAVYYCARGLWEVRALPSVYWGQGTLVTVSSSSSSGS SSSGSSSSGSYELTQPPSVSVAPGQTARITCGANDIGSKSVHWY QQKAGQAPVLVVSEDIIRPSGIPERISGSNSGNTATLTISRVEAG DEADYYCQVWDRDSDQYVFGTGTKVTVL |
| 9 | Anti-PD-1-HC | |
| 10 | Anti-PD-1-LC | |
| 11 | Anti-PD-1-scfv | QVQLVQSGVEVKKPGASVKVSCKASGYTFTNYYMYWVRQA PGQGLEWMGGINPSNGGTNFNEKFKNRVTLTTDSSTTTAYME LKSLQFDDTAVYYCARRDYRFDMGFDYWGQGTTVTVSSGG GGSGGGGSGGGGSEIVLTQSPATLSLSPGERATLSCRASKGVS TSGYSYLHWYQQKPGQAPRLLIYLASYLESGVPARFSGSGSGT DFTLTISSLEPEDFAVYYCQHSRDLPLTFGGGTKVEIK |
| 12 | hFc(=hIgG4-Fc) | ESKYGPPCPPCPAPEFLGGPSVFLFPPKPKDTLMISRTPEVTCV VVDVSQEDPEVQFNWYVDGVEVHNAKTKPREEQFNSTYRVV SVLTVLHQDWLNGKEYKCKVSNKGLPSSIEKTISKAKGQPREP QVYTLPPSQEEMTKNQVSLTCLVKGFYPSDIAVEWESNGQPE NNYKTTPPVLDSDGSFFLYSRLTVDKSRWQEGNVFSCSVMHE ALHNHYTQKSLSLSLGK |
| 13 | hIgG1-Fc | THTCPPCPAPELLGGPSVFLFPPKPKDTLMISRTPEVTCVVVDV SHEDPEVKFNWYVDGVEVHNAKTKPREEQYNSTYRVVSVLT VLHQDWLNGKEYKCKVSNKALPAPIEKTISKAKGQPREPQVY TLPPSREEMTKNQVSLTCLVKGFYPSDIAVEWESNGQPENNY KTTPPVLDSDGSFFLYSKLTVDKSRWQQGNVFSCSVMHEALH NHYTQKSLSLSPGK |
| 14 | IGHG | ASTKGPSVFPLAPCSRSTSESTAALGCLVKDYFPEPVTVSWNS GALTSGVHTFPAVLQSSGLYSLSSVVTVPSSSLGTKTYTCNVD HKPSNTKVDKRVESKYGPPCPPCPAPEFLGGPSVFLFPPKPKD TLMISRTPEVTCVVVDVSQEDPEVQFNWYVDGVEVHNAKTK PREEQFNSTYRVVSVLTVLHQDWLNGKEYKCKVSNKGLPSSI EKTISKAKGQPREQVYTLPPSQEEMTKNQVSLTCLVKGFYPSD IAVEWESNGQPENNYKTTPPVLDSDGSFFLYSRLTVDKSRWQ EGNVFSCSVMHEALHNHYTQKSLSLSLGK |
| 15 | IGKC | RTVAAPSVFIFPPSDEQLKSGTASVVCLLNNFYPREAKVQWK VDNALQSGNSQESVTEQDSKDSTYSLSSTLTLSKADYEKHKV YACEVTHQGLSSPVTKSFNRGE |

**[Table 2]**

| SEQ ID NO: | Name | Sequence |
|---|---|---|
| 16 | TGFβ Trap-hFc-FEBM | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDGGGESKYGPPCPPCPAPEFLGGPSVFL FPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQ VYTLPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGKGGGGS GGGGSSSSPIQGSWTWENGKWTWKGIIRLEQ |
| 17 | TGFβ Trap-FEBM-hFc | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDGGGGSSSSPIQGSWTWENGKWTWK GIIRLEQGGGGSGGGGSESKYGPPCPPCPAPEFLGGPSVFLFPPKPKDTLMISRTP EVTCVVVDVSQEDPEVQFNWYVDGVEVHNAKTKPREEQFNSTYRVVSVLTVL HQDWLNGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLTVDKSR WQEGNVFSCSVMHEALHNHYTQKSLSLSLGK |
| 18 | FEBM-TGFβ Trap-hFc | SSSPIQGSWTWENGKWTWKGIIRLEQGGGGSIPPHVQKSVNNDMIVTDNNGA VKFPQLCKFCDVRFSTCDNQKSCMSNCSITSICEKPQEVCVAVWRKNDENITLE TVCHDPKLPYHDFILEDAASPKCIMKEKKKPGETFFMCSCSSDECNDNIIFSEEY NTSNPDGGGGSGGGGSESKYGPPCPPCPAPEFLGGPSVFLFPPKPKDTLMISRTP EVTCVVVDVSQEDPEVQFNWYVDGVEVHNAKTKPREEQFNSTYRVVSVLTVL HQDWLNGKEYKCKVSNKGLPSSIEKTISKAKGQPREPQVYTLPPSQEEMTKNQ VSLTCLVKGFYPSDIAVEWESNGQPENNYKTTPPVLDSDGSFFLYSRLTVDKSR WQEGNVFSCSVMHEALHNHYTQKSLSLSLGK |
| 19 | FEBM-hFc-TGFβ Trap | SSSPIQGSWTWENGKWTWKGIIRLEQESKYGPPCPPCPAPEFLGGPSVFLFPPKP KDTLMISRTPEVTCVVVDVSQEDPEVQFNWYVDGVEVHNAKTKPREEQFNST YRVVSVLTVLHQDWLNGKEYKCKVSNKGLPSSIEKTISKAKGQPREPQVYTLP PSQEEMTKNQVSLTCLVKGFYPSDIAVEWESNGQPENNYKTTPPVLDSDGSFF LYSRLTVDKSRWQEGNVFSCSVMHEALHNHYTQKSLSLSLGKGGGIPPHVQK SVNNDMIVTDNNGAVKFPQLCKFCDVRFSTCDNQKSCMSNCSITSICEKPQEV CVAVWRKNDENITLETVCHDPKLPYHDFILEDAASPKCIMKEKKKPGETFFMC SCSSDECNDNIIFSEEYNTSNP |
| 20 | hFc-FEBM-TGFβ Trap | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGSSSPIQGSWTWENGKWTWKGIIRLEQGGGGSIPP HVQKSVNNDMIVTDNNGAVKFPQLCKFCDVRFSTCDNQKSCMSNCSITSICEK PQEVCVAVWRKNDENITLETVCHDPKLPYHDFILEDAASPKCIMKEKKKPGET FFMCSCSSDECNDNIIFSEEYNTSNPD |
| 21 | hFc-TGFβ Trap-FEBM | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGIPPHVQKSVNNDMIVTDNNGAVKFPQLCKFCDV RFSTCDNQKSCMSNCSITSICEKPQEVCVAVWRKNDENITLETVCHDPKLPYH DFILEDAASPKCIMKEKKKPGETFFMCSCSSDECNDNIIFSEEYNTSNPDGGGGS SSSPIQGSWTWENGKWTWKGIIRLEQ |
| 22 | FEBM-TGFβ Trap-FEBM-hFc | SSSPIQGSWTWENGKWTWKGIIRLEQGGGGSIPPHVQKSVNNDMIVTDNNGA VKFPQLCKFCDVRFSTCDNQKSCMSNCSITSICEKPQEVCVAVWRKNDENITLE TVCHDPKLPYHDFILEDAASPKCIMKEKKKPGETFFMCSCSSDECNDNIIFSEEY NTSNPDGGGGSSSSPIQGSWTWENGKWTWKGIIRLEQGGGGSGGGGSESKYG PPCPPCPAPEFLGGPSVFLFPPKPKDTLMISRTPEVTCVVVDVSQEDPEVQFNW YVDGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDIAVEWES NGQPENNYKTTPPVLDSDGSFFLYSRLTVDKSRWQEGNVFSCSVMHEALHNH YTQKSLSLSLGK |
| 23 | hFc-FEBM-TGFβ Trap-FEBM | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGSSSPIQGSWTWENGKWTWKGIIRLEQGGGGSIPP HVQKSVNNDMIVTDNNGAVKFPQLCKFCDVRFSTCDNQKSCMSNCSITSICEK PQEVCVAVWRKNDENITLETVCHDPKLPYHDFILEDAASPKCIMKEKKKPGET FFMCSYSSDECNDNIIFSEEYNTSNPDGGGGSGGGGSSSSPIQGSWTWENGKWT WKGIIRLEQ |
| 24 | FEBM-TGFβ Trap-FEBM-hFc-FEBM | SSSPIQGSWTWENGKWTWKGIIRLEQGGGGSGGGGSIPPHVQKSVNNDMIVTD NNGAVKFPQLCKFCDVRFSTCDNQKSCMSNCSITSICEKPQEVCVAVWRKNDE NITLETVCHDPKLPYHDFILEDAASPKCIMKEKKKPGETFFMCSCSSDECNDNII FSEEYNTSNPDGGGGSSSSPIQGSWTWENGKWTWKGIIRLEQGGGGSGGGGSE SKYGPPCPPCPAPEFLGGPSVFLFPPKPKDTLMISRTPEVTCVVVDVSQEDPEVQ FNWYVDGVEVHNAKTKPREEQFNSTYRVVSVLTVLHQDWLNGKEYKCKVSN KGLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKGGGSSSPIQGSWTWENGKWTWKGIIRLEQ |
| 25 | FEBM-hFc-FEBM-TGFβ Trap-FEBM | SSSPIQGSWTWENGKWTWKGIIRLEQGGGGSGGGGSESKYGPPCPPCPAPEFL GGPSVFLFPPKPKDTLMISRTPEVTCVVVDVSQEDPEVQFNWYVDGVEVHNA KTKPREEQFNSTYRVVSVLTVLHQDWLNGKEYKCKVSNKGLPSSIEKTISKAK GQPREPQVYTLPPSQEEMTKNQVSLTCLVKGFYPSDIAVEWESNGQPENNYKT TPPVLDSDGSFFLYSRLTVDKSRWQEGNVFSCSVMHEALHNHYTQKSLSLSLG KGGGSSSPIQGSWTWENGKWTWKGIIRLEQGGGGSIPPHVQKSVNNDMIVTD NNGAVKFPQLCKFCDVRFSTCDNQKSCMSNCSITSICEKPQEVCVAVWRKNDE NITLETVCHDPKLPYHDFILEDAASPKCIMKEKKKPGETFFMCSYSSDECNDNII FSEEYNTSNPDGGGGSGGGGSSSPIQGSWTWENGKWTWKGIIRLEQ |
| 26 | TGFβ Trap-FEBM-hIgG1-Fc | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPD GGGSSSSPIQGSWTWENGKWTWKGIIRLEQGGGGSGGGGSTHTCPPCPAPELL GGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAK GQPREPQVYTLPPSREEMTKNQVSLTCLVKGFYPSDIAVEWESNGQPENNYKT TPPVLDSDGSFFLYSKLTVDKSRWQQGNVFSCSVMHEALHNHYTQKSLSLSPG K |
| 27 | hIgG1-Fc-FEBM-TGFβ Trap | THTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHEAL HNHYTQKSLSLSPGKGGGGSSSSPIQGSWTWENGKWTWKGIIRLEQGGGGSIP PHVQKSVNNDMIVTDNNGAVKFPQLCKFCDVRFSTCDNQKSCMSNCSITSICE KPQEVCVAVWRKNDENITLETVCHDPKLPYHDFILEDAASPKCIMKEKKKPGE TFFMCSCSSDECNDNIIFSEEYNTSNPD |
| 28 | hFc-soluble TGFβ Trap | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGIPPHVQKSVNNDMIVTDNNGAVKFPQLCKFCDV RFSTCDNQKSCMSNCSITSICEKPQEVCVAVWRKNDENITLETVCHDPKLPYH DFILEDAASPKCIMKEKKKPGETFFMCSCSSDECNDNIIFSEEYNTSNPD |
| 29 | Anti-EDB-FN-HC-IGHG-TGFβ Trap | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSASTKGPSVFPLAPCSRSTSESTAALGCLVKDYFPEPVTVSWNSG ALTSGVHTFPAVLQSSGLYSLSSVVTVPSSSLGTKTYTCNVDHKPSNTKVDKR VESKYGPPCPPCPAPEFLGGPSVFLFPPKPKDTLMISRTPEVTCVVVDVSQEDPE VQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSVMHE ALHNHYTQKSLSLSLGKGGGGSGGGGSIPPHVQKSVNNDMIVTDNNGAVKFP QLCKFCDVRFSTCDNQKSCMSNCSITSICEKPQEVCVAVWRKNDENITLETVC HDPKLPYHDFILEDAASPKCIMKEKKKPGETFFMCSYSSDECNDNIIFSEEYNTS NPD |
| 30 | Anti-EDB-FN-LC-IGKC-TGFβ Trap | EIVLTQSPGTLSLSPGERATLSCRASQSVSSSFLAWYQQKPGQAPRLLIYYASSR ATGIPDRFSGSGSGTDFTLTISRLEPEDFAVYYCQQTGRIPPTFGQGTKVEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGECG GGGSGGGGSIPPHVQKSVNNDMIVTDNNGAVKFPQLCKFCDVRFSTCDNQKS CMSNCSITSICEKPQEVCVAVWRKNDENITLETVCHDPKLPYHDFILEDAASPK CIMKEKKKPGETFFMCSCSSDECNDNIIFSEEYNTSNPD |
| 31 | TGFβ Trap-anti-EDB-FN-hIgG | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDGGGGSGGGGSGGGGSEVQLLESGGG LVQPGGSLRLSCAASGFTFSSFSMSWVRQAPGKGLEWVSSISGSSGTTYYADS VKGRFTISRDNSKNTLYLQMNSLRAEDTAVYYCAKPFPYFDYWGQGTLVTVS SASTKGPSVFPLAPCSRSTSESTAALGCLVKDYFPEPVTVSWNSGALTSGVHTF PAVLQSSGLYSLSSVVTVPSSSLGTKTYTCNVDHKPSNTKVDKRVESKYGPPCP PCPAPEFLGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSIE KTISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLGK |
| 32 | Anti-EDB-FN-scFv-hFc-TGFβ Trap | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKSSSSGSSSSGSSSSGESKYGPPCPPCPAPEFL GGPSVFLFPPKPKDTLMISRTPEVTCVVVDVSQEDPEVQFNWYVDGVEVHNA KTKPREEQFNSTYRVVSVLTVLHQDWLNGKEYKCKVSNKGLPSSIEKTISKAK GQPREPQVYTLPPSQEEMTKNQVSLTCLVKGFYPSDIAVEWESNGQPENNYKT TPPVLDSDGSFFLYSRLTVDKSRWQEGNVFSCSVMHEALHNHYTQKSLSLSLG KGGGGSGGGGSIPPHVQKSVNNDMIVTDNNGAVKFPQLCKFCDVRFSTCDNQ KSCMSNCSITSICEKPQEVCVAVWRKNDENITLETVCHDPKLPYHDFILEDAAS PKCIMKEKKKPGETFFMCSCSSDECNDNIIFSEEYNTSNPD |
| 33 | hFc-anti-EDB-FN-scFv-TGFβ Trap | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKSSSSGSSSSGSSSSGEVQLLESGGGLVQPGGSLRLSCAA SGFTFSSFSMSWVRQAPGKGLEWVSSISGSSGTTYYADSVKGRFTISRDNSKNT LYLQMNSLRAEDTAVYYCAKPFPYFDYWGQGTLVTVSSGGGGSGGGGSGGG GSEIVLTQSPGTLSLSPGERATLSCRASQSVSSSFLAWYQQKPGQAPRLLIYYAS SRATGIPDRFSGSGSGTDFTLTISRLEPEDFAVYYCQQTGRIPPTFGQGTKVEIKS SSSGSSSSGSSSSGIPPHVQKSVNNDMIVTDNNGAVKFPQLCKFCDVRFSTCDN QKSCMSNCSITSICEKPQEVCVAVWRKNDENITLETVCHDPKLPYHDFILEDAA SPKCIMKEKKKPGETFFMCSCSSDECNDNIIFSEEYNTSNPD |
| 34 | Anti-EDB-FN-scFv-TGFβ Trap-hFc | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKSSSSGSSSSGSSSSGIPPHVQKSVNNDMIVT DNNGAVKFPQLCKFCDVRFSTCDNQKSCMSNCSITSICEKPQEVCVAVWRKN DENITLETVCHDPKLPYHDFILEDAASPKCIMKEKKKPGETFFMCSCSSDECND NIIFSEEYNTSNPDGGGGSGGGGSGGGGSESKYGPPCPPCPAPEFLGGPSVFLFP PKPKDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPREPQVY TLPPSQEEMTKNQVSLTCLVKGFYPSDIAVEWESNGQPENNYKTTPPVLDSDG SFFLYSRLTVDKSRWQEGNVFSCSVMHEALHNHYTQKSLSLSLGK |
| 35 | hFc-TGFβ Trap-Anti-EDB-FN scFv | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKSSSSGSSSSGSSSSGIPPHVQKSVNNDMIVTDNNGAVK FPQLCKFCDVRFSTCDNQKSCMSNCSITSICEKPQEVCVAVWRKNDENITLETV CHDPKLPYHDFILEDAASPKCIMKEKKKPGETFFMCSCSSDECNDNIIFSEEYNT SNPDSSSSGSSSSGSSSSGEVQLLESGGGLVQPGGSLRLSCAASGFTFSSFSMSW VRQAPGKGLEWVSSISGSSGTTYYADSVKGRFTISRDNSKNTLYLQMNSLRAE DTAVYYCAKPFPYFDYWGQGTLVTVSSGGGGSGGGGSGGGGSEIVLTQSPGT LSLSPGERATLSCRASQSVSSSFLAWYQQKPGQAPRLLIYYASSRATGIPDRFSG SGSGTDFTLTISRLEPEDFAVYYCQQTGRIPPTFGQGTKVEIK |
| 36 | Anti-EDB-FN scFv-TGFβ Trap | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKSSSSGSSSSGSSSSGIPPHVQKSVNNDMIVT DNNGAVKFPQLCKFCDVRFSTCDNQKSCMSNCSITSICEKPQEVCVAVWRKN DENITLETVCHDPKLPYHDFILEDAASPKCIMKEKKKPGETFFMCSCSSDECND NIIFSEEYNTSNPD |
| 37 | Anti-TGFβ1-HC-IGHG-FEBM | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSASTKGPSVFPLAPCSRSTSESTAALGCLVKDYFPEPVTV SWNSGALTSGVHTFPAVLQSSGLYSLSSVVTVPSSSLGTKTYTCNVDHKPSNT KVDKRVESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWLNGK EYKCKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGNVFS CSVMHEALHNHYTQKSLSLSLGKGGGSSPIQGSWTWENGKWTWKGIIRLEQ |
| 38 | Anti-TGFβ1-LC-IGKC-FEBM | SYELTQPPSVSVAPGQTARITCGANDIGSKSVHWYQQKAGQAPVLVVSEDIIRP SGIPERISGSNSGNTATLTISRVEAGDEADYYCQVWDRDSDQYVFGTGTKVTV LRTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRG ECGGGSSSPIQGSWTWENGKWTWKGIIRLEQ |
| 39 | FEBM-Anti-TGFβ1-IGHG | SSSPIQGSWTWENGKWTWKGIIRLEQGGGGSGGGGSGGGGSQVQLVQSGAEV KKPGSSVKVSCKASGGTFSSYAISWVRQAPGQGLEWMGGIIPIFGTANYAQKF QGRVTITADESTSTAYMELSSLRSEDTAVYYCARGLWEVRALPSVYWGQGTL VTVSSASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRVESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGK |
| 40 | Anti-TGFβ1 scFv-FEBM-hFc | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGSSSPIQGSWTWENGKWTWK GIIRLEQGGGGSGGGGSGGGGSESKYGPPCPPCPAPEFLGGPSVFLFPPKPKDTL MISRTPEVTCVVVDVSQEDPEVQFNWYVDGVEVHNAKTKPREEQFNSTYRVV SVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRL TVDKSRWQEGNVFSCSVMHEALHNHYTQKSLSLSLGK |
| 41 | FEBM-Anti-TGFβ1 scFv-hFc | SSSPIQGSWTWENGKWTWKGIIRLEQGGGGSGGGGSGGGGSQVQLVQSGAEV KKPGSSVKVSCKASGGTFSSYAISWVRQAPGQGLEWMGGIIPIFGTANYAQKF QGRVTITADESTSTAYMELSSLRSEDTAVYYCARGLWEVRALPSVYWGQGTL VTVSSSSSSGSSSSGSSSSGSYELTQPPSVSVAPGQTARITCGANDIGSKSVHWY QQKAGQAPVLVVSEDIIRPSGIPERISGSNSGNTATLTISRVEAGDEADYYCQV WDRDSDQYVFGTGTKVTVLGGGGSGGGGSGGGGSESKYGPPCPPCPAPEFLG GPSVFLFPPKPKDTLMISRTPEVTCVVVDVSQEDPEVQFNWYVDGVEVHNAKT KPREEQFNSTYRVVSVLTVLHQDWLNGKEYKCKVSNKGLPSSIEKTISKAKGQ PREPQVYTLPPSQEEMTKNQVSLTCLVKGFYPSDIAVEWESNGQPENNYKTTP PVLDSDGSFFLYSRLTVDKSRWQEGNVFSCSVMHEALHNHYTQKSLSLSLGK |
| 42 | hFc-FEBM-anti-TGFβ1 scFv | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGSSSPIQGSWTWENGKWTWKGIIRLEQGGGGSGG GGSGGGGSQVQLVQSGAEVKKPGSSVKVSCKASGGTFSSYAISWVRQAPGQG LEWMGGIIPIFGTANYAQKFQGRVTITADESTSTAYMELSSLRSEDTAVYYCAR GLWEVRALPSVYWGQGTLVTVSSSSSSGSSSSGSSSSGSYELTQPPSVSVAPGQ TARITCGANDIGSKSVHWYQQKAGQAPVLVVSEDIIRPSGIPERISGSNSGNTAT LTISRVEAGDEADYYCQVWDRDSDQYVFGTGTKVTVL |
| 43 | hFc-anti-TGFβ1-scFv-FEBM | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGGSGGGGSGGGGSQVQLVQSGAEVKKPGSSVKVS CKASGGTFSSYAISWVRQAPGQGLEWMGGIIPIFGTANYAQKFQGRVTITADES TSTAYMELSSLRSEDTAVYYCARGLWEVRALPSVYWGQGTLVTVSSSSSSGSS SSGSSSSGSYELTQPPSVSVAPGQTARITCGANDIGSKSVHWYQQKAGQAPVL VVSEDIIRPSGIPERISGSNSGNTATLTISRVEAGDEADYYCQVWDRDSDQYVF GTGTKVTVLSSSSGSSSSGSSSSGSSSPIQGSWTWENGKWTWKGIIRLEQ |
| 44 | Anti-TGFβ1-scFv-FEBM | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSGGGGSSSSPIQGS WTWENGKWTWKGIIRLEQ |
| 45 | FEBM-anti-TGFβ1 scFv | SSSPIQGSWTWENGKWTWKGIIRLEQGGGGSGGGGSGGGGSQVQLVQSGAEV KKPGSSVKVSCKASGGTFSSYAISWVRQAPGQGLEWMGGIIPIFGTANYAQKF QGRVTITADESTSTAYMELSSLRSEDTAVYYCARGLWEVRALPSVYWGQGTL VTVSSSSSSGSSSSGSSSSGSYELTQPPSVSVAPGQTARITCGANDIGSKSVHWY QQKAGQAPVLVVSEDIIRPSGIPERISGSNSGNTATLTISRVEAGDEADYYCQV WDRDSDQYVFGTGTKVTVL |
| 46 | Anti-EDB-FN-HC-IGHG-anti-TGFβ1 scFv | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSASTKGPSVFPLAPCSRSTSESTAALGCLVKDYFPEPVTVSWNSG ALTSGVHTFPAVLQSSGLYSLSSVVTVPSSSLGTKTYTCNVDHKPSNTKVDKR VESKYGPPCPPCPAPEFLGGPSVFLFPPKPKDTLMISRTPEVTCVVVDVSQEDPE VQFNWYVDGVEVHNAKTKPREEQFNSTYRVVSVLTVLHQDWLNGKEYKCK VSNKGLPSSIEKTISKAKGQPREQVYTLPPSQEEMTKNQVSLTCLVKGFYPSDIA VEWESNGQPENNYKTTPPVLDSDGSFFLYSRLTVDKSRWQEGNVFSCSVMHE ALHNHYTQKSLSLSLGKGGGGSGGGGSGGGGSQVQLVQSGAEVKKPGSSVKV SCKASGGTFSSYAISWVRQAPGQGLEWMGGIIPIFGTANYAQKFQGRVTITADE STSTAYMELSSLRSEDTAVYYCARGLWEVRALPSVYWGQGTLVTVSSSSSSGS SSSGSSSSGSYELTQPPSVSVAPGQTARITCGANDIGSKSVHWYQQKAGQAPVL VVSEDIIRPSGIPERISGSNSGNTATLTISRVEAGDEADYYCQVWDRDSDQYVF GTGTKVTVL |
| 47 | Anti-TGFβ1-hIgG-Anti-EDB-FN scFv | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSASTKGPSVFPLAPCSRSTSESTAALGCLVKDYFPEPVTV SWNSGALTSGVHTFPAVLQSSGLYSLSSVVTVPSSSLGTKTYTCNVDHKPSNT KVDKRVESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWLNGK EYKCKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGNVFS CSVMHEALHNHYTQKSLSLSLGKSSSSGSSSSGSSSSGEVQLLESGGGLVQPGG SLRLSCAASGFTFSSFSMSWVRQAPGKGLEWVSSISGSSGTTYYADSVKGRFTI SRDNSKNTLYLQMNSLRAEDTAVYYCAKPFPYFDYWGQGTLVTVSSGGGGSG GGGSGGGGSEIVLTQSPGTLSLSPGERATLSCRASQSVSSSFLAWYQQKPGQAP RLLIYYASSRATGIPDRFSGSGSGTDFTLTISRLEPEDFAVYYCQQTGRIPPTFGQ GTKVEIK |
| 48 | Anti-TGFβ1 scFv-anti-EDB-FN-hIgG | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSGGGGSEVQLLES GGGLVQPGGSLRLSCAASGFTFSNSAMTWVRQAPGKRLEWVASISGGGTTYY PDSVKGRFTISRDNSKNTLYLQMNSLRAEDTAVYYCARSHYWGQGTLVTVSS ASTKGPSVFPLAPCSRSTSESTAALGCLVKDYFPEPVTVSWNSGALTSGVHTFP AVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSIE KTISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLGK |
| 49 | Anti-TGFβ1 scFv-anti-EDB-FN-hIgG | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGGSGGGGSGGGGSEVQLLES GGGLVQPGGSLRLSCAASGFTFSNSAMTWVRQAPGKRLEWVASISGGGTTYY PDSVKGRFTISRDNSKNTLYLQMNSLRAEDTAVYYCARSHYWGQGTLVTVSS ASTKGPSVFPLAPCSRSTSESTAALGCLVKDYFPEPVTVSWNSGALTSGVHTFP AVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSIE KTISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLGK |
| 50 | Anti-EDB-FN scFv-Anti-TGFβ1 hIgG | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKSSSSGSSSSGSSSSGQVQLVQSGAEVKKPG SSVKVSCKASGGTFSSYAISWVRQAPGQGLEWMGGIIPIFGTANYAQKFQGRV TITADESTSTAYMELSSLRSEDTAVYYCARGLWEVRALPSVYWGQGTLVTVSS ASTKGPSVFPLAPCSRSTSESTAALGCLVKDYFPEPVTVSWNSGALTSGVHTFP AVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSIE KTISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLGK |
| 51 | Anti-EDB-FN scFv-anti-TGFβ1 scFv-hFc | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKSSSSGSSSSGSSSSGQVQLVQSGAEVKKPG SSVKVSCKASGGTFSSYAISWVRQAPGQGLEWMGGIIPIFGTANYAQKFQGRV TITADESTSTAYMELSSLRSEDTAVYYCARGLWEVRALPSVYWGQGTLVTVSS GGGGSGGGGSGGGGSSYELTQPPSVSVAPGQTARITCGANDIGSKSVHWYQQ KAGQAPVLVVSEDIIRPSGIPERISGSNSGNTATLTISRVEAGDEADYYCQVWD RDSDQYVFGTGTKVTVLGGGGGSGGGGSGGGGSESKYGPPCPPCPAPEFLGGP SVFLFPPKPKDTLMISRTPEVTCVVVDVSQEDPEVQFNWYVDGVEVHNAKTKP REEQFNSTYRVVSVLTVLHQDWLNGKEYKCKVSNKGLPSSIEKTISKAKGQPR EPQVYTLPPSQEEMTKNQVSLTCLVKGFYPSDIAVEWESNGQPENNYKTTPPV LDSDGSFFLYSRLTVDKSRWQEGNVFSCSVMHEALHNHYTQKSLSLSLGK |
| 52 | Anti-TGFβ1 scFv-anti-EDB-FN scFv-hFc | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSGGGGSEVQLLESG GGLVQPGGSLRLSCAASGFTFSSFSMSWVRQAPGKGLEWVSSISGSSGTTYYA DSVKGRFTISRDNSKNTLYLQMNSLRAEDTAVYYCAKPFPYFDYWGQGTLVT VSSGGGGSGGGGSGGGGSEIVLTQSPGTLSLSPGERATLSCRASQSVSSSFLAW YQQKPGQAPRLLIYYASSRATGIPDRFSGSGSGTDFTLTISRLEPEDFAVYYCQQ TGRIPPTFGQGTKVEIKGGGGSGGGGSGGGGSESKYGPPCPPCPAPEFLGGPSV FLFPPKPKDTLMISRTPEVTCVVVDVSQEDPEVQFNWYVDGVEVHNAKTKPRE EQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLD SDGSFFLYSRLTVDKSRWQEGNVFSCSVMHEALHNHYTQKSLSLSLGK |
| 53 | hFc-Anti-EDB-FN scFv-Anti-TGFβ1 scfv | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKSSSSGSSSSGSSSSGEVQLLESGGGLVQPGGSLRLSCAA SGFTFSSFSMSWVRQAPGKGLEWVSSISGSSGTTYYADSVKGRFTISRDNSKNT LYLQMNSLRAEDTAVYYCAKPFPYFDYWGQGTLVTVSSGGGGSGGGGSGGG GSEIVLTQSPGTLSLSPGERATLSCRASQSVSSSFLAWYQQKPGQAPRLLIYYAS SRATGIPDRFSGSGSGTDFTLTISRLEPEDFAVYYCQQTGRIPPTFGQGTKVEIKS SSSGSSSSGSSSSGQVQLVQSGAEVKKPGSSVKVSCKASGGTFSSYAISWVRQA PGQGLEWMGGIIPIFGTANYAQKFQGRVTITADESTSTAYMELSSLRSEDTAVY YCARGLWEVRALPSVYWGQGTLVTVSSSSSSGSSSSGSSSSGSYELTQPPSVSV APGQTARITCGANDIGSKSVHWYQQKAGQAPVLVVSEDIIRPSGIPERISGSNSG NTATLTISRVEAGDEADYYCQVWDRDSDQYVFGTGTKVTVL |
| 54 | hFc-Anti-TGFβ1 scfv-Anti-EDB-FN scFv | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGGSGGGGSGGGGSQVQLVQSGAEVKKPGSSVKVS CKASGGTFSSYAISWVRQAPGQGLEWMGGIIPIFGTANYAQKFQGRVTITADES TSTAYMELSSLRSEDTAVYYCARGLWEVRALPSVYWGQGTLVTVSSSSSSGSS SSGSSSSGSYELTQPPSVSVAPGQTARITCGANDIGSKSVHWYQQKAGQAPVL VVSEDIIRPSGIPERISGSNSGNTATLTISRVEAGDEADYYCQVWDRDSDQYVF GTGTKVTVLGGGGSGGGGSGGGGSEVQLLESGGGLVQPGGSLRLSCAASGFT FSSFSMSWVRQAPGKGLEWVSSISGSSGTTYYADSVKGRFTISRDNSKNTLYLQ MNSLRAEDTAVYYCAKPFPYFDYWGQGTLVTVSSGGGGSGGGGSGGGGSEIV LTQSPGTLSLSPGERATLSCRASQSVSSSFLAWYQQKPGQAPRLLIYYASSRAT GIPDRFSGSGSGTDFTLTISRLEPEDFAVYYCQQTGRIPPTFGQGTKVEIK |
| 55 | Anti-EDB-FN scFv-Anti TGFβ scFv | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKSSSSGSSSSGSSSSGQVQLVQSGAEVKKPG SSVKVSCKASGGTFSSYAISWVRQAPGQGLEWMGGIIPIFGTANYAQKFQGRV TITADESTSTAYMELSSLRSEDTAVYYCARGLWEVRALPSVYWGQGTLVTVSS GGGGSGGGGSGGGGSSYELTQPPSVSVAPGQTARITCGANDIGSKSVHWYQQ KAGQAPVLVVSEDIIRPSGIPERISGSNSGNTATLTISRVEAGDEADYYCQVWD RDSDQYVFGTGTKVTVLG |
| 56 | Anti-TGFβ1 scFv-Anti-EDB-FN scFv | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSGGGGSEVQLLESG GGLVQPGGSLRLSCAASGFTFSSFSMSWVRQAPGKGLEWVSSISGSSGTTYYA DSVKGRFTISRDNSKNTLYLQMNSLRAEDTAVYYCAKPFPYFDYWGQGTLVT VSSGGGGSGGGGSGGGGSEIVLTQSPGTLSLSPGERATLSCRASQSVSSSFLAW YQQKPGQAPRLLIYYASSRATGIPDRFSGSGSGTDFTLTISRLEPEDFAVYYCQQ TGRIPPTFGQGTKVEIK |
| 57 | Anti-PD-1-HC-IGHG-FEBM-TGFβ Trap | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKGGGSSSPIQGSWTWENGKWTWKGIIRLE QGGGGSGGGGSIPPHVQKSVNNDMIVTDNNGAVKFPQLCKFCDVRFSTCDNQ KSCMSNCSITSICEKPQEVCVAVWRKNDENITLETVCHDPKLPYHDFILEDAAS PKCIMKEKKKPGETFFMCSCSSDECNDNIIFSEEYNTSNPD |
| 58 | Anti-PD-1-HC-IGHG-TGFβ Trap-FEBM | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKGGGGSIPPHVQKSVNNDMIVTDNNGAV KFPQLCKFCDVRFSTCDNQKSCMSNCSITSICEKPQEVCVAVWRKNDENITLET VCHDPKLPYHDFILEDAASPKCIMKEKKKPGETFFMCSYSSDECNDNIIFSEEYN TSNPDGGGGSGGGGSSSSPIQGSWTWENGKWTWKGIIRLEQ |
| 59 | Anti-PD-1-LC-IGKC-FEBM-TGFβ Trap | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECGGGSSSPIQGSWTWENGKWTWKGIIRLEQGGGGSGGGGSIPPHVQKSVN NDMIVTDNNGAVKFPQLCKFCDVRFSTCDNQKSCMSNCSITSICEKPQEVCVA VWRKNDENITLETVCHDPKLPYHDFILEDAASPKCIMKEKKKPGETFFMCSCSS DECNDNIIFSEEYNTSNPD |
| 60 | Anti-PD-1-LC-IGKC-TGFβ trap-FEBM | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECGGGGSGGGGSGGGGSIPPHVQKSVNNDMIVTDNNGAVKFPQLCKFCDVR FSTCDNQKSCMSNCSITSICEKPQEVCVAVWRKNDENITLETVCHDPKLPYHDF ILEDAASPKCIMKEKKKPGETFFMCSCSSDECNDNIIFSEEYNTSNPDSSSSGSSS SGSSSSGSSSPIQGSWTWENGKWTWKGIIRLEQ |
| 61 | TGFβ Trap-FEBM-anti-PD-1-HC-IGHG | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDGGGGSGGGGSGGGGSSSSPIQGSWT WENGKWTWKGIIRLEQSSSSGSSSSGSSSSGQVQLVQSGVEVKKPGASVKVSC KASGYTFTNYYMYWVRQAPGQGLEWMGGINPSNGGTNFNEKFKNRVTLTTD SSTTTAYMELKSLQFDDTAVYYCARRDYRFDMGFDYWGQGTTVTVSSASTK GPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPPCPAP EFLGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISK AKGQPREPQVYTLPPSQEEMTKNQVSLTCLVKGFYPSDIAVEWESNGQPENNY KTTPPVLDSDGSFFLYSRLTVDKSRWQEGNVFSCSVMHEALHNHYTQKSLSLS LGK |
| 62 | FEBM-TGFβ Trap-anti-PD-1-HC-IGHG | SSSPIQGSWTWENGKWTWKGIIRLEQSSSSGSSSSGSSSSGIPPHVQKSVNNDMI VTDNNGAVKFPQLCKFCDVRFSTCDNQKSCMSNCSITSICEKPQEVCVAVWRK NDENITLETVCHDPKLPYHDFILEDAASPKCIMKEKKKPGETFFMCSCSSDECN DNIIFSEEYNTSNPDGGGGSGGGGSGGGGSQVQLVQSGVEVKKPGASVKVSCK ASGYTFTNYYMYWVRQAPGQGLEWMGGINPSNGGTNFNEKFKNRVTLTTDS STTTAYMELKSLQFDDTAVYYCARRDYRFDMGFDYWGQGTTVTVSSASTKG PSVFPLAPCSRSTSESTAALGCLVKDYFPEPVTVSWNSGALTSGVHTFPAVLQS SGLYSLSSVVTVPSSSLGTKTYTCNVDHKPSNTKVDKRVESKYGPPCPPCPAPE FLGGPSVFLFPPKPKDTLMISRTPEVTCVVVDVSQEDPEVQFNWYVDGVEVHN AKTKPREEQFNSTYRVVSVLTVLHQDWLNGKEYKCKVSNKGLPSSIEKTISKA KGQPREPQVYTLPPSQEEMTKNQVSLTCLVKGFYPSDIAVEWESNGQPENNYK TTPPVLDSDGSFFLYSRLTVDKSRWQEGNVFSCSVMHEALHNHYTQKSLSLSL GK |
| 63 | Anti-PD-1 scFv-hFc-FEBM-TGFβ Trap | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKGGGSSSPIQGSWTWENGKWTWKGIIRLEQGGGGSIPPH VQKSVNNDMIVTDNNGAVKFPQLCKFCDVRFSTCDNQKSCMSNCSITSICEKP QEVCVAVWRKNDENITLETVCHDPKLPYHDFILEDAASPKCIMKEKKKPGETF FMCSCSSDECNDNIIFSEEYNTSNPD |
| 64 | Anti-PD-1 scFv-hFc-TGFβ Trap-FEBM | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKGGGGSIPPHVQKSVNNDMIVTDNNGAVKFPQLCKFCD VRFSTCDNQKSCMSNCSITSICEKPQEVCVAVWRKNDENITLETVCHDPKLPY HDFILEDAASPKCIMKEKKKPGETFFMCSYSSDECNDNIIFSEEYNTSNPDGGG GSGGGGSSSSPIQGSWTWENGKWTWKGIIRLEQ |
| 65 | TGFβ Trap-FEBM-hFc-anti-PD1-scFv | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDGGGGSGGGGSGGGGSSSSPIQGSWT WENGKWTWKGIIRLEQSSSSGSSSSGSSSSGESKYGPPCPPCPAPEFLGGPSVFL FPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQ VYTLPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGKGGGGS GGGGSGGGGSQVQLVQSGVEVKKPGASVKVSCKASGYTFTNYYMYWVRQA PGQGLEWMGGINPSNGGTNFNEKFKNRVTLTTDSSTTTAYMELKSLQFDDTA VYYCARRDYRFDMGFDYWGQGTTVTVSSGGGGSGGGGSGGGGSEIVLTQSP ATLSLSPGERATLSCRASKGVSTSGYSYLHWYQQKPGQAPRLLIYLASYLESG VPARFSGSGSGTDFTLTISSLEPEDFAVYYCQHSRDLPLTFGGGTKVEIK |
| 66 | Anti-PD-1 scFv-FEBM-TGFβ Trap | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGSSSPI QGSWTWENGKWTWKGIIRLEQGGGGSGGGGSGGGGSIPPHVQKSVNNDMIV TDNNGAVKFPQLCKFCDVRFSTCDNQKSCMSNCSITSICEKPQEVCVAVWRKN DENITLETVCHDPKLPYHDFILEDAASPKCIMKEKKKPGETFFMCSCSSDECND NIIFSEEYNTSNPD |
| 67 | Anti-PD-1 scFv-TGFβ Trap-FEBM | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGIPPHV QKSVNNDMIVTDNNGAVKFPQLCKFCDVRFSTCDNQKSCMSNCSITSICEKPQ EVCVAVWRKNDENITLETVCHDPKLPYHDFILEDAASPKCIMKEKKKPGETFF MCSCSSDECNDNIIFSEEYNTSNPDGGGGSGGGGSGGGGSSSSPIQGSWTWENG KWTWKGIIRLEQ |
| 68 | TGFβ Trap-FEBM-anti-PD1-scFv | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDGGGGSGGGGSGGGGSSSSPIQGSWT WENGKWTWKGIIRLEQSSSSGSSSSGSSSSGQVQLVQSGVEVKKPGASVKVSC KASGYTFTNYYMYWVRQAPGQGLEWMGGINPSNGGTNFNEKFKNRVTLTTD SSTTTAYMELKSLQFDDTAVYYCARRDYRFDMGFDYWGQGTTVTVSSGGGG SGGGGSGGGGSEIVLTQSPATLSLSPGERATLSCRASKGVSTSGYSYLHWYQQ KPGQAPRLLIYLASYLESGVPARFSGSGSGTDFTLTISSLEPEDFAVYYCQHSRD LPLTFGGGTKVEIK |
| 69 | Anti-PD1-HC-IGHG-anti-EDB-FN scfv-TGFβ Trap | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKSSSSGSSSSGSSSSGEVQLLESGGGLVQP GGSLRLSCAASGFTFSSFSMSWVRQAPGKGLEWVSSISGSSGTTYYADSVKGR FTISRDNSKNTLYLQMNSLRAEDTAVYYCAKPFPYFDYWGQGTLVTVSSGGG GSGGGGSGGGGSEIVLTQSPGTLSLSPGERATLSCRASQSVSSSFLAWYQQKPG QAPRLLIYYASSRATGIPDRFSGSGSGTDFTLTISRLEPEDFAVYYCQQTGRIPPT FGQGTKVEIKSSSSGSSSSGSSSSGIPPHVQKSVNNDMIVTDNNGAVKFPQLCKF CDVRFSTCDNQKSCMSNCSITSICEKPQEVCVAVWRKNDENITLETVCHDPKLP YHDFILEDAASPKCIMKEKKKPGETFFMCSCSSDECNDNIIFSEEYNTSNPD |
| 70 | Anti-PD1-HC-IGHG-TGFβ Trap-anti-EDB-FN scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKGGGGSIPPHVQKSVNNDMIVTDNNGAV KFPQLCKFCDVRFSTCDNQKSCMSNCSITSICEKPQEVCVAVWRKNDENITLET VCHDPKLPYHDFILEDAASPKCIMKEKKKPGETFFMCSCSSDECNDNIIFSEEYN TSNPDSSSSGSSSSGSSSSGEVQLLESGGGLVQPGGSLRLSCAASGFTFSSFSMS WVRQAPGKGLEWVSSISGSSGTTYYADSVKGRFTISRDNSKNTLYLQMNSLRA EDTAVYYCAKPFPYFDYWGQGTLVTVSSGGGGSGGGGSGGGGSEIVLTQSPG TLSLSPGERATLSCRASQSVSSSFLAWYQQKPGQAPRLLIYYASSRATGIPDRFS GSGSGTDFTLTISRLEPEDFAVYYCQQTGRIPPTFGQGTKVEIK |
| 71 | Anti-PD1-LC-IGKC-anti-EDB-FN scfv-TGFβ Trap | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECSSSSGSSSSGSSSSGEVQLLESGGGLVQPGGSLRLSCAASGFTFSSFSMSWV RQAPGKGLEWVSSISGSSGTTYYADSVKGRFTISRDNSKNTLYLQMNSLRAED TAVYYCAKPFPYFDYWGQGTLVTVSSGGGGSGGGGSGGGGSEIVLTQSPGTLS LSPGERATLSCRASQSVSSSFLAWYQQKPGQAPRLLIYYASSRATGIPDRFSGSG SGTDFTLTISRLEPEDFAVYYCQQTGRIPPTFGQGTKVEIKGGGGSGGGGSIPPH VQKSVNNDMIVTDNNGAVKFPQLCKFCDVRFSTCDNQKSCMSNCSITSICEKP QEVCVAVWRKNDENITLETVCHDPKLPYHDFILEDAASPKCIMKEKKKPGETF FMCSCSSDECNDNIIFSEEYNTSNPD |
| 72 | Anti-PD1-LC-IGKC-TGFβ Trap-anti-EDB-FN scfv | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECGGGGSGGGGSIPPHVQKSVNNDMIVTDNNGAVKFPQLCKFCDVRFSTCD NQKSCMSNCSITSICEKPQEVCVAVWRKNDENITLETVCHDPKLPYHDFILEDA ASPKCIMKEKKKPGETFFMCSCSSDECNDNIIFSEEYNTSNPDSSSSGSSSSGSSS SGEVQLLESGGGLVQPGGSLRLSCAASGFTFSSFSMSWVRQAPGKGLEWVSSIS GSSGTTYYADSVKGRFTISRDNSKNTLYLQMNSLRAEDTAVYYCAKPFPYFDY WGQGTLVTVSSGGGGSGGGGSGGGGSEIVLTQSPGTLSLSPGERATLSCRASQ SVSSSFLAWYQQKPGQAPRLLIYYASSRATGIPDRFSGSGSGTDFTLTISRLEPE DFAVYYCQQTGRIPPTFGQGTKVEIK |
| 73 | Anti-EDB-FN scfv-TGFβ Trap-anti-PD1-HC-IGHG | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKGGGGSGGGGSIPPHVQKSVNNDMIVTDNN GAVKFPQLCKFCDVRFSTCDNQKSCMSNCSITSICEKPQEVCVAVWRKNDENI TLETVCHDPKLPYHDFILEDAASPKCIMKEKKKPGETFFMCSCSSDECNDNIIFS EEYNTSNPDSSSSGSSSSGSSSSGQVQLVQSGVEVKKPGASVKVSCKASGYTFT NYYMYWVRQAPGQGLEWMGGINPSNGGTNFNEKFKNRVTLTTDSSTTTAYM ELKSLQFDDTAVYYCARRDYRFDMGFDYWGQGTTVTVSSASTKGPSVFPLAP CSRSTSESTAALGCLVKDYFPEPVTVSWNSGALTSGVHTFPAVLQSSGLYSLSS VVTVPSSSLGTKTYTCNVDHKPSNTKVDKRVESKYGPPCPPCPAPEFLGGPSVF LFPPKPKDTLMISRTPEVTCVVVDVSQEDPEVQFNWYVDGVEVHNAKTKPREE QFNSTYRVVSVLTVLHQDWLNGKEYKCKVSNKGLPSSIEKTISKAKGQPREPQ VYTLPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGK |
| 74 | TGFβ Trap-anti-EDB-FN scfv-anti-PD1-HC-IGHG | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDSSSSGSSSSGSSSSGEVQLLESGGGLV QPGGSLRLSCAASGFTFSSFSMSWVRQAPGKGLEWVSSISGSSGTTYYADSVK GRFTISRDNSKNTLYLQMNSLRAEDTAVYYCAKPFPYFDYWGQGTLVTVSSG GGGSGGGGSGGGGSEIVLTQSPGTLSLSPGERATLSCRASQSVSSSFLAWYQQK PGQAPRLLIYYASSRATGIPDRFSGSGSGTDFTLTISRLEPEDFAVYYCQQTGRIP PTFGQGTKVEIKSSSSGSSSSGSSSSGQVQLVQSGVEVKKPGASVKVSCKASGY TFTNYYMYWVRQAPGQGLEWMGGINPSNGGTNFNEKFKNRVTLTTDSSTTTA YMELKSLQFDDTAVYYCARRDYRFDMGFDYWGQGTTVTVSSASTKGPSVFPL APCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPPCPAPEFLGGPS VFLFPPKPKDTLMISRTPEVTCVVVDVSQEDPEVQFNWYVDGVEVHNAKTKP REEQFNSTYRVVSVLTVLHQDWLNGKEYKCKVSNKGLPSSIEKTISKAKGQPR EPQVYTLPPSQEEMTKNQVSLTCLVKGFYPSDIAVEWESNGQPENNYKTTPPV LDSDGSFFLYSRLTVDKSRWQEGNVFSCSVMHEALHNHYTQKSLSLSLGK |
| 75 | Anti-PD1-scfv-hFc-anti-EDB-FN scfv-TGFβ Trap | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKSSSSGSSSSGSSSSGEVQLLESGGGLVQPGGSLRLSCAAS GFTFSSFSMSWVRQAPGKGLEWVSSISGSSGTTYYADSVKGRFTISRDNSKNTL YLQMNSLRAEDTAVYYCAKPFPYFDYWGQGTLVTVSSGGGGSGGGGSGGGG SEIVLTQSPGTLSLSPGERATLSCRASQSVSSSFLAWYQQKPGQAPRLLIYYASS RATGIPDRFSGSGSGTDFTLTISRLEPEDFAVYYCQQTGRIPPTFGQGTKVEIKG GGGSGGGGSIPPHVQKSVNNDMIVTDNNGAVKFPQLCKFCDVRFSTCDNQKS CMSNCSITSICEKPQEVCVAVWRKNDENITLETVCHDPKLPYHDFILEDAASPK CIMKEKKKPGETFFMCSCSSDECNDNIIFSEEYNTSNPD |
| 76 | Anti-PD1-scfv-hFc-TGFβ Trap-anti-EDB-FN scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKSSSSGSSSSGSSSSGEVQLLESGGGLVQPGGSLRLSCAAS GFTFSSFSMSWVRQAPGKGLEWVSSISGSSGTTYYADSVKGRFTISRDNSKNTL YLQMNSLRAEDTAVYYCAKPFPYFDYWGQGTLVTVSSGGGGSGGGGSGGGG SEIVLTQSPGTLSLSPGERATLSCRASQSVSSSFLAWYQQKPGQAPRLLIYYASS RATGIPDRFSGSGSGTDFTLTISRLEPEDFAVYYCQQTGRIPPTFGQGTKVEIKG GGGSGGGGSIPPHVQKSVNNDMIVTDNNGAVKFPQLCKFCDVRFSTCDNQKS CMSNCSITSICEKPQEVCVAVWRKNDENITLETVCHDPKLPYHDFILEDAASPK CIMKEKKKPGETFFMCSCSSDECNDNIIFSEEYNTSNPD |
| 77 | Anti-EDB-FN scfv-TGFβ Trap hFc-anti-PD1 scfv | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKGGGGSGGGGSIPPHVQKSVNNDMIVTDNN GAVKFPQLCKFCDVRFSTCDNQKSCMSNCSITSICEKPQEVCVAVWRKNDENI TLETVCHDPKLPYHDFILEDAASPKCIMKEKKKPGETFFMCSCSSDECNDNIIFS EEYNTSNPDSSSSGSSSSGSSSSGESKYGPPCPPCPAPEFLGGPSVFLFPPKPKDT LMISRTPEVTCVVVDVSQEDPEVQFNWYVDGVEVHNAKTKPREEQFNSTYRV VSVLTVLHQDWLNGKEYKCKVSNKGLPSSIEKTISKAKGQPREPQVYTLPPSQ EEMTKNQVSLTCLVKGFYPSDIAVEWESNGQPENNYKTTPPVLDSDGSFFLYS RLTVDKSRWQEGNVFSCSVMHEALHNHYTQKSLSLSLGKSSSSGSSSSGSSSSG QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIK |
| 78 | Anti-PD1-scfv-anti-EDB-FN scfv-TGFβ Trap | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGEVQL LESGGGLVQPGGSLRLSCAASGFTFSSFSMSWVRQAPGKGLEWVSSISGSSGTT YYADSVKGRFTISRDNSKNTLYLQMNSLRAEDTAVYYCAKPFPYFDYWGQGT LVTVSSGGGGSGGGGSGGGGSEIVLTQSPGTLSLSPGERATLSCRASQSVSSSFL AWYQQKPGQAPRLLIYYASSRATGIPDRFSGSGSGTDFTLTISRLEPEDFAVYY CQQTGRIPPTFGQGTKVEIKSSSSGSSSSGSSSSGIPPHVQKSVNNDMIVTDNNG AVKFPQLCKFCDVRFSTCDNQKSCMSNCSITSICEKPQEVCVAVWRKNDENIT LETVCHDPKLPYHDFILEDAASPKCIMKEKKKPGETFFMCSCSSDECNDNIIFSE EYNTSNPD |
| 79 | Anti-PD1-scfv-TGFβ Trap-anti-EDB-FN scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGIPPHV QKSVNNDMIVTDNNGAVKFPQLCKFCDVRFSTCDNQKSCMSNCSITSICEKPQ EVCVAVWRKNDENITLETVCHDPKLPYHDFILEDAASPKCIMKEKKKPGETFF MCSCSSDECNDNIIFSEEYNTSNPDEVQLLESGGGLVQPGGSLRLSCAASGFTFS SFSMSWVRQAPGKGLEWVSSISGSSGTTYYADSVKGRFTISRDNSKNTLYLQM NSLRAEDTAVYYCAKPFPYFDYWGQGTLVTVSSGGGGSGGGGSGGGGSEIVL TQSPGTLSLSPGERATLSCRASQSVSSSFLAWYQQKPGQAPRLLIYYASSRATGI PDRFSGSGSGTDFTLTISRLEPEDFAVYYCQQTGRIPPTFGQGTKVEIK |
| 80 | TGFβ Trap-Anti-PD1-scfv-anti-EDB-FN scfv | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDSSSSGSSSSGSSSSGQVQLVQSGVEVK KPGASVKVSCKASGYTFTNYYMYWVRQAPGQGLEWMGGINPSNGGTNFNEK FKNRVTLTTDSSTTTAYMELKSLQFDDTAVYYCARRDYRFDMGFDYWGQGT TVTVSSGGGGSGGGGSGGGGSEIVLTQSPATLSLSPGERATLSCRASKGVSTSG YSYLHWYQQKPGQAPRLLIYLASYLESGVPARFSGSGSGTDFTLTISSLEPEDFA VYYCQHSRDLPLTFGGGTKVEIKSSSSGSSSSGSSSSGEVQLLESGGGLVQPGGS LRLSCAASGFTFSSFSMSWVRQAPGKGLEWVSSISGSSGTTYYADSVKGRFTIS RDNSKNTLYLQMNSLRAEDTAVYYCAKPFPYFDYWGQGTLVTVSSGGGGSG GGGSGGGGSEIVLTQSPGTLSLSPGERATLSCRASQSVSSSFLAWYQQKPGQAP RLLIYYASSRATGIPDRFSGSGSGTDFTLTISRLEPEDFAVYYCQQTGRIPPTFGQ GTKVEIK |
| 81 | Anti-PD1-HC-IGHG-FEBM-anti-TGFβ1 scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKSSSSGSSSSGSSSSGSSSPIQGSWTWENG KWTWKGIIRLEQGGGGSGGGGSGGGGSQVQLVQSGAEVKKPGSSVKVSCKAS GGTFSSYAISWVRQAPGQGLEWMGGIIPIFGTANYAQKFQGRVTITADESTSTA YMELSSLRSEDTAVYYCARGLWEVRALPSVYWGQGTLVTVSSSSSSGSSSSGS SSSGSYELTQPPSVSVAPGQTARITCGANDIGSKSVHWYQQKAGQAPVLVVSE DIIRPSGIPERISGSNSGNTATLTISRVEAGDEADYYCQVWDRDSDQYVFGTGT KVTVL |
| 82 | Anti-PD1-HC-IGHG-anti-TGFβ1 scfv-FEBM | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKGGGGSGGGGSGGGGSQVQLVQSGAEVK KPGSSVKVSCKASGGTFSSYAISWVRQAPGQGLEWMGGIIPIFGTANYAQKFQ GRVTITADESTSTAYMELSSLRSEDTAVYYCARGLWEVRALPSVYWGQGTLV TVSSSSSSGSSSSGSSSSGSYELTQPPSVSVAPGQTARITCGANDIGSKSVHWYQ QKAGQAPVLVVSEDIIRPSGIPERISGSNSGNTATLTISRVEAGDEADYYCQVW DRDSDQYVFGTGTKVTVLGGGGSGGGGSGGGGSSSSPIQGSWTWENGKWTW KGIIRLEQ |
| 83 | Anti-PD1-LC-IGKC-FEBM-anti-TGFβ1 scfv | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECGGGGSGGGGSGGGGSSSSPIQGSWTWENGKWTWKGIIRLEQGGGGSGGG GSGGGGSQVQLVQSGAEVKKPGSSVKVSCKASGGTFSSYAISWVRQAPGQGL EWMGGIIPIFGTANYAQKFQGRVTITADESTSTAYMELSSLRSEDTAVYYCARG LWEVRALPSVYWGQGTLVTVSSSSSSGSSSSGSSSSGSYELTQPPSVSVAPGQT ARITCGANDIGSKSVHWYQQKAGQAPVLVVSEDIIRPSGIPERISGSNSGNTATL TISRVEAGDEADYYCQVWDRDSDQYVFGTGTKVTVL |
| 84 | Anti-PD1-LC-IGKC-anti-TGFβ1 scfv-FEBM | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECGGGGSGGGGSGGGGSQVQLVQSGAEVKKPGSSVKVSCKASGGTFSSYAI SWVRQAPGQGLEWMGGIIPIFGTANYAQKFQGRVTITADESTSTAYMELSSLR SEDTAVYYCARGLWEVRALPSVYWGQGTLVTVSSSSSSGSSSSGSSSSGSYELT QPPSVSVAPGQTARITCGANDIGSKSVHWYQQKAGQAPVLVVSEDIIRPSGIPE RISGSNSGNTATLTISRVEAGDEADYYCQVWDRDSDQYVFGTGTKVTVLGGG SGGGGSSSSPIQGSWTWENGKWTWKGIIRLEQ |
| 85 | Anti-TGFβ1 scfv-FEBM-anti-PD1-HC-IGHG | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGSGGGGSSSSPIQGSWTWENG KWTWKGIIRLEQSSSSGSSSSGSSSSGQVQLVQSGVEVKKPGASVKVSCKASG YTFTNYYMYWVRQAPGQGLEWMGGINPSNGGTNFNEKFKNRVTLTTDSSTTT AYMELKSLQFDDTAVYYCARRDYRFDMGFDYWGQGTTVTVSSASTKGPSVF PLAPCSRSTSESTAALGCLVKDYFPEPVTVSWNSGALTSGVHTFPAVLQSSGLY SLSSVVTVPSSSLGTKTYTCNVDHKPSNTKVDKRVESKYGPPCPPCPAPEFLGG PSVFLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAKGQP REPQVYTLPPSQEEMTKNQVSLTCLVKGFYPSDIAVEWESNGQPENNYKTTPP VLDSDGSFFLYSRLTVDKSRWQEGNVFSCSVMHEALHNHYTQKSLSLSLGK |
| 86 | FEBM-anti-TGFβ1 scfv-anti-PD1-HC-IGHG | SSSPIQGSWTWENGKWTWKGIIRLEQGGGGSGGGGSGGGGSQVQLVQSGAEV KKPGSSVKVSCKASGGTFSSYAISWVRQAPGQGLEWMGGIIPIFGTANYAQKF QGRVTITADESTSTAYMELSSLRSEDTAVYYCARGLWEVRALPSVYWGQGTL VTVSSSSSSGSSSSGSSSSGSYELTQPPSVSVAPGQTARITCGANDIGSKSVHWY QQKAGQAPVLVVSEDIIRPSGIPERISGSNSGNTATLTISRVEAGDEADYYCQV WDRDSDQYVFGTGTKVTVLGGGGSGGGGSGGGGSQVQLVQSGVEVKKPGAS VKVSCKASGYTFTNYYMYWVRQAPGQGLEWMGGINPSNGGTNFNEKFKNRV TLTTDSSTTTAYMELKSLQFDDTAVYYCARRDYRFDMGFDYWGQGTTVTVSS ASTKGPSVFPLAPCSRSTSESTAALGCLVKDYFPEPVTVSWNSGALTSGVHTFP AVLQSSGLYSLSSVVTVPSSSLGTKTYTCNVDHKPSNTKVDKRVESKYGPPCPP CPAPEFLGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSIE KTISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLGK |
| 87 | Anti-PD1-scfv-hFc-FEBM-anti-TGFβ1 scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKGGGGSGGGGSSSSPIQGSWTWENGKWTWKGIIRLEQG GGGSGGGGSGGGGSQVQLVQSGAEVKKPGSSVKVSCKASGGTFSSYAISWVR QAPGQGLEWMGGIIPIFGTANYAQKFQGRVTITADESTSTAYMELSSLRSEDTA VYYCARGLWEVRALPSVYWGQGTLVTVSSSSSSGSSSSGSSSSGSYELTQPPSV SVAPGQTARITCGANDIGSKSVHWYQQKAGQAPVLVVSEDIIRPSGIPERISGSN SGNTATLTISRVEAGDEADYYCQVWDRDSDQYVFGTGTKVTVL |
| 88 | Anti-PD1-scfv-hFc-anti-TGFβ1 scfv-FEBM | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKGGGGSGGGGSGGGGSQVQLVQSGAEVKKPGSSVKVSC KASGGTFSSYAISWVRQAPGQGLEWMGGIIPIFGTANYAQKFQGRVTITADEST STAYMELSSLRSEDTAVYYCARGLWEVRALPSVYWGQGTLVTVSSSSSSGSSS SGSSSSGSYELTQPPSVSVAPGQTARITCGANDIGSKSVHWYQQKAGQAPVLV VSEDIIRPSGIPERISGSNSGNTATLTISRVEAGDEADYYCQVWDRDSDQYVFGT GTKVTVLGGGGSGGGGSSSSPIQGSWTWENGKWTWKGIIRLEQ |
| 89 | anti-TGFβ1 scfv-FEBM-hFc-anti-PD1 scfv | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSSSSPIQGSWTWEN GKWTWKGIIRLEQGGGGSGGGGSGGGGSESKYGPPCPPCPAPEFLGGPSVFLFP PKPKDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPREPQVY TLPPSQEEMTKNQVSLTCLVKGFYPSDIAVEWESNGQPENNYKTTPPVLDSDG SFFLYSRLTVDKSRWQEGNVFSCSVMHEALHNHYTQKSLSLSLGKSSSSGSSSS GSSSSGQVQLVQSGVEVKKPGASVKVSCKASGYTFTNYYMYWVRQAPGQGL EWMGGINPSNGGTNFNEKFKNRVTLTTDSSTTTAYMELKSLQFDDTAVYYCA RRDYRFDMGFDYWGQGTTVTVSSGGGGSGGGGSGGGGSEIVLTQSPATLSLSP GERATLSCRASKGVSTSGYSYLHWYQQKPGQAPRLLIYLASYLESGVPARFSG SGSGTDFTLTISSLEPEDFAVYYCQHSRDLPLTFGGGTKVEIK |
| 90 | Anti-PD1-scfv-FEBM-anti-TGFβ1 scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKGGGGSGGGGSSSSPIQGS WTWENGKWTWKGIIRLEQGGGGSGGGGSGGGGSQVQLVQSGAEVKKPGSSV KVSCKASGGTFSSYAISWVRQAPGQGLEWMGGIIPIFGTANYAQKFQGRVTIT ADESTSTAYMELSSLRSEDTAVYYCARGLWEVRALPSVYWGQGTLVTVSSSSS SGSSSSGSSSSGSYELTQPPSVSVAPGQTARITCGANDIGSKSVHWYQQKAGQA PVLVVSEDIIRPSGIPERISGSNSGNTATLTISRVEAGDEADYYCQVWDRDSDQY VFGTGTKVTVL |
| 91 | Anti-PD1-scfv-anti-TGFβ1 scfv-FEBM | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGQVQL VQSGAEVKKPGSSVKVSCKASGGTFSSYAISWVRQAPGQGLEWMGGIIPIFGT ANYAQKFQGRVTITADESTSTAYMELSSLRSEDTAVYYCARGLWEVRALPSV YWGQGTLVTVSSSSSSGSSSSGSSSSGSYELTQPPSVSVAPGQTARITCGANDIG SKSVHWYQQKAGQAPVLVVSEDIIRPSGIPERISGSNSGNTATLTISRVEAGDEA DYYCQVWDRDSDQYVFGTGTKVTVLGGGGSGGGGSSSSPIQGSWTWENGKW TWKGIIRLEQ |
| 92 | Anti-TGFβ1 scfv-FEBM-anti-PD1 scfv | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSSSSPIQGSWTWEN GKWTWKGIIRLEQSSSSGSSSSGSSSSGQVQLVQSGVEVKKPGASVKVSCKAS GYTFTNYYMYWVRQAPGQGLEWMGGINPSNGGTNFNEKFKNRVTLTTDSST TTAYMELKSLQFDDTAVYYCARRDYRFDMGFDYWGQGTTVTVSSGGGGSGG GGSGGGGSEIVLTQSPATLSLSPGERATLSCRASKGVSTSGYSYLHWYQQKPG QAPRLLIYLASYLESGVPARFSGSGSGTDFTLTISSLEPEDFAVYYCQHSRDLPL TFGGGTKVEIK |
| 93 | Anti-PD1-HC-IGHG-anti-EDB-FN scfv-anti-TGFβ1 scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKSSSSGSSSSGSSSSGEVQLLESGGGLVQP GGSLRLSCAASGFTFSSFSMSWVRQAPGKGLEWVSSISGSSGTTYYADSVKGR FTISRDNSKNTLYLQMNSLRAEDTAVYYCAKPFPYFDYWGQGTLVTVSSGGG GSGGGGSGGGGSEIVLTQSPGTLSLSPGERATLSCRASQSVSSSFLAWYQQKPG QAPRLLIYYASSRATGIPDRFSGSGSGTDFTLTISRLEPEDFAVYYCQQTGRIPPT FGQGTKVEIKSSSSGSSSSGSSSSGQVQLVQSGAEVKKPGSSVKVSCKASGGTF SSYAISWVRQAPGQGLEWMGGIIPIFGTANYAQKFQGRVTITADESTSTAYMEL SSLRSEDTAVYYCARGLWEVRALPSVYWGQGTLVTVSSSSSSGSSSSGSSSSGS YELTQPPSVSVAPGQTARITCGANDIGSKSVHWYQQKAGQAPVLVVSEDIIRPS GIPERISGSNSGNTATLTISRVEAGDEADYYCQVWDRDSDQYVFGTGTKVTVL |
| 94 | Anti-PD1-HC-IGHG-anti-TGFβ1 scfv-anti-EDB-FN scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGKGGGGSGGGGSGGGGSQVQLVQSGAEVK KPGSSVKVSCKASGGTFSSYAISWVRQAPGQGLEWMGGIIPIFGTANYAQKFQ GRVTITADESTSTAYMELSSLRSEDTAVYYCARGLWEVRALPSVYWGQGTLV TVSSSSSSGSSSSGSSSSGSYELTQPPSVSVAPGQTARITCGANDIGSKSVHWYQ QKAGQAPVLVVSEDIIRPSGIPERISGSNSGNTATLTISRVEAGDEADYYCQVW DRDSDQYVFGTGTKVTVLSSSSGSSSSGSSSSGEVQLLESGGGLVQPGGSLRLS CAASGFTFSSFSMSWVRQAPGKGLEWVSSISGSSGTTYYADSVKGRFTISRDNS KNTLYLQMNSLRAEDTAVYYCAKPFPYFDYWGQGTLVTVSSGGGGSGGGGS GGGGSEIVLTQSPGTLSLSPGERATLSCRASQSVSSSFLAWYQQKPGQAPRLLIY YASSRATGIPDRFSGSGSGTDFTLTISRLEPEDFAVYYCQQTGRIPPTFGQGTKV EIK |
| 95 | Anti-PD1-LC-IGKC-anti-EDB-FN scfv-anti-TGFβ1 scfv | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECSSSSGSSSSGSSSSGEVQLLESGGGLVQPGGSLRLSCAASGFTFSSFSMSWV RQAPGKGLEWVSSISGSSGTTYYADSVKGRFTISRDNSKNTLYLQMNSLRAED TAVYYCAKPFPYFDYWGQGTLVTVSSGGGGSGGGGSGGGGSEIVLTQSPGTLS LSPGERATLSCRASQSVSSSFLAWYQQKPGQAPRLLIYYASSRATGIPDRFSGSG SGTDFTLTISRLEPEDFAVYYCQQTGRIPPTFGQGTKVEIKSSSSGSSSSGSSSSG QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVL |
| 96 | Anti-PD1-LC-IGKC-anti-TGFβ1 scfv-anti-EDB-FN scfv | EIVLTQSPATLSLSPGERATLSCRASKGVSTSGYSYLHWYQQKPGQAPRLLIYL ASYLESGVPARFSGSGSGTDFTLTISSLEPEDFAVYYCQHSRDLPLTFGGGTKVE IKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNR GECGGGGSGGGGSGGGGSQVQLVQSGAEVKKPGSSVKVSCKASGGTFSSYAI SWVRQAPGQGLEWMGGIIPIFGTANYAQKFQGRVTITADESTSTAYMELSSLR SEDTAVYYCARGLWEVRALPSVYWGQGTLVTVSSSSSSGSSSSGSSSSGSYELT QPPSVSVAPGQTARITCGANDIGSKSVHWYQQKAGQAPVLVVSEDIIRPSGIPE RISGSNSGNTATLTISRVEAGDEADYYCQVWDRDSDQYVFGTGTKVTVLSSSS GSSSSGSSSSGEVQLLESGGGLVQPGGSLRLSCAASGFTFSSFSMSWVRQAPGK GLEWVSSISGSSGTTYYADSVKGRFTISRDNSKNTLYLQMNSLRAEDTAVYYC AKPFPYFDYWGQGTLVTVSSGGGGSGGGGSGGGGSEIVLTQSPGTLSLSPGER ATLSCRASQSVSSSFLAWYQQKPGQAPRLLIYYASSRATGIPDRFSGSGSGTDF TLTISRLEPEDFAVYYCQQTGRIPPTFGQGTKVEIK |
| 97 | Anti-EDB-FN scfv-anti-TGFβ1 scfv-anti-PD1-HC-IGHG | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKSSSSGSSSSGSSSSGQVQLVQSGAEVKKPG SSVKVSCKASGGTFSSYAISWVRQAPGQGLEWMGGIIPIFGTANYAQKFQGRV TITADESTSTAYMELSSLRSEDTAVYYCARGLWEVRALPSVYWGQGTLVTVSS SSSSGSSSSGSSSSGSYELTQPPSVSVAPGQTARITCGANDIGSKSVHWYQQKA GQAPVLVVSEDIIRPSGIPERISGSNSGNTATLTISRVEAGDEADYYCQVWDRDS DQYVFGTGTKVTVLGGGGSGGGGSGGGGSQVQLVQSGVEVKKPGASVKVSC KASGYTFTNYYMYWVRQAPGQGLEWMGGINPSNGGTNFNEKFKNRVTLTTD SSTTTAYMELKSLQFDDTAVYYCARRDYRFDMGFDYWGQGTTVTVSSASTK GPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPPCPAP EFLGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISK AKGQPREPQVYTLPPSQEEMTKNQVSLTCLVKGFYPSDIAVEWESNGQPENNY KTTPPVLDSDGSFFLYSRLTVDKSRWQEGNVFSCSVMHEALHNHYTQKSLSLS LGK |
| 98 | Anti-TGFβ1 scfv-anti-EDB-FN scfv-anti-PD1-HC-IGHG | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSGGGGSEVQLLESG GGLVQPGGSLRLSCAASGFTFSSFSMSWVRQAPGKGLEWVSSISGSSGTTYYA DSVKGRFTISRDNSKNTLYLQMNSLRAEDTAVYYCAKPFPYFDYWGQGTLVT VSSGGGGSGGGGSGGGGSEIVLTQSPGTLSLSPGERATLSCRASQSVSSSFLAW YQQKPGQAPRLLIYYASSRATGIPDRFSGSGSGTDFTLTISRLEPEDFAVYYCQQ TGRIPPTFGQGTKVEIKSSSSGSSSSGSSSSGQVQLVQSGVEVKKPGASVKVSCK ASGYTFTNYYMYWVRQAPGQGLEWMGGINPSNGGTNFNEKFKNRVTLTTDS STTTAYMELKSLQFDDTAVYYCARRDYRFDMGFDYWGQGTTVTVSSASTKG PSVFPLAPCSRSTSESTAALGCLVKDYFPEPVTVSWNSGALTSGVHTFPAVLQS SGLYSLSSVVTVPSSSLGTKTYTCNVDHKPSNTKVDKRVESKYGPPCPPCPAPE FLGGPSVFLFPPKPKDTLMISRTPEVTCVVVDVSQEDPEVQFNWYVDGVEVHN AKTKPREEQFNSTYRVVSVLTVLHQDWLNGKEYKCKVSNKGLPSSIEKTISKA KGQPREPQVYTLPPSQEEMTKNQVSLTCLVKGFYPSDIAVEWESNGQPENNYK TTPPVLDSDGSFFLYSRLTVDKSRWQEGNVFSCSVMHEALHNHYTQKSLSLSL GK |
| 99 | Anti-PDI-scfv-hFc-anti-EDB-FN scfv-anti-TGFβ1 scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKGGGGSGGGGSGGGGSEVQLLESGGGLVQPGGSLRLSC AASGFTFSSFSMSWVRQAPGKGLEWVSSISGSSGTTYYADSVKGRFTISRDNSK NTLYLQMNSLRAEDTAVYYCAKPFPYFDYWGQGTLVTVSSGGGGSGGGGSG GGGSEIVLTQSPGTLSLSPGERATLSCRASQSVSSSFLAWYQQKPGQAPRLLIYY ASSRATGIPDRFSGSGSGTDFTLTISRLEPEDFAVYYCQQTGRIPPTFGQGTKVEI KGGGGSGGGGSGGGGSQVQLVQSGAEVKKPGSSVKVSCKASGGTFSSYAISW VRQAPGQGLEWMGGIIPIFGTANYAQKFQGRVTITADESTSTAYMELSSLRSED TAVYYCARGLWEVRALPSVYWGQGTLVTVSSSSSSGSSSSGSSSSGSYELTQPP SVSVAPGQTARITCGANDIGSKSVHWYQQKAGQAPVLVVSEDIIRPSGIPERISG SNSGNTATLTISRVEAGDEADYYCQVWDRDSDQYVFGTGTKVTVL |
| 100 | Anti-PD1-scfv-hFc-anti-TGFβ1 scfv-anti-EDB-FN scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGESKY GPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEAL HNHYTQKSLSLSLGKGGGGSGGGGSGGGGSQVQLVQSGAEVKKPGSSVKVSC KASGGTFSSYAISWVRQAPGQGLEWMGGIIPIFGTANYAQKFQGRVTITADEST STAYMELSSLRSEDTAVYYCARGLWEVRALPSVYWGQGTLVTVSSSSSSGSSS SGSSSSGSYELTQPPSVSVAPGQTARITCGANDIGSKSVHWYQQKAGQAPVLV VSEDIIRPSGIPERISGSNSGNTATLTISRVEAGDEADYYCQVWDRDSDQYVFGT GTKVTVLGGGGSGGGGSGGGGSEVQLLESGGGLVQPGGSLRLSCAASGFTFSS FSMSWVRQAPGKGLEWVSSISGSSGTTYYADSVKGRFTISRDNSKNTLYLQMN |
| | | SLRAEDTAVYYCAKPFPYFDYWGQGTLVTVSSGGGGSGGGGSGGGGSEIVLT QSPGTLSLSPGERATLSCRASQSVSSSFLAWYQQKPGQAPRLLIYYASSRATGIP DRFSGSGSGTDFTLTISRLEPEDFAVYYCQQTGRIPPTFGQGTKVEIK |
| 101 | Anti-EDB-FN scfv-anti-TGFβ1 scfv-hFc-anti-PD1 scfv | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSGGGGSGGGGSGGGGSEIVLTQSPGTLSLSPGERATLSCRASQSV SSSFLAWYQQKPGQAPRLLIYYASSRATGIPDRFSGSGSGTDFTLTISRLEPEDF AVYYCQQTGRIPPTFGQGTKVEIKGGGGSGGGGSGGGGSQVQLVQSGAEVKK PGSSVKVSCKASGGTFSSYAISWVRQAPGQGLEWMGGIIPIFGTANYAQKFQG RVTITADESTSTAYMELSSLRSEDTAVYYCARGLWEVRALPSVYWGQGTLVT VSSSSSSGSSSSGSSSSGSYELTQPPSVSVAPGQTARITCGANDIGSKSVHWYQQ KAGQAPVLVVSEDIIRPSGIPERISGSNSGNTATLTISRVEAGDEADYYCQVWD RDSDQYVFGTGTKVTVLGGGGSGGGGSGGGGSESKYGPPCPPCPAPEFLGGPS VFLFPPKPKDTLMISRTPEVTCVVVDVSQEDPEVQFNWYVDGVEVHNAKTKP REEQFNSTYRVVSVLTVLHQDWLNGKEYKCKVSNKGLPSSIEKTISKAKGQPR EPQVYTLPPSQEEMTKNQVSLTCLVKGFYPSDIAVEWESNGQPENNYKTTPPV LDSDGSFFLYSRLTVDKSRWQEGNVFSCSVMHEALHNHYTQKSLSLSLGKSSS SGSSSSGSSSSGQVQLVQSGVEVKKPGASVKVSCKASGYTFTNYYMYWVRQA PGQGLEWMGGINPSNGGTNFNEKFKNRVTLTTDSSTTTAYMELKSLQFDDTA VYYCARRDYRFDMGFDYWGQGTTVTVSSGGGGSGGGGSGGGGSEIVLTQSP ATLSLSPGERATLSCRASKGVSTSGYSYLHWYQQKPGQAPRLLIYLASYLESG VPARFSGSGSGTDFTLTISSLEPEDFAVYYCQHSRDLPLTFGGGTKVEIK |
| 102 | Anti-PD1-scfv-anti-EDB-FN scfv-anti-TGFβ1 scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGEVQL LESGGGLVQPGGSLRLSCAASGFTFSSFSMSWVRQAPGKGLEWVSSISGSSGTT YYADSVKGRFTISRDNSKNTLYLQMNSLRAEDTAVYYCAKPFPYFDYWGQGT LVTVSSGGGGSGGGGSGGGGSEIVLTQSPGTLSLSPGERATLSCRASQSVSSSFL AWYQQKPGQAPRLLIYYASSRATGIPDRFSGSGSGTDFTLTISRLEPEDFAVYY CQQTGRIPPTFGQGTKVEIKGGGGSGGGGSGGGGSQVQLVQSGAEVKKPGSSV KVSCKASGGTFSSYAISWVRQAPGQGLEWMGGIIPIFGTANYAQKFQGRVTIT ADESTSTAYMELSSLRSEDTAVYYCARGLWEVRALPSVYWGQGTLVTVSSSSS SGSSSSGSSSSGSYELTQPPSVSVAPGQTARITCGANDIGSKSVHWYQQKAGQA PVLVVSEDIIRPSGIPERISGSNSGNTATLTISRVEAGDEADYYCQVWDRDSDQY VFGTGTKVTVL |
| 103 | Anti-PDI-scfv-anti-TGFβ1 scfv-anti-EDB-FN scfv | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSGGGGSGGGGSGGGGSEIVLTQSPATLSLSPGERATL SCRASKGVSTSGYSYLHWYQQKPGQAPRLLIYLASYLESGVPARFSGSGSGTD FTLTISSLEPEDFAVYYCQHSRDLPLTFGGGTKVEIKSSSSGSSSSGSSSSGQVQL VQSGAEVKKPGSSVKVSCKASGGTFSSYAISWVRQAPGQGLEWMGGIIPIFGT ANYAQKFQGRVTITADESTSTAYMELSSLRSEDTAVYYCARGLWEVRALPSV YWGQGTLVTVSSSSSSGSSSSGSSSSGSYELTQPPSVSVAPGQTARITCGANDIG SKSVHWYQQKAGQAPVLVVSEDIIRPSGIPERISGSNSGNTATLTISRVEAGDEA DYYCQVWDRDSDQYVFGTGTKVTVLGGGGSGGGGSGGGGSEVQLLESGGGL VQPGGSLRLSCAASGFTFSSFSMSWVRQAPGKGLEWVSSISGSSGTTYYADSV KGRFTISRDNSKNTLYLQMNSLRAEDTAVYYCAKPFPYFDYWGQGTLVTVSS GGGGSGGGGSGGGGSEIVLTQSPGTLSLSPGERATLSCRASQSVSSSFLAWYQQ KPGQAPRLLIYYASSRATGIPDRFSGSGSGTDFTLTISRLEPEDFAVYYCQQTGR IPPTFGQGTKVEIK |
| 104 | Anti-TGFβ1 scfv-anti-EDB-FN scfv-anti-PD1 scfv | QVQLVQSGAEVKKPGSSVKVSCKASGGTFSSYAISWVRQAPGQGLEWMGGIIP IFGTANYAQKFQGRVTITADESTSTAYMELSSLRSEDTAVYYCARGLWEVRAL PSVYWGQGTLVTVSSSSSSGSSSSGSSSSGSYELTQPPSVSVAPGQTARITCGAN DIGSKSVHWYQQKAGQAPVLVVSEDIIRPSGIPERISGSNSGNTATLTISRVEAG DEADYYCQVWDRDSDQYVFGTGTKVTVLGGGGSGGGGSGGGGSEVQLLESG GGLVQPGGSLRLSCAASGFTFSSFSMSWVRQAPGKGLEWVSSISGSSGTTYYA DSVKGRFTISRDNSKNTLYLQMNSLRAEDTAVYYCAKPFPYFDYWGQGTLVT VSSGGGGSGGGGSGGGGSEIVLTQSPGTLSLSPGERATLSCRASQSVSSSFLAW YQQKPGQAPRLLIYYASSRATGIPDRFSGSGSGTDFTLTISRLEPEDFAVYYCQQ TGRIPPTFGQGTKVEIKSSSSGSSSSGSSSSGQVQLVQSGVEVKKPGASVKVSCK ASGYTFTNYYMYWVRQAPGQGLEWMGGINPSNGGTNFNEKFKNRVTLTTDS STTTAYMELKSLQFDDTAVYYCARRDYRFDMGFDYWGQGTTVTVSSGGGGS GGGGSGGGGSEIVLTQSPATLSLSPGERATLSCRASKGVSTSGYSYLHWYQQK PGQAPRLLIYLASYLESGVPARFSGSGSGTDFTLTISSLEPEDFAVYYCQHSRDL PLTFGGGTKVEIK |
| 105 | Soluble TGFβ Trap-hFc | IPPHVQKSVNNDMIVTDNNGAVKFPQLCKFCDVRFSTCDNQKSCMSNCSITSIC EKPQEVCVAVWRKNDENITLETVCHDPKLPYHDFILEDAASPKCIMKEKKKPG ETFFMCSCSSDECNDNIIFSEEYNTSNPDGGGGGSGGGGSGGGGSESKYGPPCP PCPAPEFLGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSIE KTISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLGK |
| 106 | hFc-soluble TGFβ Trap | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGGSGGGGSGGGGSIPPHVQKSVNNDMIVTDNNGA VKFPQLCKFCDVRFSTCDNQKSCMSNCSITSICEKPQEVCVAVWRKNDENITLE TVCHDPKLPYHDFILEDAASPKCIMKEKKKPGETFFMCSCSSDECNDNIIFSEEY NTSNPD |
| 107 | hFc-FEBM | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEA LHNHYTQKSLSLSLGKGGGGSGGGGSSSSPIQGSWTWENGKWTWKGIIRLEQ |
| 108 | Anti-EDB-FN-HC-IGHG | EVQLLESGGGLVQPGGSLRLSCAASGFTFSSFSMSWVRQAPGKGLEWVSSISGS SGTTYYADSVKGRFTISRDNSKNTLYLQMNSLRAEDTAVYYCAKPFPYFDYW GQGTLVTVSSASTKGPSVFPLAPCSRSTSESTAALGCLVKDYFPEPVTVSWNSG ALTSGVHTFPAVLQSSGLYSLSSVVTVPSSSLGTKTYTCNVDHKPSNTKVDKR VESKYGPPCPPCPAPEFLGGPSVFLFPPKPKDTLMISRTPEVTCVVVDVSQEDPE VQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSVMHE ALHNHYTQKSLSLSLGK |
| 109 | Anti-PD-1-HC-IGHG | QVQLVQSGVEVKKPGASVKVSCKASGYTFTNYYMYWVRQAPGQGLEWMGG INPSNGGTNFNEKFKNRVTLTTDSSTTTAYMELKSLQFDDTAVYYCARRDYRF DMGFDYWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKP SNTKVDKRVESKYGPPCPPCPAPEFLGGPSVFLFPPKPKDTLMISRTPEVTCVVV DVSQEDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNV FSCSVMHEALHNHYTQKSLSLSLGK |

### 1-4. Production and purification of fusion proteins

### Transient CHO expression

The signal sequence MGWSCIILFLVATATGAYA (SEQ ID NO: 112) was included at the N-terminus of the fusion protein of Example 1-2. The fusion protein was cloned into the KpnI (5' end) and NotI (3' end) sites of the pCAG mammalian expression plasmid vector (FUJIFILM Wako, Japan), and then produced by transient transfection of Chinese hamster ovary (CHO) cells.

That is, the ExpiCHO^{™} expression system kit (Thermo fisher Scientific, USA) was used. Plasmid DNA was transfected into 400 mL to 1,200 mL of ExpiCHO-S cells in serum-free ExpiCHO^{™} expression medium (Thermo fisher Scientific, USA) containing GlutaMax (Thermo fisher Scientific, USA) and maintained at 37°C.

Transfection conditions were performed according to the user guide of the ExpiCHO^{™} expression system. DNA 1ug/mL and ExpiFectamine^{™} CHO Reagent were diluted in OptiPRO SFM (Thermo fisher Scientific, USA) for 1L of culture flask and treated. 18 to 22 hours after transfection, ExpiCHO^{™} Feed and ExpiCHO^{™} Enhancer were added, and the cells were harvested after maintaining them at 37°C for 7-11 days after transfection. The cell viability at the final harvest was 70% - 80%.

### Purification

The supernatant harvested from CHO cells was filtered through a 0.22 µm PES filter (Corning, NY, USA) and loaded onto a column XK16 (Cytiva, USA) packed with Protein A MebSelect Sure LX (Cytiva, USA) resin. The solution was washed with 5 column volumes each of Wash 1 buffer and Wash 2 buffer, and the protein was eluted using 5 column volumes of 0.02 M sodium pH 4.0 and pH 3.8 buffers.

The purified fraction samples of the eluted protein were subjected to size exclusion chromatography (SEC) analysis and sodium dodecyl sulfate polyacrylamide gel electrophoresis (SDS-PAGE) analysis under non-reducing conditions to select samples with high purity. In addition, the purified fraction samples with purity of 90% or less were re-loaded onto the protein A column for the purpose of reprocessing and eluted as described above to maximize the production yield. In addition, the eluted purified protein was analyzed by the same method.

The purified fraction samples containing the fusion protein were pooled, and the eluted fractions were neutralized to approximately pH 7.0 using 1 M Tris. For SEC analysis, and 20 to 40 µg of protein was diluted in PBS (Enzynomics, Korea) and injected onto a TSKgel G3000SWxL (Tosoh, Japan) SEC analytical column (5.0 µm, 7.8 X 300 mm) equipped with a TSKgel guard column (7.0 µm, 6.0 X 40 mm) using an Agilent (Agilent infinity II) system. Resolution was performed at a flow rate of 0.8 mL/min at room temperature for 30 minutes, and the target protein peak was detected at UV 280 nm/214 nm using an Agilent VWD detector (Agilent, USA). SDS-PAGE analysis was performed under non-reducing conditions with EzStainAQua (ATTO, Japan) staining, followed by destaining using triple-distilled water.

### 1-5. Evaluation of target binding level of fusion proteins

The level of simultaneous binding of each fusion protein to its target was assessed using a sandwich ELISA method.

That is, 96-well ELISA plates (Nunc, USA) coated with recombinant EDB-FN-6x His (Abcam) were blocked with PBS solution containing 1% BSA (Abcam, USA) for 1 hour at room temperature, and then, various serially diluted (1:2) target proteins such as FEBM-, L19 hIgG-, L19 scfv-fusion TGFβ-Trap or soluble Trap control were reacted for 2 hours at room temperature, and washed X times with washing buffer, and then, biotinylated TGF-β1 (R&D Systems, Catalog #NFTG0, USA) (1:50 diluted in PBS) was reacted for 2 hours at room temperature, and then, 100 µL of ELISA substrate (streptavidin conjugated with horseradish peroxidase) was added to each well after 4 times of dilution, and then, 2N H₂SO₄ was added to stop the HRP reaction. Then, the level of binding was assessed by measuring the optical density (OD) at 450 nm.

### 1-6. Creation of breast cancer orthotopic transplantation mouse model and confirmation of fusion protein effects

A breast cancer orthotopic transplantation mouse model was created by injecting 5 × 10⁴ 4T-1-LMT-2-Luc cell lines produced in Example 1-2 into BALB/c mice (Orient Bio, Korea).

### Confirmation of in vivo fusion protein distribution

After intravenous injection of 20 mg/kg of Cy5-labeled various fusion proteins (e.g., hFc-soluble TGFβ Trap, FEBM-hFc-TGFβ Trap, hFc-FEBM-TGFβ Trap, and hFc-TGFβ Trap-FEBM, etc.) into orthotopic transplantation mouse models, mice were sacrificed 6 days later, and tissues were collected and analyzed for Cy5 fluorescence signals using IVIS imaging-Lumina S5 (PerkinElmer, USA).

### Tumor size analysis

Orthotopic transplantation mouse models were injected subcutaneously with 20 mg/kg of various fusion proteins (e.g., hFc-soluble TGFβ Trap, FEBM-hFc-TGFβ Trap, hFc-FEBM-TGFβ Trap, hFc-TGFβ Trap-FEBM, etc.) and controls (e.g., control-hFc) at 4-day intervals from Days 5 to 25. Tumor sizes were measured using electronic calipers on Days 5, 9, 13, 17, 21 and 25.

### Transition rate analysis

On day 7 of the growth of the orthotopic transplantation mouse model, D-luciferin was injected intraperitoneally to use the BLI (bioluminescence) signal as a background. The growth of primary fat pad tumors was monitored by BLI signal and separated into 5 groups with similar BLI signals. Various fusion proteins (e.g., hFc-soluble TGFβ Trap, FEBM-hFc-TGFβ Trap, hFc-FEBM-TGFβ Trap, hFc-TGFβ Trap-FEBM, etc.) and control groups (e.g., control-hFc) at 20 mg/kg were injected subcutaneously at 4-day intervals from day 5 to 25.

The metastasis rate was analyzed by BLI signals on Days 5, 11, 18, 21 and 25 using IVIS imaging-Lumina S5 (PerkinElmer, USA). The dead mice were dissected within 6 hours and analyzed for the presence of metastases.

### Survival rate analysis

In the same way as the metastasis analysis, various fusion proteins were prepared in the mouse model, and the number of mice that survived up to 50 days was counted to create a survival curve.

### Anti-pSMAD and anti-CD8 immunohistochemical analysis

The orthotopic transplantation mouse model was administered with 20 mg/kg of various fusion proteins (e.g., hFc-soluble TGFβ Trap, FEBM-hFc-TGFβ Trap, hFc-FEBM-TGFβ Trap, hFc-TGFβ Trap-FEBM, etc.) and control groups (e.g., control-hFc) at 4-day intervals from day 5 to day 25, and then sacrificed 24 hours later. The 4T-1-L2-Luc tumor tissues were fixed in formalin and embedded in paraffin to create 5 µm tumor sections, which were then mounted on SuperFrost Plus slides (Thermo fisher Scientific, USA) and stained according to the user guide of the Leica Bond autostainer.

That is, the slides were baked, dewaxed and rehydrated, and then exposed to antigens for 20 minutes using ER2 at 95°C, blocked with 2.5% normal goat serum, and then reacted with primary SMAD2 phosphorylation antibody (Ser465/467, clone 138D4, NEB, 0.6 µg/mL, USA) and primary mCD8a antibody (clone 4SM15, eBioscience, 2.5 µg/mL, USA) for 60 minutes. Antigens were detected using secondary anti-rabbit and anti-rat secondary antibodies conjugated with HRP (Vector Labs, MP-7444, USA), and visualized using DAB substrate (Abcam, AB64238, USA). Signals were quantified using Definiens Tissue Studio software, and the total number of cells was calculated by counting the number of hematoxylin-stained nuclei. Positive signals were detected by setting the threshold for DAB chromogen above the background signal.

### 1-7. Creation of pancreatic cancer orthotopic transplantation mouse model and confirmation of fusion protein effects

A pancreatic cancer orthotopic transplantation mouse model was created by injecting 2 × 10⁶ PANC02-Luc cell lines produced in Example 1-2 into C57BL/6J mice (Orient Bio, Korea).

### Tumor weight analysis

The orthotopic transplantation mouse model was subcutaneously injected with 30 mg/kg of various fusion proteins (e.g., hFc-soluble TGFβ Trap, FEBM-hFc-TGFβ Trap, hFc-FEBM-TGFβ Trap, hFc-TGFβ Trap-FEBM, etc.) and controls (e.g., control-hFc) at 5-day intervals from day 7 to day 57. Tumor weights were measured on day 63 after all mice were sacrificed and tumors were collected.

### Transition rate analysis

The orthotopic transplantation mouse model was grown for 7 days, and then, D-luciferin was injected intraperitoneally to use the bioluminescence (BLI) signal as a background. The growth of primary fat pad tumors was monitored by BLI signal and separated into 7 groups with similar BLI signals. Various fusion proteins (e.g., hFc-soluble TGFβ Trap, FEBM-hFc-TGFβ Trap, hFc-FEBM-TGFβ Trap, hFc-TGFβ Trap-FEBM, etc.) and controls (e.g., control-hFc) at 30 mg/kg were injected subcutaneously at 5-day intervals from day 7 to day 57.

The cancer metastasis rate was analyzed by BLI signals on Days 7 and 62 using IVIS imaging -Lumina S5 (PerkinElmer, USA). The dead mice were dissected within 6 hours and analyzed for the presence of metastases. All mice were sacrificed on day 63.

### Survival rate analysis

In the same way as the metastasis analysis, various fusion proteins were prepared in the mouse model, and the number of mice that survived up to 63 days was counted to create a survival curve.

### Anti-pSMAD and anti-CD8 immunohistochemical analysis

Panc02-Luc tumor tissues were fixed in formalin and embedded in paraffin to create 5 µm tumor sections, which were analyzed using the same method as the analysis of the breast cancer orthotopic transplantation mouse model.

### 1-8. Creation of mouse model of tumor growth after melanoma vein transplantation -> melanoma lung metastasis and confirmation of the effects of fusion proteins

A melanoma transplantation mouse model was created by intravenously injecting 1 × 10⁵ B16-F10-Luc cell line produced in Example 1-2 into C57BL/6 male mice (Orient Bio, Korea).

### Growth rate analysis

Intravenous transplantation mouse models were injected intravenously with 30 mg/kg of various fusion proteins (e.g., hFc-soluble TGFβ Trap, hFc-FEBM-TGFβ Trap, hFc-TGFβ Trap-FEBM, hFc-FEBM-anti-TGFB1 scFv, Anti-EDB-FN-TGFβ Trap-hFc, or hFc-anti-EDB-FN scFv-anti-TGFβ1 scFv) and controls (e.g., control-hFc) at 3-day intervals from day 5 to day 25. On day 5, D-luciferin was injected intraperitoneally to use the bioluminescence (BLI) signal as a background. The growth of primary fat pad tumors was monitored by BLI signal and separated into 5 groups with similar BLI signals. The growth rate of B16-F10-Luc-tumor in the lung was analyzed by BLI signals on Days 5, 11, 18, 21 and 28 using IVIS imaging-Lumina S5 (PerkinElmer, USA). The average radiant efficiency value is the value normalized to the overall value by each ROI size.

### Example 2. Confirmation of cancer association of fibronectin gene FN1 and EDB domain

As a result of analyzing the expression of fibronectin EDB transcripts by the method of Example 1-1, it was confirmed that 4 transcripts among the fibronectin transcripts in the human genome region include EDB exons that are specifically expressed in cancer tissues, as described in FIG. 1A. As a result of analyzing the expressions of EDB exons and the entire FN1 gene by the expression analysis method of Example 1-1, it was confirmed that both EDB exons and FN1 gene showed higher expression in tumor tissues than in normal tissues, as described in FIG. 1B.

In addition, when the expression of the FN1 EDB+ gene in various cancers was analyzed in TCGA data using the prognostic analysis method of Example 1-1, it was confirmed that in most cancers, the higher the expression of the FN1 EDB+ gene, the worse the prognosis, as described in FIGS. 2A to 2F. In addition, when the FN1 EDB domain was analyzed in humans, monkeys, rats and mice using the sequence alignment method of Example 1-1, it was confirmed that it was 100% conserved in the four species, as described in FIG. 3.

Moreover, as a result of comparing the expression levels of FN1-EDB+ transcripts in 11 different tumor tissues and normal tissues that were orthotopically transplanted by the method of Example 1-2, as described in FIG. 4, it was confirmed that FN1 EDB+ expression was significantly higher in all solid tumor tissues than in three normal tissues, including 4T-1 (breast cancer), Colon26 (colon cancer), PANC02 (pancreatic cancer), KRasG12D/+TP53-/-PLC (Primary lung cancer cell lines derived from spontaneous Kras/p53 GEMMs, lung cancer), B16F10 (melanoma), Hepa6 (liver cancer), GL26 (brain tumor), RM1 (prostate cancer), YTN16 (gastric cancer), RENCA (renal cancer) and KRasG12D/+TP53-/- PSC (Primary sarcoma cell lines derived from spontaneous Kras/p53 GEMMs, sarcoma). TP53-/- PTLC (Primary T lymphoma cell lines derived from spontaneous p53 GEMMs, lymphoma) hematological cancers were confirmed to express similarly to normal tissues, and among the transplanted solid tumors, pancreatic cancer tumors showed the highest FN1 EDB+ expression, while the lowest expression was confirmed in transplanted melanoma tumors.

Subsequently, tumor models of FN1-EDB high (pancreatic cancer model), FN1-EDB intermediate (breast cancer model), and FN1-EDB low (melanoma model) levels were used to evaluate efficacy.

### Example 3. Production of bispecific Fc fusion proteins including FEBM and TGFβ Trap and confirmation of binding capability

### 3-1. Production and purification of bispecific Fc fusion proteins including FEBM and TGFβ Trap

The bispecific Fc fusion protein including FEBM and TGFβ Trap designed by the method of Example 1-3 was purified by the method of Example 1-4, and as a result, as described in a and b of FIG. 5, it was confirmed that the monomer purity was 90% or more, and the endotoxin level was 0.1 EU/mL or less. In this example, among the bispecific Fc fusion proteins including FEBM and TGFβ Trap of Table 2,
Control-hFc (SEQ ID NO: 12): Human IgG4 heavy chain Fc region (hIgG4-Fc)
hFc-FEBM (SEQ ID NO: 107): FEBM polypeptide is fused to the C-terminus of hIgG4-Fc (hIgG4-Fc-FEBM)
Soluble TGFβ Trap-hFc (SEQ ID NO: 105): TGFβRII ectodomain domain is fused to the N-terminus of hIgG4-Fc.
TGFβ Trap-hFc-FEBM (SEQ ID NO: 16): TGFβRII ectodomain domain is fused to the N-terminus of hIgG4-Fc, and FEBM polypeptide is fused to the C-terminus of Fc.
TGFβ Trap-FEBM-hFc (SEQ ID NO: 17): FEBM polypeptide is fused to the N-terminus of hIgG4-Fc, onto which the TGFβRII ectodomain domain is fused.
FEBM-TGFβ Trap-hFc (SEQ ID NO: 18): TGFβRII ectodomain domain is fused to the N-terminus of hIgG4-Fc, onto which FEBM polypeptide is fused.
hFc-soluble TGFβ Trap (SEQ ID NO: 106): TGFβRII ectodomain domain is fused to the C-terminus of hIgG4-Fc.
FEBM-hFc-TGFβ Trap (SEQ ID NO: 19): FEBM polypeptide is fused to the N-terminus of hIgG4-Fc, and TGFβRII ectodomain domain is fused to the C-terminus.
hFc-FEBM-TGFβ Trap (SEQ ID NO: 20): FEBM polypeptide is fused to the C-terminus of hIgG4-Fc, followed by TGFβRII ectodomain domain.
hFc-TGFβ Trap-FEBM (SEQ ID NO: 21): TGFβRII ectodomain domain fused to the C-terminus of hIgG4-Fc, followed by FEBM polypeptide fusion.

Proteins such as these were produced and purified.

### 3-2. Evaluation of binding capability of dual-specific Fc fusion proteins including FEBM and TGFβ Trap

As a result of evaluating the binding capability of the fusion proteins purified in Example 3-1 by the method of Example 1-5, it was confirmed that consistent binding affinity was formed in the range of 304.3 to 370 ng/mL for both targets despite structural changes at different positions of the FEBM-TGFβ Trap structure, as described in FIG. 6.

### 3-3. Confirmation of binding capability of bispecific Fc fusion proteins including FEBM and TGFβ Trap in breast cancer orthotopic transplantation model

4T1 breast cancer tumors are a commonly used mouse model to study triplenegative breast cancer (TNBC) due to their aggressive growth, metastatic potential, and ability to simulate immune responses seen in human cancers. These tumors often do not respond to anti-PD-1 or anti-PD-L1 therapy, either as monotherapy or in combination with other treatments, reflecting the immunosuppressive nature of the tumor microenvironment (TME) in this model. Highly immunosuppressive tumor microenvironment: One of the characteristics of the 4T1 tumor model is the lack of infiltration of CD8+ cytotoxic T cells, which are essential for an effective anti-tumor immune response. This lack of immune cell infiltration is due in part to the dense extracellular matrix (ECM) and fibrous stroma surrounding the tumor, which constitute a physical barrier.

In previous studies, 4T1 breast cancer tumors showed activated TGFβ signaling, and in recent studies, intermediate to high expression of EDB-FN was observed in solid tumors. Based on these results, the inventors of the present invention decided that it would be appropriate to evaluate a drug that dually targets FN1 EDB+, which binds to tumor ECM and enables local TGFβ trapping.

As such, the *in vivo* distribution of tumors and organs collected from 4T-1 orthotopic transplantation mice produced by the method of Example 1-6 was confirmed, and as described in FIG. 7, relatively high fluorescence signal intensity was observed in the liver and kidneys because the probe was mainly excreted through these organs, and despite the appearance of a high fluorescence background in the liver tissues of mice not administered Cy5, it was confirmed that the tumors treated with hFc-FEBM-TGFβ Trap or hFc-TGFβ Trap-FEBM dual fusion protein showed the highest fluorescence intensity compared to hFc-soluble TGFβ Trap and FEBM-hFc-TGFβ Trap based on the radiation efficiency excluding autofluorescence.

In addition, both hFc-FEBM-TGFβ Trap and hFc-TGFβ Trap-FEBM proteins showed significantly higher fluorescence intensities in tumors than in 9 types of normal tissues and organs, and hFc-FEBM-TGFβ Trap showed slightly higher tumor specificity than hFc-TGFβ Trap-FEBM protein, hFc-soluble TGFβ Trap did not show tumor specificity, and FEBM-hFc-TGFβ Trap showed some tumor specificity compared to all normal tissues except the liver, but the difference was not significant, and hFc-FEBM-TGFβ Trap and hFc-TGFβ Trap-FEBM proteins were confirmed to bind with high specificity to EDB-FN, which is abundantly expressed in 4T-1 orthotopic transplant tumors.

### Example 4. Confirmation of therapeutic effects of bispecific Fc fusion proteins including FEBM and TGFβ Trap

### 4-1. Tumor growth rate analysis

An advance breast tumor model that is orthotopically transplanted in the breast pad is clinically more relevant than subcutaneous tumor models due to the establishment of organ-specific tumors. The method of Examples 1-6 was used to establish highly metastatic cell lines, and as a result, hFc-FEBM-TGFβ Trap and hFc-TGFβ Trap-FEBM exhibited statistically significant potent antitumor activity compared to other experimental groups in an orthotopic transplantation mouse advanced breast cancer model in which intermediate levels of EDB expression were observed among all solid tumors, as described in FIG. 8A.

In addition, as described in FIG. 8B, the tumor volumes of the hFc-soluble TGFβ Trap, FEBM-hFc-TGFβ Trap, hFc-FEBM-TGFβ Trap, or hFc-TGFβ Trap-FEBM administration groups on day 25 were confirmed to decrease by about 32.5%, about 42.4%, about 92.8% and about 74.9%, respectively, compared to the tumor volume of the isotype control group. That is, it was confirmed that the administration of hFc-FEBM-TGFβ Trap or hFc-TGFβ Trap-FEBM inhibited tumor growth to a greater extent (>90% and >50%, respectively) than hFc-soluble TGFβ Trap.

### 4-2. Analysis of lung metastasis rates

As a result of analyzing the lung metastasis rates of the breast cancer orthotopic transplantation model by the administration of hFc-soluble TGFβ Trap, FEBM-hFc-TGFβ Trap, hFc-FEBM-TGFβ Trap and hFc-TGFβ Trap-FEBM fusion proteins using the method of Example 1-6, as described in FIG. 9, on day 33, the lung metastasis characteristics of the hFc-soluble TGFβ Trap, FEBM-hFc-TGFβ Trap, hFc-FEBM-TGFβ Trap, or hFc-TGFβ Trap-FEBM administration groups were confirmed to have decreased by an average of 0%, 0%, 100%, and 100%, respectively, compared to the syngeneic control group.

In addition, when hFc-FEBM-TGFβ Trap was administered, the primary tumor completely responded, whereas hFc-TGFβ Trap-FEBM responded in 2 out of 5 mice, and hFc-FEBM-TGFβ Trap showed a 0% lung metastasis rate. On day 43, the hFc-soluble TGFβ Trap and FEBM-hFc-TGFβ Trap administration groups showed a 100% lung metastasis rate, and hFc-TGFβ Trap-FEBM showed a 40% lung metastasis rate.

### 4-3. Survival rate analysis

As a result of analyzing the survival rate of breast cancer orthotopic transplantation models administered with hFc-soluble TGFβ Trap, FEBM-hFc-TGFβ Trap, hFc-FEBM-TGFβ Trap and hFc-TGFβ Trap-FEBM fusion proteins using the method of Example 1-6, as described in FIG. 10, it was confirmed that hFc-TGFβ Trap-FEBM showed a statistically significant 40% increase in survival rate based on 50 days compared to the isotype control group, and more surprisingly, it was confirmed that all mice survived for 50 days even after drug administration was discontinued when hFc-FEBM-TGFβ Trap was administered.

### 4-4. Analysis of α-SMA expression in tumor margin and tumor center

Alpha-smooth muscle actin (α-SMA) is a key marker of myofibroblasts and cancer-associated fibroblasts (CAFs), which play a critical role in shaping the extracellular matrix (ECM) of the tumor microenvironment (TME). The expression thereof tends to differ between the tumor margin and center, and α-SMA-positive CAFs form a dense stroma that promotes the accumulation of ECM proteins, contributing to the creation of an immunosuppressive environment. This stroma acts as a physical barrier to restrict the infiltration of immune cells into the tumor core, and high α-SMA expression is correlated with immune cell infiltration and low immune resistance.

The 4T-1 tumor margin was defined as an ROI consisting of a tumor epithelium island that includes an area extending 200 to 300 µm on both sides of boundary or multiple areas of tumor stroma larger than 50 µm, and the tumor core was defined as the entire area within the tumor margin.

As a result of quantifying alpha SMA staining in the tumor margin and tumor center of 4T-1 using the method of Example 1-6, as described in the left panel of FIG. 11, it was confirmed that α-SMA expression in the tumor margin of the hFc-soluble TGFβ Trap, FEBM-hFc-TGFβ Trap, hFc-FEBM-TGFβ Trap, or hFc-TGFβ Trap-FEBM administration groups was reduced by about 21.0%, about 38.1%, about 83.7%, and about 61.7%, respectively, compared to the isotype control group. In particular, the groups administered with hFc-FEBM-TGFβ Trap and hFc-TGFβ Trap-FEBM suppressed α-SMA expression in the tumor margin to a greater extent (>80% and >50%, respectively) than hFc-soluble TGFβ Trap.

In addition, as described in the right panel of FIG. 11, it was confirmed that α-SMA expression in the tumor center of the hFc-soluble TGFβ Trap, FEBM-hFc-TGFβ Trap, hFc-FEBM-TGFβ Trap, or hFc-TGFβ Trap-FEBM administration groups increased by about 2.5-fold, about 4.4-fold, about 20-fold, and about 9.7-fold, respectively, compared to the isotype control group. In particular, the groups administered with hFc-FEBM-TGFβ Trap and hFc-TGFβ Trap-FEBM showed a greater increase (more than 8-fold and 4-fold, respectively) in α-SMA expression in the tumor center than that of hFc-soluble TGFβ Trap.

### 4-5. Confirmation of decreased phosphorylation of Smad2 and increased cytotoxic immune CD8+ T cells

As a result of confirming the phosphorylation of Smad2 and the expression level of CD8 by the method of Example 1-6, as described in FIG. 12A, it was confirmed that the phosphorylation of Smad2 in the tumors of the hFc-soluble TGFβ Trap, FEBM-hFc-TGFβ Trap, hFc-FEBM-TGFβ Trap, or hFc-TGFβ Trap-FEBM administration groups decreased by about 23.8%, about 39.2%, about 67.2%, and about 57.8%, respectively, compared to the isotype control group (control-hFc).

In particular, the groups administered with FEBM-hFc-TGFβ Trap, hFc-FEBM-TGFβ Trap and hFc-TGFβ Trap-FEBM suppressed p-smad2 levels to a greater extent (>21%, >57%, and >43%) than hFc-soluble TGFβ Trap, respectively. Although hFc-soluble TGFβ Trap, characterized by systemic exposure, did not sustain Smad2 phosphorylation in tumor tissues of mice sacrificed 2 days after the last administration, the FEBM fusion protein suppressed Smad2 phosphorylation more effectively through TGFβ Trap due to tumor specificity and higher persistence in the tumor microenvironment.

In addition, as described in FIG. 12B, the expression of CD8, which is a memory marker effective in killing cancer cells in the hFc-soluble TGFβ Trap, FEBM-hFc-TGFβ Trap, hFc-FEBM-TGFβ Trap or hFc-TGFβ Trap-FEBM administration groups, was confirmed to increase about 2.6-fold, about 4.2-fold, about 18.7-fold, and about 10.1-fold, respectively, compared to the isotype control group (Control-hFc). In particular, the tumor-specific CD8+ T cells in the groups administered with hFc-FEBM-TGFβ Trap and hFc-TGFβ Trap-FEBM were confirmed to increase about 8-fold and about 4-fold, respectively, compared to hFc-soluble TGFβ Trap.

CD8 activation by the administration of Fc-FEBM-TGFβ Trap or hFc-TGFβ Trap-FEBM could be achieved directly or indirectly by 1) a potent inhibitory effect of CD8+ immune cell deactivation due to stromal-immune evasion-related cell interaction in the TME or 2) an effect of inducing stromal cell imbalance/reprogramming due to TME-specific targeted TGFβ inhibition, leading to CD8+ immune cell infiltration into the immune-decentralized TME. Tumors administered with hFc-FEBM-TGFβ Trap and hFc-TGFβ Trap-FEBM showed reduced p-Smad2 levels and increased CD8+ T cell abundance in the tumor center and tumor border, regardless of tumor epithelium and stroma.

### Example 5. Production of bispecific Fc fusion proteins including anti-EDB-FN antibody and TGFβ Trap and confirmation of binding capability

### 5-1. Production and purification of bispecific Fc fusion proteins including anti-EDB-FN antibody and TGFβ Trap

The bispecific Fc fusion protein including Anti-EDB-FN antibody and TGFβ Trap designed by the method of Example 1-3 was purified by the method of Example 1-4, and as a result, as described in a and b of FIG. 13, it was confirmed that the monomer purity was 90% or more and the endotoxin level was 0.1 EU/mL or less. In this example, among the bispecific Fc fusion proteins including Anti-EDB-FN antibody and TGFβ Trap of Table 2,
Control-hIgG: RecombiMAb human IgG4 (S228P) isotype control, anti-hen egg lysozyme Cat: CP147, Bio X cell, USA)
Anti-EDB-FN-hIgG (SEQ ID NO: 108): Anti-EDB-FN antibody (L19) hIgG4 form
Soluble TGFβ Trap-hFc (SEQ ID NO: 105): TGFβRII ectodomain domain is fused to the N-terminus of hIgG4-Fc.
Anti-EDB-FN-hIgG-TGFβ Trap (L19-hIgG-TGFβ Trap, SEQ ID NO: 29): TGFβRII ectodomain is fused to the C terminus of L19 hIgG4-Fc.
Anti-EDB-FN-hIgG-LC-TGFβ Trap (L19-hIgG-LC-TGFβ Trap, SEQ ID NO: 30): The TGFβRII ectodomain is fused to the C-terminus of L19 hIgG4-light chain (LC).
Anti-EDB-FN scFv-hFc-TGFβ Trap (L19 scFv-hFc-TGFβ Trap, SEQ ID NO: 32): The L19 scFv antibody fragment is fused to the N-terminus of the heavy chain Fc region, and the TGFβRII ectodomain is fused to the C-terminus of the heavy chain Fc region.
Anti-EDB-FN scFv-TGFβ Trap-hFc (L19 scFv-TGFβ Trap-hFc, SEQ ID NO: 34): The L19 scFv antibody fragment and the TGFβRII ectodomain are fused at the N-terminus of IgG4-Fc.
hFc-Anti-EDB-FN-TGFβ Trap (SEQ ID NO: 33): The TGFβRII ectodomain is fused to the C terminus of the L19 scFv antibody fragment and linked beneath the heavy chain Fc region.
hFc-TGFβ Trap-Anti-EDB-FN (SEQ ID NO: 35): L19 scFv antibody fragment is fused to the C-terminus of the TGFβRII ectodomain and Trap below the heavy chain Fc region.
Anti-EDB-FN scFv-TGFβ Trap (SEQ ID NO: 36): L19 scFv antibody fragment is fused onto TGFβ Trap without FC fusion

Proteins such as these were produced and purified.

### 5-2. Evaluation of binding capability of dual-specific Fc fusion proteins including anti-EDB-FN antibody and TGFβ Trap

The binding capability of the fusion proteins purified in Example 5-1 was evaluated by the method of Example 1-5, and as described in FIG. 14, it was confirmed that consistent co-binding affinity was shown for both targets at a binding level in the range of 10 to 86 ng/mL despite structural changes at different positions of L19 antibody or L19 scfv and TGFβ Trap. Compared to the Fc fusion form, Anti-EDB-FN scFv-TGFβ Trap without Fc fusion was confirmed to have a lower simultaneous binding affinity for both targets.

### Example 6. Confirmation of therapeutic effects of bispecific Fc fusion proteins including anti-EDB-FN antibody and TGFβ Trap

### 6-1. Tumor growth rate analysis

An advanced breast tumor model that is transplanted orthotopically in the breast pad is clinically more relevant than a subcutaneous tumor model due to the establishment of organ-specific tumors. The method of Examples 1-6 was used to establish highly metastatic cell lines, and as a result, as described in FIG. 15A, anti-EDB-FN scfv-TGFβ Trap-hFc and hFc-anti-EDB-FN scFv-TGFβ Trap exhibited statistically significant potent antitumor activity compared to other experimental groups in an orthotopic transplantation mouse advanced breast cancer model.

In addition, as described in FIG. 15B, the tumor volumes of the anti-EDB-FN-hIgG, anti-EDB-FN-hIgG-TGFβ Trap, anti-EDB-FN scfv-TGFβ Trap-hFc, or hFc-anti-EDB-FN scFv-TGFβ Trap administration groups on day 25 were confirmed to have decreased by about 1%, about 6%, about 57%, and about 51%, respectively, compared to the tumor volume of the isotype control group (control hIgG). In addition, it was confirmed that only anti-EDB-FN scfv-TGFβ Trap-hFc and hFc-anti-EDB-FN scFv-TGFβ Trap administration inhibited tumor growth by more than 50% compared to anti-EDB-FN-hIgG-TGFβ Trap, which was designed with a standard conceptual structure.

### 6-2. Analysis of lung metastasis rates

As a result of analyzing the lung metastasis rates of breast cancer orthotopic transplantation models administered with a dual heavy-chain fusion protein consisting of L19 antibody-TGFβ Trap or L19 scfv-TGFβ Trap based on the binding between L19 antibody or L19 scfv of EDB FN by the method of Example 1-6, as described in FIG. 16, on day 35, the lung metastasis rates of the anti-EDB-FN-hIgG, anti-EDB-FN-hIgG-TGFβ Trap, anti-EDB-FN scfv-TGFβ Trap-hFc, or hFc-anti-EDB-FN scFv-TGFβ Trap administration groups were confirmed to have decreased by an average of 0%, 0%, 80%, and 60%, respectively, compared to the isotype control group (control hIgG).

### 6-3. Survival rate analysis

The survival rate of breast cancer orthotopic transplantation models administered with dual heavy chain fusion proteins consisting of L19 antibody-TGFβ Trap or L19 scfv-TGFβ Trap based on the binding between L19 antibody or L19 scfv of EDB FN by the method of Example 1-6 was analyzed. As a result, as described in FIG. 17, compared to the isotype control group and anti-EDB-FN-hIgG, anti-EDB-FN scFv-hFc-TGFβ Trap designed as a standard bispecific molecule did not improve the survival rate at all up to 35 days, whereas the groups administered with anti-EDB-FN hIgG-TGFβ Trap-hFc and hFc-anti-EDB-FN scFv-TGFβ Trap showed statistically significant increases of 60% and 60%, respectively.

The commonality between the therapeutic effects of the two substances, hFc-FEBM-TGFβ Trap and hFc-TGFβ Trap-FEBM, and anti-EDB-FN hIgG-TGFβ Trap-hFc and hFc-anti-EDB-FN scFv-TGFβ Trap, is determined to be that they have the structural characteristic of directly linking the EDB-FN target and the TGFβ trap, reflecting the three-dimensional structure targeting the tumor ECM and the activity of TGFβ trapping.

### Example 7. Confirmation of therapeutic effects of pancreatic cancer orthotopic transplantation models by administration of bispecific Fc fusion protein consisting of FEBM-, L19 antibody-TGFβ Trap or L19 scfv-TGFβ Trap

### 7-1. Tumor growth rate analysis of bispecific Fc fusion protein consisting of FEBM-TGFβ Trap

It is known that the pancreatic cancer model, which is advanced Panc02 immune desert tumors orthotopically transplanted into the tail of the pancreas, is more clinically relevant than the subcutaneous tumor model due to the establishment of organ-specific tumors. Accordingly, hFc-soluble TGFβ Trap, FEBM-hFc-TGFβ Trap, hFc-FEBM-TGFβ Trap, or hFc-TGFβ Trap-FEBM and isotype control Fc were administered to pancreatic cancer orthotopic transplantation mice produced by the method of Example 1-7, and the therapeutic effects were confirmed by the method described in Example 1-7.

As a result, as described in FIG. 18A, hFc-soluble TGFβ Trap showed no statistical significance in reducing tumor weight compared to the isotype control group, whereas hFc-FEBM-TGFβ Trap and hFc-TGFβ Trap-FEBM showed statistically significant potent antitumor activity compared to other experimental groups in the orthotopic mouse advanced pancreatic cancer model in which the highest level of EDB expression was observed among all solid tumors.

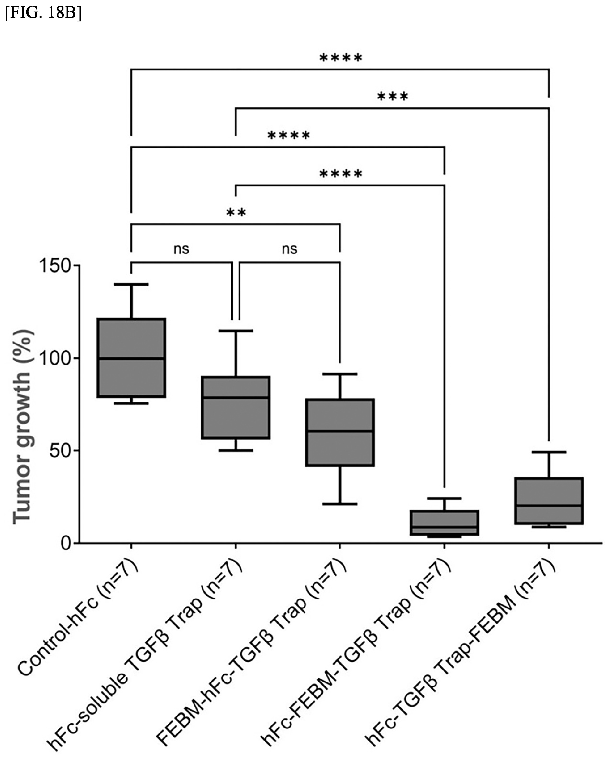

As described in FIG. 18B, on day 63 after cell transplantation, the tumor weights of the hFc-soluble TGFβ Trap, FEBM-hFc-TGFβ Trap, hFc-FEBM-TGFβ Trap, or hFc-TGFβ Trap-FEBM administration groups were confirmed to have decreased by about 22.1%, about 38.7%, about 90.1%, and about 76.4%, respectively, compared to the isotype control group (control hIgG). It was confirmed that the administration of hFc-FEBM-TGFβ Trap or hFc-TGFβ Trap-FEBM inhibited tumor growth to a greater extent (>85% and >70%, respectively) than hFc-soluble TGFβ Trap.

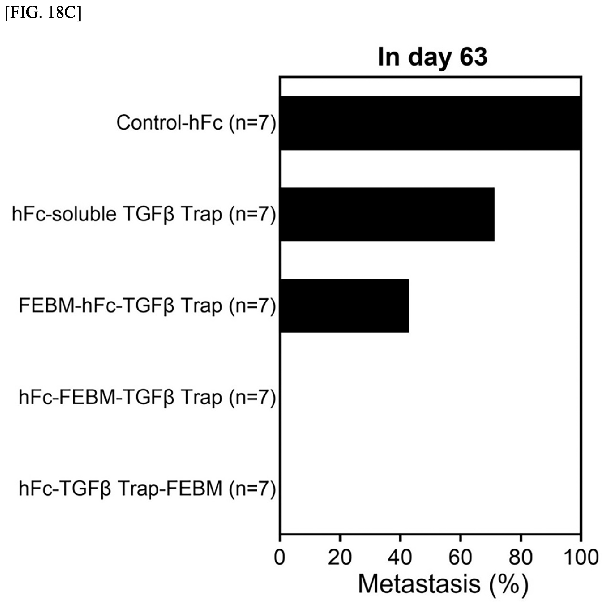

In addition, as described in FIG. 18C, on day 63, the metastasis rates including liver, pleura, and diaphragm in the hFc-soluble TGFβ Trap, FEBM-hFc-TGFβ Trap, hFc-FEBM-TGFβ Trap, or hFc-TGFβ Trap-FEBM administration groups were confirmed to have decreased by an average of 20%, 40%, 100%, and 100%, respectively, compared to the homologous control group.

### 7-2. Confirmation of decreased phosphorylation of Smad2 and increased cytotoxic immune CD8+ T cells by dual heavy-chain fusion protein consisting of FEBM-TGFβ Trap

As a result of confirming the phosphorylation of Smad2 and the expression level of CD8 by the method of Example 1-7, as described in FIG. 19A, it was confirmed that the phosphorylation of Smad2 in the tumors of the hFc-soluble TGFβ Trap, FEBM-hFc-TGFβ Trap, hFc-FEBM-TGFβ Trap, or hFc-TGFβ Trap-FEBM administration groups was reduced by about 17.2%, about 33%, about 84.5%, and about 72.8%, respectively, compared to the isotype control group. In particular, it was confirmed that the FEBM-hFc-TGFβ Trap, hFc-FEBM-TGFβ Trap and hFc-TGFβ Trap-FEBM administration groups suppressed the p-smad2 level to a greater extent (>80% and >65%, respectively) than the hFc-soluble TGFβ Trap. Although hFc-soluble TGFβ Trap, characterized by systemic exposure, failed to sustain Smad2 phosphorylation in tumor tissues of mice sacrificed 2 days after the last administration, the FEBM fusion structure was determined to be more effective in inhibiting Smad2 phosphorylation via TGFβ Trap due to its tumor specificity and higher persistence in the tumor microenvironment.

In addition, as described in FIG. 19B, the expression of CD8, which is a memory marker effective in killing cancer cells in the hFc-soluble TGFβ Trap, FEBM-hFc-TGFβ Trap, hFc-FEBM-TGFβ Trap, or hFc-TGFβ Trap-FEBM administration groups, was confirmed to increase about 3.4-fold, about 7.7-fold, about 25.4-fold, and about 16.1-fold, respectively, compared to the isotype control group (Control-Fc). In particular, the tumor-specific CD8+ T cells in the hFc-FEBM-TGFβ Trap and hFc-TGFβ Trap-FEBM administration groups were confirmed to increase about 7.4-fold and about 4.7-fold, respectively, compared to the hFc-soluble TGFβ Trap.

Tumors treated with hFc-FEBM-TGFβ Trap or hFc-TGFβ Trap-FEBM showed reduced p-Smad2 levels and an abundance of CD8+ T cells in the tumor center and tumor border, regardless of tumor epithelium and stroma.

### 7-3. Tumor growth rate analysis of bispecific Fc fusion proteins including anti-EDB-FN antibody and TGFβ Trap

30 mg/kg anti-EDB-FN-hIgG, anti-EDB-FN-hIgG-TGFβ Trap, or anti-EDB-FN scFv-TGFβ Trap-hFc and isotype control Fc were administered to pancreatic cancer orthotopic transplantation mice produced by the method of Example 1-7, and the therapeutic effect was confirmed by the method described in Example 1-7.

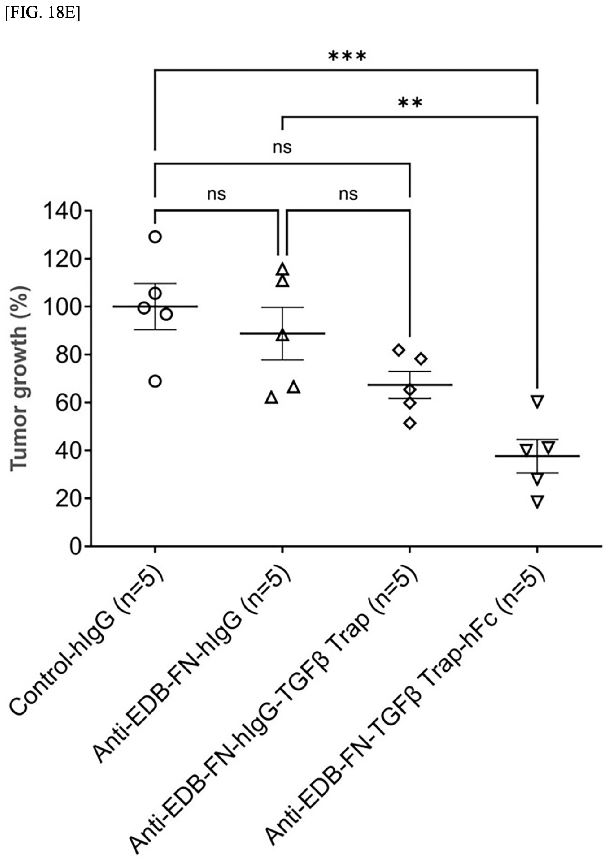

As a result, as described in FIG. 18B, the anti-EDB-FN scfv-TGFβ Trap-hFc administration group, which has L19 scfv in the form of an antibody platform in addition to FEBM, a polypeptide that binds to EDB-FN of the tumor ECM, was confirmed to exhibit statistically significant potent antitumor activity compared to other experimental groups in the orthotopic transplanted mouse advanced pancreatic cancer model, and as described in FIG. 18E, on day 63 after cell transplantation, the tumor weights of the anti-EDB-FN-hIgG, anti-EDB-FN-hIgG-TGFβ Trap, or anti-EDB-FN scfv-TGFβ Trap-hFc administration groups were confirmed to have decreased by about 9.2%, about 31.2%, and about 60.4%, respectively, compared to the isotype control group. Compared to anti-EDB-FN-hIgG-TGFβ Trap, which was designed with a standard conceptual structure, the administration of anti-EDB-FN scfv-TGFβ Trap-hFc alone inhibited tumor growth by more than 50%.

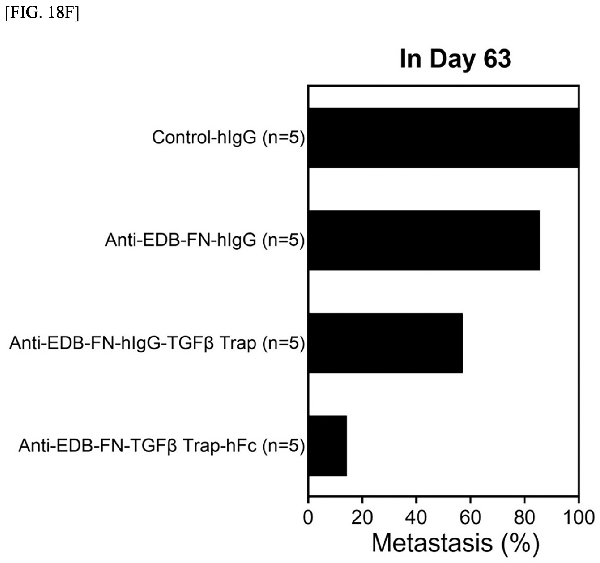

In addition, as described in FIG. 18F, on day 63, the metastasis rates including liver, pleura, and diaphragm in the anti-EDB-FN-hIgG, anti-EDB-FN-hIgG-TGFβ Trap, or anti-EDB-FN scfv-TGFβ Trap-hFc administration groups were confirmed to have decreased by an average of 20%, 40%, and 80%, respectively, compared to the homologous control group.

### Example 8. Therapeutic effect of administration of bispecific Fc fusion proteins consisting of FEBM-, L19 antibody-TGFβ Trap or L19 scfv-TGFβ Trap in tumor growth model after orthotopic transplantation or lung metastasis of melanoma

B16-F10 is a tumor model that is cytokine-deficient and MHC-I suppressed, and is invisible to the immune system and therefore does not significantly infiltrate immune cells. Therefore, despite its moderately high mutation burden, this tumor is often classified as an immunologically cold tumor. B16-F10 is a malignant melanoma cell whose most notable characteristic is its strong metastatic potential to spread throughout the body. Since the lung metastasis model through the tail vein is known to represent the clinical response, the metastatic melanoma tumor model prepared by the method of Example 1-8 was administered with Control hFc, hFc-soluble TGFβ Trap, hFc-FEBM-TGFβ Trap, hFc-TGFβ Trap-FEBM, hFc-FEBM-anti-TGFβ1 scfv, Anti-EDB-FN-TGFβ Trap-hFc, and hFc-anti-EDB-FN scfv-anti-TGFβ1 scfv, and the tumor growth was analyzed.

Since melanoma can arise from any organ with melanocytes, primary tumors can also arise in the lungs, and lung metastases are the most common site of distant metastatic disease in malignant melanoma. Therefore, it is important to observe whether tumor growth can be suppressed in the lungs, which are the most common clinical therapeutic site for melanoma. After confirming that cells metastasized to the lungs were settled on day 5 after transplantation, tumor growth was evaluated.

As a result, as described in FIG. 20A, despite relatively low expression of fibronectin EDB compared to other solid cancers, the administration of well-structured hFc-FEBM-TGFβ Trap, hFc-TGFβ Trap-FEBM, hFc-FEBM-anti-TGFβ1 scfv, Anti-EDB-FN-TGFβ Trap-hFc, or hFc-anti-EDB-FN scfv-anti-TGFβ1 scfv for ECM fused TGFβ trapping was confirmed to exhibit potent anticancer and anti-metastatic activities in the metastatic B16F10 melanoma model after 10 days.

In addition, as described in FIG. 20B, on day 25 after tumor transplantation, the average luminescence activity within the tumors of the hFc-soluble TGFβ Trap (lane 2), hFc-FEBM-TGFβ Trap (lane 3), hFc-TGFβ Trap-FEBM (lane 4), hFc-FEBM-anti-TGFβ1 scfv (lane 5), Anti-EDB-FN-TGFβ Trap-hFc (lane 6), and hFc-anti-EDB-FN scfv-anti-TGFβ1 scfv (lane 7) administration groups was confirmed to have decreased by about 28.5%, about 96.1%, about 88.8%, about 72.2%, about 76.9%, and about 81.7%, respectively, compared to the luminescence activity of the isotype control group (Control-Fc). hFc-FEBM-TGFβ Trap (lane 3), hFc-TGFβ Trap-FEBM (lane 4), hFc-FEBM-anti-TGFβ1 scfv (lane 5), Anti-EDB-FN-TGFβ Trap-hFc (lane 6) and hFc-anti-EDB-FN scfv-anti-TGFβ1 scfv (lane 7) groups showed statistically significant tumor growth inhibition to a greater extent (>94%, >84%, >60.6%, >67.2%, and >74.1%, respectively) than hFc-soluble TGFβ Trap (lane 2).

That is, although the mRNA expression of EDB-FN1 in tumors of B16-F10 transplanted mice was higher than that in normal tissues, it was the lowest level compared to other solid tumors, and the optimal structural combination for EDB-FN targeting/TGFβ Trapping was confirmed to have sufficient effectiveness.

### Example 9. Confirmation of cancer therapeutic effect by administration of triple-specific Fc fusion protein consisting of FEBM or L19 scfv, TGFβ Trap and PD-1 antibody or scFv

Most solid tumor patients with high-frequency microsatellite instability-H (MSI-H) have the opportunity to receive combination therapy with anti-PD-1 antibodies, and for cancers with low or no defects in gene replication, numerous trials and thousands of clinical trials are underway related to the fusion of anti-PD-1 antibodies or scfv. Therefore, in the present invention, the tumor suppression efficacy of the fusion of anti-PD-1 antibodies or scfv to the optimal structure targeting EDB-FN/TGFβ was compared with that of the existing clinical trial Anti-PD1 / TGFβ Trap in metastatic breast cancer and pancreatic cancer models.

### 9-1. Construction and production of triple-specific fusion proteins

The construction and production of a triple fusion protein consisting of FEBM-, L19 scfv structure for EDB-FN targeting and TGFβ Trap for inhibiting TGFβ signaling based on anti-PD-1 antibody or anti-PD-1 scfv were performed by the methods of Examples 1-3 and 1-4, and among the fusion proteins in Table 2, Anti-PD-1-hIgG-FEBM-TGFβ Trap (SEQ ID NO: 57) and Anti-EDB-FN scfv-TGFβ Trap-hFc-anti-PD1 scfv (SEQ ID NO: 77) fusion proteins were constructed and produced.

In addition, control hIgG (RecombiMAB human IgG4(S228P) isotype control, BioXcell, USA) was used as a control, Anti-PD-1 (SEQ ID NO: 109) was constructed and produced, and Bs anti-PD-1 / TGFβ Trap (Bintrafusp alfa, Cat. No.: HY-P99480, MedChemExpress, China), which is in clinical trial, was used in later examples.

### 9-2. Analysis of tumor growth rates in breast cancer orthotopic transplantation model

A breast cancer orthotopic transplantation model was produced by the method of Example 1-6, and then, 15 mg/kg of Anti-PD-1 hIgG4, Bs anti-PD-1-hIgG4/TGFβ Trap, anti-PD-1-hIgG-FEBM-TGFβ Trap and anti-EDB-FN scFv-TGFβ Trap-hFc-anti-PD-1 scFv and control-hFc were administered, and the tumor growth rate was analyzed by the method of Example 1-6.

As a result, as described in FIG. 21, on day 25, the tumor volumes of the anti-PD-1 hIgG4, Bs anti-PD-1-hIgG4/TGFβ Trap, anti-PD-1-hIgG-FEBM-TGFβ Trap, and anti-EDB-FN scfv-TGFβ Trap-hFc-anti-PD-1 scfv administration groups were confirmed to have decreased by about 2.2%, about 3.1%, about 88%, and about 75.5%, respectively, compared to the tumor volume of the isotype control group (control hIgG).

### 9-3. Analysis of tumor growth rates in pancreatic cancer orthotopic transplantation model

A pancreatic cancer orthotopic transplantation model was created by the method of Example 1-7, and then, 20 mg/kg of Anti-PD-1 hIgG4, Bs anti-PD-1-hIgG4/TGFβ Trap, anti-PD-1-hIgG-FEBM-TGFβ Trap and anti-EDB-FN scFv-TGFβ Trap-hFc-anti-PD-1 scFv and control-hFc were administered, and the tumor growth rates were analyzed by the method of Example 1-7.

As a result, as described in FIG. 21B, on day 57, the tumor volumes of the anti-PD-1 hIgG4, Bs anti-PD-1-hIgG4/TGFβ Trap, anti-PD-1-hIgG-FEBM-TGFβ Trap, and anti-EDB-FN scfv-TGFβ Trap-hFc-anti-PD-1 scfv administration groups were confirmed to have decreased by about 16.0%, about 20.1%, about 89.2%, and about 74.8%, respectively, compared to the tumor volume of the isotype control group (control hIgG).

That is, the administration of anti-PD-1 alone and soluble-TGFβ Trap dual fusion substances in breast cancer and pancreatic cancer models did not observe statistically significant antitumor activity compared to the isotype control group (control hIgG), but the administration of anti-PD-1-hIgG-FEBM-TGFβ Trap and anti-EDB-FN scfv-TGFβ Trap-hFc-anti-PD-1 scfv proteins was confirmed to exhibit potent antitumor effects, and it was confirmed that the anti-PD-1 fused triple protein under the dual structure of linked EDB-FN targeting/TGFβ Trap structure exhibited potent anticancer efficacy in cancer types that did not respond to anti-PD-1 alone or in combination.

While the specific parts of the present invention have been described in detail above, it will be apparent to those skilled in the art that such specific descriptions are merely preferred embodiments and that the scope of the present invention is not limited thereby. Accordingly, the actual scope of the present invention will be defined by the appended claims and their equivalents.

### INDUSTRIAL APPLICABILITY

The fusion protein according to the present invention secures a local effect rather than a systemic effect by directly localizing TGFβ inhibition within the cancer microenvironment by fixing TGFβ, which is important for the antitumor immune response, to the extracellular matrix, thereby exhibiting excellent anticancer effects in all cancer types, and can be advantageously used for the prevention or treatment of cancer by exhibiting excellent anticancer effects even in pancreatic cancer, where general TGFβ inhibitors do not work due to the rigidity of the extracellular matrix.

### SEQUENCE LIST

An electronic file is attached.

## Claims

1. A fusion protein, comprising:
(a) a polypeptide that specifically binds to extradomain B of fibronectin (EDB-FN); and
(b) a polypeptide that specifically binds to transforming growth factor β (TGFβ).

2. The fusion protein of claim 1, wherein the fusion protein further comprises (c) an antibody constant region.

3. The fusion protein of claim 2, wherein the fusion protein further comprises (d) a polypeptide that specifically binds to a tumor antigen.

4. The fusion protein of claim 3, wherein the tumor antigen is any one or more selected from the group consisting of PD-1, PD-L1, CTLA-4, CD80, CD86 and VEGF.

5. The fusion protein of claim 3, wherein the fusion protein further comprises a polypeptide that specifically binds to PD-1.

6. The fusion protein of claim 2, wherein the fusion protein comprises, from the N-terminus to the C-terminus, one or more structures selected from the group consisting of:
(i) a polypeptide that specifically binds to EDB-FN; and a polypeptide that specifically binds to TGFβ;
(ii) a polypeptide that specifically binds to TGFβ; and a polypeptide that specifically binds to EDB-FN;
(iii) one or more polypeptides that specifically bind to EDB-FN; an antibody constant region; and one or more polypeptides that specifically bind to TGFβ;
(iv) one or more polypeptides that specifically bind to TGFβ; an antibody constant region; and one or more polypeptides that specifically bind to EDB-FN;
(v) one or more polypeptides that specifically bind to EDB-FN; one or more polypeptides that specifically bind to TGFβ; and an antibody constant region;
(vi) one or more polypeptides that specifically bind to EDB-FN; one or more polypeptides that specifically bind to TGFβ; an antibody constant region; and one or more polypeptides that specifically bind to EDB-FN;
(vii) one or more polypeptides that specifically bind to TGFβ; one or more polypeptides that specifically bind to EDB-FN; an antibody constant region; and one or more polypeptides that specifically bind to EDB-FN;
(viii) an antibody constant region; one or more polypeptides that specifically bind to EDB-FN; and one or more polypeptides that specifically bind to TGFβ;
(ix) an antibody constant region; one or more polypeptides that specifically bind to TGFβ; and one or more polypeptides that specifically bind to EDB-FN; and
(x) one or more polypeptides that specifically bind to EDB-FN; an antibody constant region; one or more polypeptides that specifically bind to TGFβ; and one or more polypeptides that specifically bind to EDB-FN.

7. The fusion protein of claim 6, wherein the fusion protein comprises, from the N-terminus to the C-terminus, a structure selected from the group consisting of:
(7-1) an antibody constant region; a polypeptide that specifically binds to EDB-FN; and a polypeptide that specifically binds to TGFβ; and
(7-2) an antibody constant region; a polypeptide that specifically binds to TGFβ; and a polypeptide that specifically binds to EDB-FN.

8. The fusion protein of claim 5, wherein the fusion protein comprises, from the N-terminus to the C-terminus, one or more structures selected from the group consisting of:
(A) a polypeptide that specifically binds to PD-1; a polypeptide that specifically binds to EDB-FN; and a polypeptide that specifically binds to TGFβ;
(B) a polypeptide that specifically binds to PD-1; a polypeptide that specifically binds to TGFβ; and a polypeptide that specifically binds to EDB-FN;
(C) a polypeptide that specifically binds to TGFβ; a polypeptide that specifically binds to EDB-FN; and a polypeptide that specifically binds to PD-1;
(D) a polypeptide that specifically binds to PD-1; an antibody constant region; a polypeptide that specifically binds to EDB-FN; and a polypeptide that specifically binds to TGFβ;
(E) a polypeptide that specifically binds to PD-1; an antibody constant region; a polypeptide that specifically binds to TGFβ; and a polypeptide that specifically binds to EDB-FN;
(F) a polypeptide that specifically binds to TGFβ; a polypeptide that specifically binds to EDB-FN; a polypeptide that specifically binds to PD-1; and an antibody constant region; and
(G) a polypeptide that specifically binds to EDB-FN; a polypeptide that specifically binds to TGFβ; a polypeptide that specifically binds to PD-1; and an antibody constant region.

9. The fusion protein of claim 1, wherein the polypeptide that specifically binds to EDB-FN is a fibronectin EDB binding motif (FEBM), an antibody that specifically binds to EDB-FN or a fragment thereof.

10. The fusion protein of claim 1, wherein the polypeptide that specifically binds to TGFβ is a TGFβ receptor type 2 ectodomain (TGFβRII ectodomain, TGFβ Trap), an antibody that specifically binds to TGFβ or a fragment thereof.

11. The fusion protein of claim 2, wherein the antibody constant region is a heavy-chain constant region or a light-chain constant region.

12. The fusion protein of claim 5, wherein the polypeptide that specifically binds to PD-1 is an antibody that specifically binds to PD-1 or a fragment thereof.

13. The fusion protein of claim 9, wherein the polypeptide that specifically binds to EDB-FN is any one or more of polypeptides represented by amino acid sequences of SEQ ID NOs: 1 to 4.

14. The fusion protein of claim 10, wherein the polypeptide that specifically binds to TGFβ is any one or more of polypeptides represented by amino acid sequences of SEQ ID NOs: 5 to 8.

15. The fusion protein of claim 11, wherein the antibody constant region is any one or more of polypeptides represented by amino acid sequences of SEQ ID NOs: 12 to 15.

16. The fusion protein of claim 12, wherein the polypeptide that specifically binds to PD-1 is any one or more of polypeptides represented by amino acid sequences of SEQ ID NOs: 9 to 11.

17. The fusion protein of claim 6, wherein the fusion protein comprises, from the N-terminus to the C-terminus, any one or more polypeptides selected from the group consisting of:
(4-1) a polypeptide of SEQ ID NO: 5; a polypeptide of SEQ ID NO: 12 and a polypeptide of SEQ ID NO: 1;
(4-2) a polypeptide of SEQ ID NO: 5; a polypeptide of SEQ ID NO: 1; and a polypeptide of SEQ ID NO: 12;
(4-3) a polypeptide of SEQ ID NO: 1; a polypeptide of SEQ ID NO: 5; and a polypeptide of SEQ ID NO: 12;
(4-4) a polypeptide of SEQ ID NO: 1; a polypeptide of SEQ ID NO: 12; and a polypeptide of SEQ ID NO: 5;
(4-5) a polypeptide of SEQ ID NO: 12; a polypeptide of SEQ ID NO: 1; and a polypeptide of SEQ ID NO: 5;
(4-6) a polypeptide of SEQ ID NO: 12; a polypeptide of SEQ ID NO: 5; and a polypeptide of SEQ ID NO: 1;
(4-7) a polypeptide of SEQ ID NO: 1; a polypeptide of SEQ ID NO: 5; a polypeptide of SEQ ID NO: 1; and a polypeptide of SEQ ID NO: 12;
(4-8) a polypeptide of SEQ ID NO: 12; a polypeptide of SEQ ID NO: 1; a polypeptide of SEQ ID NO: 5; and a polypeptide of SEQ ID NO: 1;
(4-9) a polypeptide of SEQ ID NO: 1; a polypeptide of SEQ ID NO: 5; a polypeptide of SEQ ID NO: 1; a polypeptide of SEQ ID NO: 12; and a polypeptide of SEQ ID NO: 1;
(4-10) a polypeptide of SEQ ID NO: 1; a polypeptide of SEQ ID NO: 12; a polypeptide of SEQ ID NO: 1; a polypeptide of SEQ ID NO: 5; and a polypeptide of SEQ ID NO: 1;
(4-11) a polypeptide of SEQ ID NO: 5; a polypeptide of SEQ ID NO: 1; and a polypeptide of SEQ ID NO: 13;
(4-12) a polypeptide of SEQ ID NO: 13; a polypeptide of SEQ ID NO: 1; and a polypeptide of SEQ ID NO: 5;
(4-13) a polypeptide of SEQ ID NO: 2; a polypeptide of SEQ ID NO: 14; and a polypeptide of SEQ ID NO: 5;
(4-14) a polypeptide of SEQ ID NO: 3; a polypeptide of SEQ ID NO: 15; a polypeptide of SEQ ID NO: 5;
(4-15) a polypeptide of SEQ ID NO: 5; a polypeptide of SEQ ID NO: 2; and a polypeptide of SEQ ID NO: 13;
(4-16) a polypeptide of SEQ ID NO: 4; a polypeptide of SEQ ID NO: 12; and a polypeptide of SEQ ID NO: 5;
(4-17) a polypeptide of SEQ ID NO: 12; a polypeptide of SEQ ID NO: 4; and a polypeptide of SEQ ID NO: 5;
(4-18) a polypeptide of SEQ ID NO: 4; a polypeptide of SEQ ID NO: 5; and a polypeptide of SEQ ID NO: 12;
(4-19) a polypeptide of SEQ ID NO: 12; a polypeptide of SEQ ID NO: 5; and a polypeptide of SEQ ID NO: 4;
(4-20) a polypeptide of SEQ ID NO: 4; and a polypeptide of SEQ ID NO: 5;
(4-21) a polypeptide of SEQ ID NO: 6; a polypeptide of SEQ ID NO: 14; and a polypeptide of SEQ ID NO: 1;
(4-22) a polypeptide of SEQ ID NO: 7; a polypeptide of SEQ ID NO: 15; and a polypeptide of SEQ ID NO: 1;
(4-23) a polypeptide of SEQ ID NO: 1; a polypeptide of SEQ ID NO: 6; and a polypeptide of SEQ ID NO: 14;
(4-24) a polypeptide of SEQ ID NO: 2; a polypeptide of SEQ ID NO: 14; and a polypeptide of SEQ ID NO: 5;
(4-25) a polypeptide of SEQ ID NO: 1; a polypeptide of SEQ ID NO: 8; and a polypeptide of SEQ ID NO: 12;
(4-26) a polypeptide of SEQ ID NO: 12; a polypeptide of SEQ ID NO: 1; and a polypeptide of SEQ ID NO: 8;
(4-27) a polypeptide of SEQ ID NO: 12; a polypeptide of SEQ ID NO: 8; and a polypeptide of SEQ ID NO: 1;
(4-28) a polypeptide of SEQ ID NO: 8; and a polypeptide of SEQ ID NO: 1;
(4-29) a polypeptide of SEQ ID NO: 1; and a polypeptide of SEQ ID NO: 8;
(4-30) a polypeptide of SEQ ID NO: 2; a polypeptide of SEQ ID NO: 14; and a polypeptide of SEQ ID NO: 8;
(4-31) a polypeptide of SEQ ID NO: 8; a polypeptide of SEQ ID NO: 14; and a polypeptide of SEQ ID NO: 4;
(4-32) a polypeptide of SEQ ID NO: 8; a polypeptide of SEQ ID NO: 2; and a polypeptide of SEQ ID NO: 14;
(4-33) a polypeptide of SEQ ID NO: 4; a polypeptide of SEQ ID NO: 6; and a polypeptide of SEQ ID NO: 14;
(4-34) a polypeptide of SEQ ID NO: 4; a polypeptide of SEQ ID NO: 8; and a polypeptide of SEQ ID NO: 12;
(4-35) a polypeptide of SEQ ID NO: 8; a polypeptide of SEQ ID NO: 4; and a polypeptide of SEQ ID NO: 12;
(4-36) a polypeptide of SEQ ID NO: 12; a polypeptide of SEQ ID NO: 4; and a polypeptide of SEQ ID NO: 8;
(4-37) a polypeptide of SEQ ID NO: 12; a polypeptide of SEQ ID NO: 8; and a polypeptide of SEQ ID NO: 4;
(4-38) a polypeptide of SEQ ID NO: 4; and a polypeptide of SEQ ID NO: 8; and
(4-39) a polypeptide of SEQ ID NO: 8; and a polypeptide of SEQ ID NO: 4.

18. The fusion protein of claim 6, wherein the fusion protein comprises, from the N-terminus to the C-terminus:
(4-5) a polypeptide of SEQ ID NO: 12; a polypeptide of SEQ ID NO: 1; and a polypeptide of SEQ ID NO: 5; or
(4-6) a polypeptide of SEQ ID NO: 12; a polypeptide of SEQ ID NO: 5; and a polypeptide of SEQ ID NO: 1.

19. The fusion protein of claim 8, wherein the fusion protein comprises, from the N-terminus to the C-terminus, any one or more polypeptides selected from the group consisting of:
(5-1) a polypeptide of SEQ ID NO: 9; a polypeptide of SEQ ID NO: 14; a polypeptide of SEQ ID NO: 1; and a polypeptide of SEQ ID NO: 5;
(5-2) a polypeptide of SEQ ID NO: 9; a polypeptide of SEQ ID NO: 14; a polypeptide of SEQ ID NO: 5; and a polypeptide of SEQ ID NO: 1;
(5-3) a polypeptide of SEQ ID NO: 10; a polypeptide of SEQ ID NO: 15; a polypeptide of SEQ ID NO: 1; and a polypeptide of SEQ ID NO: 5;
(5-4) a polypeptide of SEQ ID NO: 10; a polypeptide of SEQ ID NO: 15; a polypeptide of SEQ ID NO: 5; and a polypeptide of SEQ ID NO: 1;
(5-5) a polypeptide of SEQ ID NO: 5; a polypeptide of SEQ ID NO: 1; a polypeptide of SEQ ID NO: 9; and a polypeptide of SEQ ID NO: 14;
(5-6) a polypeptide of SEQ ID NO: 1; a polypeptide of SEQ ID NO: 5; a polypeptide of SEQ ID NO: 9; and a polypeptide of SEQ ID NO: 14;
(5-7) a polypeptide of SEQ ID NO: 11; a polypeptide of SEQ ID NO: 12; a polypeptide of SEQ ID NO: 1; and a polypeptide of SEQ ID NO: 5;
(5-8) a polypeptide of SEQ ID NO: 11; a polypeptide of SEQ ID NO: 12; a polypeptide of SEQ ID NO: 5; and a polypeptide of SEQ ID NO: 1;
(5-9) a polypeptide of SEQ ID NO: 5; a polypeptide of SEQ ID NO: 1; a polypeptide of SEQ ID NO: 12; and a polypeptide of SEQ ID NO: 11;
(5-10) a polypeptide of SEQ ID NO: 11; a polypeptide of SEQ ID NO: 1; and a polypeptide of SEQ ID NO: 5;
(5-11) a polypeptide of SEQ ID NO: 11; a polypeptide of SEQ ID NO: 5; and a polypeptide of SEQ ID NO: 1;
(5-12) a polypeptide of SEQ ID NO: 5; a polypeptide of SEQ ID NO: 1; and a polypeptide of SEQ ID NO: 11;
(5-13) a polypeptide of SEQ ID NO: 9; a polypeptide of SEQ ID NO: 14; a polypeptide of SEQ ID NO: 4; and a polypeptide of SEQ ID NO: 5;
(5-14) a polypeptide of SEQ ID NO: 9; a polypeptide of SEQ ID NO: 14; a polypeptide of SEQ ID NO: 5; and a polypeptide of SEQ ID NO: 4;
(5-15) a polypeptide of SEQ ID NO: 10; a polypeptide of SEQ ID NO: 15; a polypeptide of SEQ ID NO: 4; and a polypeptide of SEQ ID NO: 5;
(5-16) a polypeptide of SEQ ID NO: 10; a polypeptide of SEQ ID NO: 15; a polypeptide of SEQ ID NO: 5; and a polypeptide of SEQ ID NO: 4;
(5-17) a polypeptide of SEQ ID NO: 4; a polypeptide of SEQ ID NO: 5; a polypeptide of SEQ ID NO: 9; and a polypeptide of SEQ ID NO: 14;
(5-18) a polypeptide of SEQ ID NO: 5; a polypeptide of SEQ ID NO: 4; a polypeptide of SEQ ID NO: 9; and a polypeptide of SEQ ID NO: 14;
(5-19) a polypeptide of SEQ ID NO: 11; a polypeptide of SEQ ID NO: 12; a polypeptide of SEQ ID NO: 4; and a polypeptide of SEQ ID NO: 5;
(5-20) a polypeptide of SEQ ID NO: 11; a polypeptide of SEQ ID NO: 12; a polypeptide of SEQ ID NO: 5; and a polypeptide of SEQ ID NO: 4;
(5-21) a polypeptide of SEQ ID NO: 4; a polypeptide of SEQ ID NO: 5; a polypeptide of SEQ ID NO: 12; and a polypeptide of SEQ ID NO: 11;
(5-22) a polypeptide of SEQ ID NO: 11; a polypeptide of SEQ ID NO: 4; and a polypeptide of SEQ ID NO: 5;
(5-23) a polypeptide of SEQ ID NO: 11; a polypeptide of SEQ ID NO: 5; and a polypeptide of SEQ ID NO: 4;
(5-24) a polypeptide of SEQ ID NO: 5; a polypeptide of SEQ ID NO: 11; and a polypeptide of SEQ ID NO: 4;
(5-25) a polypeptide of SEQ ID NO: 9; a polypeptide of SEQ ID NO: 14; a polypeptide of SEQ ID NO: 1; and a polypeptide of SEQ ID NO: 8;
(5-26) a polypeptide of SEQ ID NO: 9; a polypeptide of SEQ ID NO: 14; a polypeptide of SEQ ID NO: 8; and a polypeptide of SEQ ID NO: 1;
(5-27) a polypeptide of SEQ ID NO: 10; a polypeptide of SEQ ID NO: 15; a polypeptide of SEQ ID NO: 1; and a polypeptide of SEQ ID NO: 8;
(5-28) a polypeptide of SEQ ID NO: 10; a polypeptide of SEQ ID NO: 15; a polypeptide of SEQ ID NO: 8; and a polypeptide of SEQ ID NO: 1;
(5-29) a polypeptide of SEQ ID NO: 8; a polypeptide of SEQ ID NO: 1; a polypeptide of SEQ ID NO: 9; and a polypeptide of SEQ ID NO: 14;
(5-30) a polypeptide of SEQ ID NO: 1; a polypeptide of SEQ ID NO: 8; a polypeptide of SEQ ID NO: 9; and a polypeptide of SEQ ID NO: 14;
(5-31) a polypeptide of SEQ ID NO: 11; a polypeptide of SEQ ID NO: 12; a polypeptide of SEQ ID NO: 1; and a polypeptide of SEQ ID NO: 8;
(5-32) a polypeptide of SEQ ID NO: 11; a polypeptide of SEQ ID NO: 12; a polypeptide of SEQ ID NO: 8; and a polypeptide of SEQ ID NO: 1;
(5-33) a polypeptide of SEQ ID NO: 8; a polypeptide of SEQ ID NO: 1; a polypeptide of SEQ ID NO: 12; and a polypeptide of SEQ ID NO: 11;
(5-34) a polypeptide of SEQ ID NO: 11; a polypeptide of SEQ ID NO: 1; and a polypeptide of SEQ ID NO: 8;
(5-35) a polypeptide of SEQ ID NO: 11; a polypeptide of SEQ ID NO: 8; and a polypeptide of SEQ ID NO: 1;
(5-36) a polypeptide of SEQ ID NO: 8; a polypeptide of SEQ ID NO: 1; and a polypeptide of SEQ ID NO: 11;
(5-37) a polypeptide of SEQ ID NO: 9; a polypeptide of SEQ ID NO: 14; a polypeptide of SEQ ID NO: 4; and a polypeptide of SEQ ID NO: 8;
(5-38) a polypeptide of SEQ ID NO: 9; a polypeptide of SEQ ID NO: 14; a polypeptide of SEQ ID NO: 8; and a polypeptide of SEQ ID NO: 4;
(5-39) a polypeptide of SEQ ID NO: 10; a polypeptide of SEQ ID NO: 15; a polypeptide of SEQ ID NO: 4; and a polypeptide of SEQ ID NO: 8;
(5-40) a polypeptide of SEQ ID NO: 10; a polypeptide of SEQ ID NO: 15; a polypeptide of SEQ ID NO: 8; and a polypeptide of SEQ ID NO: 4;
(5-41) a polypeptide of SEQ ID NO: 4; a polypeptide of SEQ ID NO: 8; a polypeptide of SEQ ID NO: 9; and a polypeptide of SEQ ID NO: 14;
(5-42) a polypeptide of SEQ ID NO: 8; a polypeptide of SEQ ID NO: 4; a polypeptide of SEQ ID NO: 9; and a polypeptide of SEQ ID NO: 14;
(5-43) a polypeptide of SEQ ID NO: 11; a polypeptide of SEQ ID NO: 12; a polypeptide of SEQ ID NO: 4; and a polypeptide of SEQ ID NO: 8;
(5-44) a polypeptide of SEQ ID NO: 11; a polypeptide of SEQ ID NO: 12; a polypeptide of SEQ ID NO: 8; and a polypeptide of SEQ ID NO: 4;
(5-45) a polypeptide of SEQ ID NO: 4; a polypeptide of SEQ ID NO: 8; a polypeptide of SEQ ID NO: 12; and a polypeptide of SEQ ID NO: 11;
(5-46) a polypeptide of SEQ ID NO: 11; a polypeptide of SEQ ID NO: 4; and a polypeptide of SEQ ID NO: 8;
(5-47) a polypeptide of SEQ ID NO: 11; a polypeptide of SEQ ID NO: 8; and a polypeptide of SEQ ID NO: 4; and
(5-48) a polypeptide of SEQ ID NO: 8; a polypeptide of SEQ ID NO: 4; and a polypeptide of SEQ ID NO: 11.

20. The fusion protein of claim 17, wherein the fusion protein is any one selected from the group consisting of SEQ ID NOs: 16 to 56.

21. The fusion protein of claim 8, wherein the fusion protein is represented by an amino acid sequence of SEQ ID NO: 20, or represented by an amino acid sequence of SEQ ID NO: 21.

22. The fusion protein of claim 19, wherein the fusion protein is any one selected from the group consisting of SEQ ID NOs: 57 to 104.

23. A nucleic acid, encoding the fusion protein according to any one of claims 1 to 22.

24. A recombinant expression vector, comprising the nucleic acid of claim 23.

25. A host cell, transfected with the recombinant expression vector of claim 24.

26. A method for producing a fusion protein, comprising:
culturing the host cell of claim 25 to produce a fusion protein; and isolating and purifying the produced fusion protein.

27. A composition for preventing or treating cancer, comprising the fusion protein according to any one of claims 1 to 22.
